(19) Europäisches Patentamt
European Patent Office
Office européen des brevets

(11) EP 2 934 599 B1

(12) **EUROPEAN PATENT SPECIFICATION**

(45) Date of publication and mention of the grant of the patent:
**27.05.2020 Bulletin 2020/22**

(21) Application number: **13821508.2**

(22) Date of filing: **20.12.2013**

(51) Int Cl.:
*A61K 49/00* (2006.01) *A61K 49/16* (2006.01)
*A61K 49/18* (2006.01) *A61K 49/22* (2006.01)

(86) International application number:
**PCT/GB2013/053398**

(87) International publication number:
**WO 2014/096859 (26.06.2014 Gazette 2014/26)**

(54) **MICROPARTICLE COMPOSITIONS**

MIKROPARTIKELZUSAMMENSETZUNGEN

COMPOSITIONS DE MICROPARTICULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2012 GB 201223332**
**03.01.2013 GB 201300064**

(43) Date of publication of application:
**28.10.2015 Bulletin 2015/44**

(73) Proprietor: **Yeh, James Shue-Min**
**London, Greater London SE1 2AU (GB)**

(72) Inventor: **Yeh, James Shue-Min**
**London, Greater London SE1 2AU (GB)**

(74) Representative: **Stepney, Gregory John**
**Withers & Rogers LLP**
**4 More London Riverside**
**London SE1 2AU (GB)**

(56) References cited:
**WO-A1-2008/131217 WO-A1-2011/038043**

- **YOICHI NEGISHI ET AL: "AG73-modified Bubble liposomes for targeted ultrasound imaging of tumor neovasculature", BIOMATERIALS, vol. 34, no. 2, 22 November 2012 (2012-11-22), pages 501-507, XP055112949, ISSN: 0142-9612, DOI: 10.1016/j.biomaterials.2012.09.056**
- **YEH J S M ET AL: "Molecular imaging of the heart using contrast ultrasound: acoustic quantification of molecular expressions", EUROPEAN HEART JOURNAL, vol. 29, no. Suppl. 1, September 2008 (2008-09), page 21, XP002723002, & ANNUAL CONGRESS OF THE EUROPEAN-SOCIETY-OF-CARDIOLOGY; MUNICH, GERMANY; AUGUST 30 -SEPTEMBER 03, 2008**
- **VILLANUEVA ET AL: "Molecular imaging of cardiovascular disease using ultrasound", JOURNAL OF NUCLEAR CARDIOLOGY, MOSBY, ST. LOUIS, MO, US, vol. 15, no. 4, 1 July 2008 (2008-07-01), pages 576-586, XP023313591, ISSN: 1071-3581, DOI: 10.1016/J.NUCLCARD.2008.05.005 [retrieved on 2008-07-29]**
- **KLIBANOV A L: "LIGAND-CARRYING GAS-FILLED MICROBUBBLES: ULTRASOUND CONTRAST AGENTS FOR TARGETED MOLECULAR IMAGING", BIOCONJUGATE CHEMISTRY, ACS, WASHINGTON, DC, US, vol. 16, no. 1, 1 January 2005 (2005-01-01), pages 9-17, XP001222916, ISSN: 1043-1802, DOI: 10.1021/BC049898Y**
- **LANZA G M ET AL: "TARGETED ULTRASONIC CONTRAST AGENTS FOR MOLECULAR IMAGING AND THERAPY", PROGRESS IN CARDIOVASCULAR DISEASES, SAUNDERS, PHILADELPHIA, PA, US, vol. 44, no. 1, 1 July 2001 (2001-07-01), pages 13-31, XP008034236, ISSN: 0033-0620, DOI: 10.1053/PCAD.2001.26440**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **B. A. KAUFMANN ET AL: "Molecular Imaging of Inflammation in Atherosclerosis With Targeted Ultrasound Detection of Vascular Cell Adhesion Molecule-1", CIRCULATION, vol. 116, no. 3, 17 July 2007 (2007-07-17) , pages 276-284, XP055020746, ISSN: 0009-7322, DOI: 10.1161/CIRCULATIONAHA.106.684738**

Remarks:

The file contains technical information submitted after the application was filed and not included in this specification

## Description

Field of the Invention

The present invention relates to microparticle compositions. In particular, though not exclusively, it concerns microparticle compositions suitable for ultrasound imaging of chosen molecular moieties.

Background to the Invention

Diagnostic sonography (ultrasonography) is an ultrasound-based diagnostic imaging technique used for visualising tissues including tendons, muscles, joints, vessels and internal organs for possible pathology or lesions. A common example is obstetric sonography, which is routinely used during pregnancy. However, so far, ultrasound imaging has been limited to defining anatomical structure and movement, and blood flow.

Ultrasound molecular imaging, on the other hand, facilitates imaging of a given molecular moiety/moieties of interest (expressions) by way of targeting microbubbles, which comprise targeting ligands (molecular binding elements) on the particle shell.[1] This method works by *intravenous* (*iv*) administration of the targeting microbubbles, which then circulate and accumulate in regions expressing the molecules of interest. Accumulation of the microbubbles may be depicted on an ultrasound image as areas of bright signal locating the molecules. One such microparticle compositions for imaging is disclosed in Y. Negishi et al, Biomaterials 34 (2013) p.501-507 (published on-line on 22 October 2012).

This technique has allowed *in vivo* molecular imaging in animal models. However, ultrasound molecular imaging has not yet been performed in humans, partly due to uncertainties regarding its safety and efficacy, including:

(1) the traditional use of biotin-(strept)avidin conjugation chemistry for attaching targeting ligands (molecular binding elements) to the microbubble shell. Streptavidin and avidin are immunogenic proteins, best avoided for human use;
(2) the traditional use of high ultrasound power imaging techniques involving 'instantaneous' particle destruction,[1, 2] which raises safety concerns.[3] Furthermore, the technique makes real-time bedside visualisation of the molecular target (molecular moiety of interest) difficult, and continuous imaging for assessing the beating heart or multi-plane imaging using a single-bolus of microbubbles impossible; and
(3) the low degree of quantification of ultrasound molecular imaging, diminishing its utility as a clinical diagnostic tool.

Accordingly, it is an aim to provide a microparticle composition which is non-immunogenic, and thus suitable for use in the ultrasound molecular imaging of human subjects as well as animals. Furthermore, it is an aim to provide a microparticle composition which is specific and effective *in vivo* for highly quantitative and real-time ultrasound molecular imaging of molecular moieties of interest in one or more tissues/organs. The microparticles so produced may have one or more favourable characteristics *in vivo* which include, but are not limited to, being non-toxic, sufficiently stable for continuous and/or multi-plane imaging with a single-bolus microparticle administration, having favourable kinetics and acoustic response for highly quantitative analysis of the molecular moieties of interest, a sufficiently high targeting specificity and efficiency to the molecular moieties of interest, and lacking non-specific binding/persistence in tissues not expressing the molecular moieties (target molecules) of interest (except in tissues of the reticuloendothelial system (RES) such as the liver and spleen which are the usual routes of microparticle elimination in the body).

Summary of the Invention

The invention provides microparticles, its intermediate compositions and kits, methods of preparing said microparticle compositions and imaging methods emplyoing said microparticles. These are as defined by the claims. This composition provides a native microparticle structure which can be modified as appropriate for imaging of a selected molecular target(s) (molecular moiety/moieties of interest). As such, the microparticles may further comprise at least one molecular binding element, wherein the at least one molecular binding element is covalently attached to the shell of the core microparticle structure.

In terms of imaging, the specific composition has been found to yield sufficiently echogenic and stable microparticles, which lack non-specific binding, and produce no immediate adverse effects *in vivo.* The microparticles also facilitate efficient target binding under flow conditions by means of the molecular binding element(s) attached to the shell of the core structure. Favourable kinetics and acoustic response of the microparticles allow highly quantitative real-time ultrasound molecular imaging *in vivo.*

The term "molecular imaging" refers to the imaging of molecular moieties, such as, but not limited to, molecules, cells, or particles (including those present on artificial/implanted materials, e.g., metals, polymers or drugs on a coronary stent, prosthetic heart valve or closure device). As such, the term "molecular imaging" may be used to refer to the imaging of phenotypes (detecting the presence, absence, and/or degree of), by targeting one or a combination of the molecular

moieties with the microparticles. Examples of phenotypes include, but are not limited to, the physiological state, pathological state, pathophysiological state, disease state, or operational state (e.g., of a device). For example, molecular imaging using microparticles targeting E-selectin (Esel) (an endothelial adhesion molecule expressed during endothelial activation in inflammation) can be used for imaging detection and assessment of the degree of Esel expression, as well as endothelial activation and/or inflammation. Non-targeting microparticles, such as microparticles containing irrelevant molecular binding elements, or none at all, may be used in molecular imaging, often as negative controls or for assessing the degree of non-specific microparticle binding/adhesion/retention in the tissue/organ/subject/system, or as agents for calibration of imaging signals (e.g., calibration of microparticle ultrasound imaging signal intensity vs. microparticle concentration), or for assessing perfusion or delineating blood-tissue boundary, etc.

The term "microparticle" refers to small particles which behave as a whole unit in terms of their transport and properties, and which typically exhibit an average particle size diameter (determined, for example, by a microscopy, electrozone sensing, or laser diffraction technique) in the range of 0.1 to 10 $\mu$m (preferably 1 to 5 $\mu$m). The term "microbubble" may be used synonymously with the term "microparticle". A liposome is an exemplary type of microparticle which may be considered as an artificially-prepared vesicle composed of a lipid bilayer. Similarly, a micelle is a type of microparticle which may be considered as an artificially-prepared particle composed of a hydrophilic shell and a hydrophobic centre.

The microparticles of the invention comprise a core microparticle structure having a central area and a shell. The core microparticle structure is made up of a predominantly lipid-based composition, which comprises (i) a phosphatidylcholine lipid; (ii) a phosphatidylethanolamine lipid comprising at least one maleimide moiety; and (iii) an alkoxylated fatty acid. These components readily self-assemble to form a stable microparticle core structure.

The phosphatidylcholine and phosphatidylethanolamine lipids of the invention according to components (i) and (ii), respectively, are typically neutral phospholipids. As such, they are lipids comprising a hydrophilic head group comprising a phosphate group, and a hydrophobic tail group. As used herein, the term "neutral" refers to an entity that resides in an uncharged or neutral zwitterionic form at a selected pH. A suitable pH may, for example, be 7.4 +/- 0.5.

In addition, the phosphatidylcholine and phosphatidylethanolamine lipids of the invention according to components (i) and (ii), respectively, typically possess a hydrophobic tail group adorning the hydrophilic head group which is saturated or unsaturated. Preferably, the hydrophobic tail groups are saturated fatty acid groups or derivatives thereof. The term "fatty acid" refers to linear or branched, saturated or unsaturated carboxylic acids or derivatives thereof comprising a carbon chain of $C_{7-24}$. Typically, saturated fatty acids comprise a carbon chain of $C_{10-24}$, or more preferably $C_{10-20}$.

The phosphatidylcholine lipid of component (i) is preferably a phosphatidylcholine lipid comprising fatty acid chains of $C_{7-24}$, more preferably $C_{8-22}$, most preferably $C_{10-20}$. Specific examples of such lipids include, for example, dioleoylphosphatidylcholine (DOPC), dipalmitoylphosphatidylcholine (DPPC), palmitoyloleoylphosphatidylcholine (POPC), and distearoylphosphatidylcholine (DSPC). When the fatty acid chain of such lipids is in the range of $C_{10-20}$, it is believed that a more stable microparticle is formed. Most preferably, the phosphatidylcholine lipid of component (i) is a saturated $C_{10-20}$ phosphatidylcholine lipid, such as distearoylphosphatidylcholine (DSPC; 1, 2-distearoyl-*sn*-glycero-3-phosphocholine).

The phosphatidylethanolamine lipid of component (ii) preferably comprises a phosphatidylethanolamine lipid comprising fatty acid chains of $C_{7-24}$, more preferably $C_{8-22}$, most preferably $C_{10-20}$. Such lipids include structures based on, for example, dioleoyl phosphatidylethanolamine (DOPE), palmitoyloleoylphosphatidylethanolamine (POPE), and distearoylphosphatidylethanolamine (DSPE). When the fatty acid chain of such lipids is in the range of $C_{10-20}$, it is believed that a more stable microparticle is formed. Most preferably, the phosphatidylethanolamine lipid of component (ii) is a saturated $C_{10-20}$ phosphatidylethanolamine lipid, such as one comprising distearoylphosphatidylethanol amine (DSPE).

In a preferred aspect of the invention, the phosphatidylethanolamine lipid of component (ii) is pegylated. The term "PEGylated" refers to an entity, typically a polymer- or lipid-based entity, which is covalently attached to a polyethylene glycol (PEG) polymer chain. The PEG chain has a molecular formula of $C_{2n}H_{4n+2}O_{n+1}$ and a molecular mass of less than 20,000 g/mole. In this case, the maleimide moiety may be attached to the PEG polymer chain, more preferably the PEG polymer chain provides a covalent linkage between the maleimide moiety and the phosphatidylethanolamine lipid portion.

Accordingly, the phosphatidylethanolamine lipid of component (ii) preferably comprises a polyethylene glycol chain with a molecular weight of at least 500, such as at least 1000, 1500, or 2000. In terms of self-assembly, a polyethylene glycol chain with a molecular weight of between 1500 and 2500 is preferred.

The phosphatidylethanolamine lipid of component (ii) comprises at least one maleimide moiety. A maleimide moiety is a chemical entity with the general formula $H_2C_2(CO)_2NH$, which may be represented by the following structure.

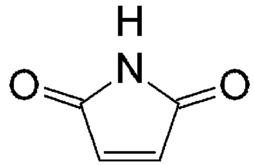

Preferably, the NH group of the maleimide moiety of component (ii) is covalently attached to the phosphatidylethanolamine portion, potentially via the PEG polymer chain.

A particularly preferred phosphatidylethanolamine lipid of the microparticle composition is 1,2-distearoyl-*sn*-glycero-3-phosphoethanolamine-*N*-[maleimide(polyethylene glycol)-2000] (abbreviated as DSPE-$PEG_{2000}$-Mal).

In particular, the use of a maleimide moiety as a handle for the molecular binding element is a departure from the immunogenic (strept)avidin-biotin conjugation chemistry traditionally used in preparing such particles. Although other conjugation chemistries may be used, it was surprisingly found that a maleimide linker yields microparticles with a low level of immunogenicity. Furthermore, the pH stability of the maleimide linkage was found to be advantageous in terms of avoiding degradation of the molecular binding element(s), and thus the microparticles during preparation, and by preventing dissociation of the molecular binding element(s) from the microparticle shell *in vivo*.

The alkoxylated fatty acid according to component (iii) of the invention may comprise a fatty acid group which is linear or branched, saturated or unsaturated. Furthermore, the fatty acid chain may be a $C_{7-24}$, more preferably a $C_{8-22}$, most preferably a $C_{10-20}$ chain. As such, fatty acid chains comprising linear, saturated or unsaturated (preferably saturated) $C_{10-20}$ chain are preferred. The alkoxy group (i.e. the group that forms an ester with the fatty acid group) may be a linear or branched alkyl group (preferably $C_{1-50}$ alkyl or $C_{1-24}$ alkyl), alkenyl group (preferably $C_{1-50}$ alkenyl or $C_{1-24}$ alkenyl), or polyethylene glycol group, singularly bonded to the oxygen of the fatty acid.

The term '$C_{x-y}$ alkyl' as used herein refers to a linear or branched saturated hydrocarbon group containing from x to y carbon atoms. For example, $C_{1-50}$ alkyl refers to a linear or branched saturated hydrocarbon group containing from 1 to 50 carbon atoms. Examples of $C_{1-50}$ alkyl groups include methyl, ethyl, n-propyl, isopropyl, n-butyl, isobutyl, sec-butyl, tert-butyl, n-pentyl, isopentyl, neopentyl, hexyl, isohexyl, 1,1-dimethylbutyl, 2,2-dimethylbutyl, 3,3-dimethylbutyl, 2-ethylbutyl, heptyl, octyl, nonyl, decyl, undecyl, dodecyl, myristyl, palmityl, stearyl, icosyl, triacontyl, tetracontyl, and pentacontyl.

The term '$C_{x-y}$ alkenyl' as used herein refers to a linear or branched hydrocarbon group containing one or more carbon-carbon double bonds and having from x to y carbon atoms. Examples of $C_{1-50}$ alkenyl groups include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, heptenyl, octenyl, nonenyl, decenyl, undecenyl, dodecenyl, tridecenyl, pentadecenyl, and hexedecenyl.

The term 'hydrocarbon' refers to an entity which consists of hydrogen and carbon only. Such an entity may be saturated or unsaturated, branched or linear.

In a preferred aspect, the alkoxylated fatty acid is a polyethylene glycol fatty acid. As such, a suitable alkoxylated fatty acid may be, for example, a $C_{10-20}$ saturated polyethylene glycol fatty acid ester. In terms of microparticle stability, a particularly preferred polyethylene glycol fatty acid ester is $PEG_{40}$ stearate.

Unless otherwise indicated, components (i)-(iii) or any further components mentioned herein may be present in salt form as well as in free (acid or base) form.

The microparticle composition according to the invention may comprise at least one molecular binding element covalently attached to the shell of the core microparticle structure. In this way, a targeting microparticle is produced which is specific to a chosen molecular moiety (expression), e.g., attaches specifically to a chosen molecular moiety. In one preferred embodiment, the at least one molecular binding element is covalently attached to the core microparticle structure via the at least one maleimide moiety. Furthermore, the microparticle composition may comprise one or more different molecular binding elements in order to target one or more molecular moieties (expressions).

The molecular binding element may be any inorganic or organic molecule which is capable of binding to a complementary molecular moiety. The precise identity and nature of the molecular binding element will depend on the molecular moiety of interest which is to be recognised by the microparticles and imaged. For example, the molecular moiety may be a molecule, protein, receptor, particle or cell, including those present on an artificial or implanted material. The molecular moiety may be present on the surface of cells. The molecular binding element can be any suitable element which is capable of binding to the molecular moiety of interest. The molecular binding element should bind specifically to the molecular moiety of interest so that it does not bind to other molecular moieties. Suitable molecular binding elements may therefore comprise, but not limited to, proteins, peptides, nucleic acids, carbohydrates, lipids, inorganic moieties, or small organic moieties (e.g. drug molecules). In some embodiments, the molecular binding moiety may be a metal (e.g., ions of nickel, cobalt, manganese or zinc, which bind to histidine-tag).[4] In particular embodiments, the molecular binding element may be a binding protein, an affibody, or an antibody molecule.

The term "binding protein" refers to any protein capable of binding specifically to a molecule (hereinafter called its "binding partner"). A specific binding protein has a binding site at which it binds to its binding partner. The binding protein generally only binds with high affinity at this site to its binding partner and other molecules with a very similar structure to its binding partner. Molecules that are not similar to the binding partner will be bound with low affinity, if at all. Binding proteins are well known to those skilled in the art. Examples of binding proteins include but are not limited to T-cell receptors, HLA proteins, cell surface receptors and enzymes.[5] The term binding protein also includes a functional fragment of these or any other molecules that is capable of specific binding. A functional fragment is a part of the binding protein which is still able to bind its binding partner specifically with high affinity.

The term "affibody" refers to a small molecule based on a 58 amino acid residue protein domain, derived from one of the IgG-binding domains of staphylococcal protein A. These can be selected to have specific affinity for a small molecule (see, for example, patent reference WO 95/19274 A1).[6-8]

The term "antibody" or "antibody molecule" refers to polyclonal or monoclonal antibodies of any isotype, or antigen binding fragments thereof, such as Fv, Fab, $F(ab')_2$ fragments and single chain Fv fragments. The antibody molecule may be a recombinant antibody molecule, such as a chimeric antibody molecule, a CDR grafted antibody molecule (also known as a humanised antibody molecule) or a fragment thereof. Such antibodies and methods for their production are well known in the art. The antibody molecule can be produced in any suitable manner, e.g. using hybridomas or phage technology. One skilled in the art would know how to produce an antibody having specific binding affinity to the small molecule.[8] The antibody molecule can be produced from any suitable organism, for example, from sheep, mice, rabbits, goats, donkeys, camels, lamas or sharks. Preferably, the antibody molecules are human, humanised, chimeric or rodent antibody molecules.

The term "bind specifically" refers to an interaction between two molecules which forms a complex that is relatively stable under physiological conditions. It is characterised by a high affinity as distinguished from non-specific binding which usually has a low affinity.

The molecular binding element is preferably an antibody molecule. In this case, the antibody molecule may be covalently attached to the maleimide group by means of a pendant thiol group (e.g. -SH), thereby creating a thioether linkage (e.g. -S-).

A specific example of an appropriate molecular binding element is an antibody for E-selectin (Esel). Esel is an endothelial adhesion molecule, classically expressed only on activated endothelial cells (basal expression in resting endothelial cells is lacking).[9] In particular, it can be used for the specific detection of endothelial activation or inflammation.[10, 11] As such, one embodiment of the present invention has allowed the specific imaging of Esel in the heart and kidneys for the first time using ultrasound.

Other intravascular molecules/cells which are involved in various pathophysiological processes of different diseases and which can be the target of the molecular binding element include, but are not limited to:

(i) P-selectin (Psel), selectins non-selectively (i.e. combination of Esel, Psel, Lsel non-selectively), inter-cellular adhesion molecule-1 (ICAM-1), vascular cell adhesion molecule-1 (VCAM-1), mucosal addressin cell adhesion molecule-1 (MAdCAM-1) and $\beta_1$,$\beta_3$-containing integrin (e.g., $\alpha_5\beta_1$, $\alpha_v\beta_3$, $\alpha_{IIb}\beta_3$ (glycoprotein IIb/IIIa receptor (GPIIb/IIIa receptor))) in the inflammatory processes of atherosclerosis, ischaemia-reperfusion injury, acute myocardial infarction & primary angioplasty, chronic ischaemia, heart transplant rejection, encephalomyelitis, Crohn's disease and tumour irradiation, in tissues such as the aorta, heart, skeletal muscle, kidney, brain, bowel and prostate cancer (hind limb implant).

(ii) Vascular endothelial growth factor receptor-2 (VEGFR2), $\beta_1$,$\beta_3$- or $\alpha_v$- containing integrins (e.g., $\alpha_5\beta_1$, $\alpha_v\beta_3$) and endoglin in the angiogenesis of: (a) tumour (breast, prostate, ovarian, colon, pancreatic cancer, melanoma, angiosarcoma, fibrosarcoma and glioma) located in the brain, pancreas, mammary fat pad, hind limb, flanks or inguinal area; (b) chronic ischaemia (hind limb skeletal muscle); and (c) matrigel plug angiogenesis.

(iii) $\alpha_{IIb}\beta_3$ (GPIIb/IIIa receptor) in thrombosis (blood clots) in the carotid artery, left atrial appendage, inferior vena cava and abdominal artery in stroke or thrombo-embolic disease.

(iv) L-selectin (Lsel) in lymph nodes.

(v) Reporter molecules such as the major histocompatibility complex (MHC) class I H-2Kk protein on, for example, transfected bone-marrow derived endothelial progenitor cells in endothelial progenitor cell therapy.

(vi) Activated leukocytes such as in ischaemia-reperfusion injury, acute myocardial infarction & primary angioplasty, and heart transplant rejection in the heart or kidneys (phosphatidyl serine may be used as a molecular binding element to target activated leukocytes through possible mechanisms including, but not limited to, charge interaction and binding through complement).

In some embodiments, the molecular binding element may be selected from the following table.

**Table 1 - Ultrasound molecular imaging *in vivo***

| Target molecule | Molecular binding element (targeting ligand) on microbubbles[a] | Disease model | Organ/tissue | Reference |
|---|---|---|---|---|
| P-selectin | Ab | Ischaemia-reperfusion injury | Heart (open & closed chest) | Kaufmann BA *et al,* 2007[12] |
| P-selectin | Ab | Ischaemia-reperfusion injury | Kidney (open abdomen) | Lindner JR *et al,* 2001[1] |
| P-selectin | Ab | Ischaemia-reperfusion injury | Kidney (open abdomen) | Andonian S *et al,* 2009[13] |
| P-selectin | Ab | Ischaemia-reperfusion injury | Skeletal muscle (hind-limb ischaemia) | Kaufmann BA *et al,* 2010[14] |
| L-selectin | Ab | Normal lymph node | Popliteal lymph node | Hauff P *et al,* 2004[15] |
| Selectins (non-selectively to E-, P-, and L-selectin) | Sialyl Lewis X | Ischaemia -reperfusion injury | Heart (open chest) | Villanueva FS *et al,* 2007[16] |
| ICAM-1 | Ab | Cardiac transplant rejection | Heart (abdominal heterotopic transplant) | Weller GE *et al,* 2003[17] |
| ICAM-1 | Ab | Experimental autoimmune encephalomyelitis (model for multiple sclerosis) | Brain (closed skull) | Linker RA *et al,* 2005[18] |
| ICAM-1 | Ab | Experimental autoimmune encephalomyelitis (model for multiple sclerosis) | Brain (closed skull) | Reinhardt M *et al,* 2005[19] |
| VCAM-1 | Ab | Atherosclerosis | Aorta | Kaufmann BA *et al,* 2007[20] |
| VCAM-1, or $\alpha_5$-integrins, or activated leukocytes[b] | Ab against VCAM-1 or $\alpha_5$-integrins, or phosphatidylserine (DSPS) against activated leukocytes | Inflammation & angiogenesis (chronic ischaemia - ligation of distal common iliac artery) | Skeletal muscle (hindlimb ischaemia); matrigel plug implanted in abdomen | Behm CZ *et al,* 2008[21] |
| MAdCAM-1 | Ab | Crohn's disease (small bowel): SAMP1/YitFc (SAMP) mouse strain | Bowel | Bachmann C *et al,* 2006[22] |

(continued)

| Target molecule | Molecular binding element (targeting ligand) on microbubbles[a] | Disease model | Organ/tissue | Reference |
|---|---|---|---|---|
| VEGFR2 | Ab | Tumourigenesis/ angiogenesis (prostate cancer in PSP-TGMAP transgenic mice) | Prostate cancer (GEM-CaP tumour) | Xuan *et al,* 2009[23] |
| VEGFR2 | Ab | Tumour angiogenesis (murine breast cancer, 4T1 and 67NR) | Subcutaneous tumour implant in hind limb | Lyshchik A *et al,* 2007[24] |
| VEGFR2 | Ab | Tumour angiogenesis (murine breast cancer, 67NR) | Subcutaneous tumour implant in hind limb | Lee DJ *et al,* 2008[25] |
| VEGFR2 | Ab | Tumour angiogenesis (mouse angiosarcoma; rat malignant glioma) | Tumour implanted in flank | Willmann JK *et al,* 2008a[26] |
| VEGFR2 | Ab | Tumour angiogenesis (human melanoma) | Subdermal tumour implant in hind limb | Rychak JJ *et al,* 2007[27] |
| VEGFR2 | Phage-derived heterodimer peptide against VEGFR | Tumour angiogenesis (human colon carcinoma, LS174T) | Subcutaneous tumour implant in hind limb | Pysz MA *et al,* 2010[28] |
| VEGFR2 | Phage-derived heterodimer peptide against VEGFR | Tumour angiogenesis (rat breast adenocarcinoma, 13762 MAT B III, CRL-1666) | Tumour implanted in mammary fat pad | Pochon S *et al,* 2010[29] |
| VEGFR2 | Phage-derived heterodimer peptide against VEGFR | Tumour angiogenesis (rat breast adenocarcinoma, 13762 MAT B III, CRL-1666) | Tumour implanted in mammary fat pad | Pillai R *et al,* 2010[30] |
| Endoglin (CD105), VEGFR2, VEGF-VEGFR complex | Ab | Tumour angiogenesis (murine Pan02 and human MiaPaca-2 pancreatic adenocarcinoma) | Tumour implanted in pancreas and flank | Korpanty G *et al,* 2007[31] |

(continued)

| Target molecule | Molecular binding element (targeting ligand) on microbubbles[a] | Disease model | Organ/tissue | Reference |
|---|---|---|---|---|
| $\alpha_v\beta_3$-integrin, and/or VEGFR2 | Ab | Tumour angiogenesis (human ovarian cancer, SK-OV-3) | Tumour implanted in flank | Willmann JK *et al*, 2008b[32] |
| $\alpha_v\beta_3$-integrin, or VEGFR2 | Peptide (cyclic RGD) against $\alpha_v\beta_3$, or Ab against VEGFR2 | Tumour angiogenesis (human squamous carcinoma, HaCaT-ras-A-5RT3) | Subcutaneous tumour implant in hind limb | Palmowski M *et al*, 2008[33] |
| $\alpha_v\beta_3$-integrin, or ICAM-1 | Peptide (cyclic RGD) against $\alpha_v\beta_3$, or Ab against ICAM-1 | Tumour response to carbon ion (prostate cancer, AT-1) | Subcutaneous tumour implant in hind limb | Palmowski M *et al*, 2009[34] |
| $\alpha_v\beta_3$-integrin | Peptide (cRGDyK) | Tumour angiogenesis (fibrosarcoma cell, HT 1080) | Subcutaneous tumour implanted in inguinal area | Jun HY *et al*, 2010[35] |
| $\alpha_v\beta_3$-integrin | Peptide (disulfide-constrained cystine knot (knottin) peptide containing RGD, or cRGDfK), or Ab | Tumour angiogenesis (human ovarian adenocarcinoma, SKOV-3) | Tumour implanted in flank | Willmann JK *et al*, 2010[36] |
| $\beta_1$, $\beta_3$-containing integrins, or $\alpha_v$-containing integrin | Peptide (echistatin - contains cyclic RGD) against $\beta_1$, $\beta_3$-containing integrins, or Ab against $\alpha_v$-containing integrin | Angiogenesis | Matrigel plug in abdomen | Leong-Poi H *et al*, 2003[37] |
| $\beta_1$, $\beta_3$-containing integrins | Peptide (echistatin - contains cyclic RGD) | Angiogenesis (chronic ischaemia) | Skeletal muscle (chronic hind limb ischaemia - iliac artery ligation) | Leong-Poi H *et al*, 2005[38] |
| $\beta_1$, $\beta_3$-containing integrins | Peptide (echistatin - contains cyclic RGD) | Tumour angiogenesis (human glioma) | Tumour implanted in brain | Ellegala DB *et al*, 2003[39] |
| $\beta_1$, $\beta_3$-containing integrins | Peptide (echistatin - contains cyclic RGD) | Angiogenesis | Matrigel plug in groin | Stieger SM *et al*, 2008[40] |
| Tumour angiogenic endothelium[c] | Peptide (cyclic RRL) | Tumour angiogenesis (human prostate carcinoma, PC3 & Clone C) | Tumour implanted in flanks | Weller GE *et al*, 2005[41] |

(continued)

| Target molecule | Molecular binding element (targeting ligand) on microbubbles[a] | Disease model | Organ/tissue | Reference |
|---|---|---|---|---|
| H-2Kk (mouse MHC class I H-2Kk protein) on transfected bone-marrow derived endothelial progenitor cell | Ab | Angiogenesis (endothelial progenitor cell engraftment/ therapy) | Subcutaneous matrigel plug implant in ventral surface of abdomen | Kuliszewski MA *et al,* 2009[42] |
| $\beta_1$, $\beta_3$-containing integrins (excluding glycoprotein IIb/IIIa receptor ($\alpha_{IIb}\beta_3$)), or activated leukocytes[b] | Peptide (echistatin - contains cyclic RGD), or phosphatidyl-serine (DSPS) against activated leukocytes | Acute coronary thrombosis & primary angioplasty | Heart (open chest) | Sakuma T *et al,* 2005[43] |
| Glycoprotein IIb/ IIIa receptor ($\alpha_{IIb}\beta_3$) on activated platelets | Fab (abciximab) | Thrombosis | Thrombus (human blood clot) previously mixed with the targeting bubbles *in vitro,* then implanted in the distal common carotid artery (internal and external carotids ligated) for imaging | Alonso A *et al,* 2007[44] |
| Glycoprotein IIb/ IIIa receptor ($\alpha_{IIb}\beta_3$) on activated platelets | Peptide containing KQAGDV sequence | Thrombosis | Abdominal artery thrombus | Tardy I *et al,* 2002[45] |
| Glycoprotein IIb/ IIIa receptor ($\alpha_{IIb}\beta_3$) on activated platelets | Peptide (linear KQAGDV or RGD analogue?) | Thrombosis | Inferior vena cava thrombus; left atrial appendage thrombus | Takeuchi M *et al,* 1999[46] |
| Glycoprotein IIb/ IIIa receptor ($\alpha_{IIb}\beta_3$) on activated platelets | Peptide (hexapeptide - sequence not disclosed) | Thrombosis | Femoral vein thrombus | Wang B *et al,* 2006[47] |
| Activated leukocytes[b] | Phosphatidylserine (DSPS) against activated leukocytes | Ischaemia-reperfusion injury | Kidney | Lindner JR *et al,* 2000[2] |
| Activated leukocytes[b] | Phosphatidylserine against activated leukocytes | Ischaemia-reperfusion injury | Kidney | Jing XX *et al,* 2008[48] |
| Activated leukocytes[b] | Phosphatidylserine (DSPS) against activated leukocytes | Ischaemia-reperfusion injury | Heart (open chest) | Christiansen JP *et al,* 2002[49] |
| Activated leukocytes[b] | Sonovue shell component | Cardiac transplant rejection | Heart (abdominal heterotopic transplant) | Kondo I *et al,* 2004[50] |

**a.** Peptide sequences in standard single letter nomenclature, with prefix 'c' for 'cyclo-'. **b.** Phosphatidylserine interaction with leukocyte includes charge interaction and complement receptor(s) (undefined) on activated leukocytes. **c.** Actual molecular target unknown. **Abbreviations:** DSPS (distearoylphosphatidylserine); Ab (antibody).

In embodiments, whether a molecular binding element(s) is conjugated to a maleimide moiety of component (ii) or not, any maleimide moieties (i.e. those containing active maleimide function) on the microparticles or component (ii) may be deactivated by, for example, hydrolysis or by reaction with excess thiols. In particular, it may be preferable to use a suitable molecule with a single thiol group per molecule, e.g., L-Cysteine (purification of the microparticles or components from the reactants may be subsequently required).

When the molecular binding element is conjugated to the microparticle, the conjugation reaction ratio used in the conjugation reaction is preferably at least 2 x $10^6$ molecular binding elements (e.g. antibody molecules) per microparticle. In some embodiments, the conjugation reaction ratio may be at least 3 x $10^6$ molecular binding elements per microparticle. Further, the conjugation reaction ratio may be at least 4 x $10^6$ molecular binding elements per microparticle. When using reaction ratios of this order, microparticles are produced which are able to sufficiently bind under flow conditions as well as static conditions. Lower levels (e.g. approximately 1x$10^6$ molecular binding elements per particle) produce microparticles which are only able to target under static conditions.

Expressed in another way, and assuming that all of component (ii) is incorporated into the shell of the microparticles, the conjugation reaction ratio used in the reaction to conjugate the molecular binding element to the microparticle is such that the conjugation reaction molar ratio of the at least one molecular binding element to component (ii) is ≥1:1. Otherwise, the conjugation reaction molar ratio of the at least one molecular binding element to component (ii) may be ≥5:1. Further, the conjugation reaction molar ratio may be ≥8:1, ≥9:1, or ≥10:1. In some embodiments, the conjugation reaction molar ratio may be about 10:1.

In some embodiments, the microparticles have at least about 0.3 x $10^5$ molecular binding elements (e.g. antibody molecules) per microparticle. This allows the microparticles to be able to sufficiently bind under flow conditions as well as static conditions. In some embodiments, the microparticles have at least about 1 x $10^5$ molecular binding elements (e.g. antibody molecules) per microparticle. The microparticles may have at least about 2 x $10^5$ molecular binding elements (e.g. antibody molecules) per microparticle. Further, the microparticles may have at least about 3 x $10^5$ molecular binding elements (e.g. antibody molecules) per microparticle. In particular embodiments, the microparticles may have between about 3 x $10^5$ and about 4 x $10^5$ molecular binding elements (e.g. antibody molecules) per microparticle.

The microparticles may have at least about 2500 conjugated molecular binding elements (e.g. antibody molecules) per $\mu m^2$ microparticle surface area. Further, the microparticles may have at least about 5000 conjugated molecular binding elements (e.g. antibody molecules) per $\mu m^2$ microparticle surface area. In some embodiments, the microparticles may have at least about 7500 conjugated molecular binding elements (e.g. antibody molecules) per $\mu m^2$ microparticle surface area.

It has been found that by keeping the conjugation sites on each molecular binding element to a minimum (e.g. reducing only 1 interchain disulfide bond to produce 2 -SH groups per antibody), and deactivating any unreacted groups after particle conjugation (e.g. by alkylation of unreacted -SH groups), significant particle aggregation due to cross-linking could be avoided.

The molecular binding element may be covalently attached to the component (ii) of the microparticle composition either before or after formation of the microparticles. When binding element is covalently attached to component (ii) before formation of the microparticles, the molar ratio of the resulting component is 5 to 15.

The structural morphology of the microparticles is not limited in any way and the particles may exhibit a liposomal or micellar structure. In some embodiments, the core microparticle structure is micellar. When the core microparticle structure is micellar, hydrophilic head groups of the constituent lipids create the shell of the core structure, while hydrophobic tails extend inward to the central area of the core. In other embodiments, the core microparticle structure is liposomal.

In a preferred aspect of the invention, the microparticle composition contains a fluid medium in the central area of the core microparticle structure. The fluid medium may be any physiologically acceptable medium, which may or may not contain active ingredients for delivery to the locations specifically targeted by the molecular binding element. Preferably, the fluid medium comprises a physiologically acceptable gas. Suitable physiologically acceptable gases include air, nitrogen, carbon dioxide, xenon, krypton, sulfur hexafluoride, chlorotrifluoromethane, dichlorodifluoro-methane, bromotrifluoromethane, bromochlorodifluoromethane, tetrafluoromethane, dibromo-difluoromethane, dichlorotetrafluoroethane, chloropentafluoroethane, hexafluoroethane, hexafluoropropylene, octafluoropropane, hexafluoro-butadiene, octafluoro-2-butene, octafluorocyclobutane, decafluorobutane, perfluorocyclopentane, dodecafluoropentane, and tetradecafluorohexane. From a practical perspective, and in terms of patient safety, air, nitrogen, sulfur hexafluoride, chlorotrifluoromethane, dichlorodifluoro-methane, bromotrifluoromethane, bromochlorodifluoromethane, tetrafluoromethane, dibromo-difluoromethane, dichlorotetrafluoroethane, chloropentafluoroethane, hexafluoroethane, hexafluoropropylene, octafluoropropane, decafluorobutane, dodecafluoropentane, and tetradecafluorohexane are preferred. In some embodiments, the physiologically acceptable gas is selected from air, nitrogen, sulfur hexafluoride, octafluoropropane, decafluorobutane, dodecafluoropentane, and tetradecafluorohexane. In other embodiments, the physiologically acceptable gas may be selected from sulfur hexafluoride, chlorotrifluoromethane, dichlorodifluoro-methane, bromotrifluoromethane, bromochlorodifluoromethane, tetrafluoromethane, dibromo-difluoromethane, dichlorotetrafluoroethane, chloropentafluoroethane, hexafluoroethane, hexafluoropropylene, octafluoropropane, decafluorobutane, dodecafluoropentane, and tetradecafluorohexane. Further, the physiologically acceptable gas may be selected from sulfur hexafluoride, octafluoropropane, decafluorobutane, dodecafluoropentane, and tetradecafluorohexane. In particular embodiments, the physiologically acceptable gas is octafluoropropane.

The microparticles may have an average diameter (as determined, for example, by microscopy, electrozone sensing, or laser diffraction techniques) in the range of 0.1 to 10 μm, 0.2 to 10 μm or 0.5 to 10 μm. In some embodiments, the microparticles may have an average diameter in the range of 0.5 to 8 μm, 0.5 to 7 μm or 0.8 to 6 μm. In particular embodiments, the microparticles may have an average diameter in the range of 1 to 5 μm. The microparticles may possess a predominately spherical morphology. In addition, the particle size distribution may be such that at least 90% or at least 95% of the particles are under 10 μm (preferably under 6 μm) in diameter. In particular embodiments, all the microparticles will have a diameter of less than 10μm. In further embodiments, at least 95% of the microparticles will have a diameter of less than 6μm. Microparticles of this size can traverse through the microvasculature or microcirculation, including the pulmonary capillary bed, unimpeded when administered for example systemically (e.g. *iv*). In terms of charge, the particles may have a near neutral charge, zeta potential in the range >-30mV to <+30mV, when measured with/without purification of the microparticles from their unincorporated shell components. The zeta potential may preferably be in the range >-20mV to <+20mV, more preferably >-20mV to <+10mV. A specific example of a microparticle comprising an antibody as the molecular binding element has a zeta potential of approximately 5mV in 1mM KCl at pH 7.4.

Microparticles may be administered via any suitable route, examples include, but are not limited to, *iv*, intra-arterial, intramuscular, intra-lymphatic, or direct injection/administration into tissue or tissue cavity/space.

The microparticles possess a composition in which the molar ratio of (i) to (iii) satisfies the following ranges: (i) 70 to 80; (ii) 5 to 15; and (iii) 10 to 20. With this specific component composition in the molar ratio specified, it has been advantageously found that a targeting microparticle may be produced which is functional and viable *in vivo* (particularly humans) for effective, quantitative and real-time ultrasound molecular imaging.

The composition of the microparticles may be used synonymously with the composition of the component mixture used to generate the microparticles (e.g., an intermediate microparticle composition).

The microparticle formulation may also comprise one or more labelling moieties, or pharmaceutically acceptable salts thereof. A labelling moiety is an entity which aids the visualisation of the microparticles by imaging techniques. The molar ratio of the labelling moiety in the composition may be 0.2 to 50, preferably 0.5 to 5.

One example of a labelling moiety is a fluorescent dye, which can be used as a visualisation tool. Examples of fluorescent dyes include, but are not limited to, 1,2-dioleoyl-sn-glycero-3-phosphoethanolamine-*N*-(5- dimethylamino-1-naphthalenesulfonyl, 1,2-dioleoyl-sn-glycero-3-phosphoethanolamine-*N*-(1-pyrenesulfonyl), 1,2-dioleoyl-sn-glycero-3-phosphoethanolamine-*N*-(carboxyfluorescein), 1-oleoyl-2- [6-[(7-nitro-2-1,3-benzoxadiazol-4-yl)amino]hexanoyl]-sn-glycero-3-phospho-L-Serine, 25-[*N*-[(7-nitrobenz-2-oxa-1,3-diazol-4-yl)-methyl]amino]-27-norcholesterol, oleoyl-2-[6-[(7-nitro-2-1,3-benzoxadiazol-4-yl)amino]hexanoyl]-sn-glycero-3-phosphoethanolamine, 1,2-dioleoyl-sn-glycero-3-phosphoethanolamine-*N*-(Lissamine Rhodamine B Sulfonyl), rhodamine dihexadecanoyl phosphoethanolamine, 3,3'-dioctadecyloxacarbocyanine perchlorate (DiO), BODIPY FL fluorescent probe, Fluorescein-5-isothiocyanate (FITC), 5-Carboxyfluorescein, and 1,1'-dioctadecyl-3,3,3',3'-tetramethylindocarbocyanine perchlorate (Dil). A preferred fluorescent dye is 1,1'-dioctadecyl-3,3,3',3'-tetramethylindocarbocyanine perchlorate (Dil). The molar ratio of the fluorescent dye in the composition may be 0.5 to 5, preferably 1 to 4, even more preferably 1.5 to 3. The microparticle composition may comprise more than one fluorescent dye.

The labelling moieties may be incorporated in the microparticle composition via attachment to one or more of components (i), (ii), or (iii), a molecular binding element, or an additional component (e.g., another lipid component). Where the labelling moiety is added via an existing microparticle component(s), the molar ratio of the unlabelled component(s) (e.g., component (i), (ii), (iii), or the conjugation ratio of the molecular binding element) may remain unchanged or reduced proportionately so that the sum molar ratio of a particular component(s) with label plus without label remains unchanged. For example, if FITC is introduced via component (i) as FITC-DSPC at a molar ratio of 2 to a component composition comprising DSPC/DSPE-$PEG_{2000}$-Mal/$PEG_{40}$ stearate at a molar ratio of 75/9/14, the final component composition can either comprise DSPC-FITC/DSPC/DSPE-$PEG_{2000}$-Mal/$PEG_{40}$ stearate at a molar ratio 2/75/9/14 or 2/73/9/14. In another embodiment, where the label is added via an additional component (e.g., via DSPE as another lipid), the molar ratio of the other microparticle components may remain unchanged. For example, if Dil (containing DSPE) is introduced at a molar ratio of 2 to a component composition comprising DSPC/DSPE-$PEG_{2000}$-Mal/$PEG_{40}$ stearate at a molar ratio of 75/9/14, the final component composition may then comprise Dil/DSPC/DSPE-$PEG_{2000}$-Mal/$PEG_{40}$ stearate at a molar ratio of 2/75/9/14. More than one type of labelling moiety may be used for a given microparticle, using the same principles above.

Alternative labelling moieties include those known in the art for use in various imaging techniques, such as magnetic resonance imaging (MRI), scintigraphy, single photon emission computed tomography (SPECT), positron emission tomography (PET), computed tomography (CT), X-ray imaging/fluoroscopy, fluorescence imaging, bioluminescence imaging, microscopy, optical methods, ultrasound imaging, or multi-modal variants thereof. Examples of such

labelling moieties may include, but are not limited to, paramagnetic, superparamagnetic or ultrasuperparamagnetic particles (e.g., gadolinium (Gd), iron oxide, iron, platinum, manganese), radionuclides (e.g., technetium-99m, thallium-201, iodine-123, iodine-131, gallium-67, indium-111, fluorine-18, carbon-11, nitrogen-13, oxygen-15, rubidium-82), radio-opaque particles (e.g., iodine, barium, metal), fluorophores or fluorescent dye (e.g., those mentioned above), enzyme substrates (e.g., that for luciferase), and gold nanoparticles. Further examples of labels may include, but are not limited to, Gd-DTPA-bis(stearylamide) (Gd-BSA); Gd-DTPA-bis(myrisitylamide) (GdDTPA-BMA); 1,2-dimyristoyl-sn-glycero-3-phosphoethanolaminediethylene-triaminepentaacetate: Gd3+ (DMPEDTPA:Gd3+); D35-1.2-dihexanoyl-sn-glycero-3-phosphocholine; gadolinium (III) 2-[4,7-bis-carboxymethyl-10-[(*N,N*-distearylamidomethyl-*N"*-amido-methyl]-1,4,7,10-tetra-azacyclododec-1-yl]-acetic acid (Gd.DOTA.DSA); gadolinium (III) 1,4,7,10-tetraazacyclododecane-1,4,7,10-tetraacetic acid mono(N1-Cholesteryloxy-3-carbonyl-1,2-diaminoethane)amide (Gd.DOTA.Chol); gadolinium (III) 1,4,7,10-tetraazacyclododecane-1,4,7,10-tetraacetic acid mono(N1-distearoylphosphatidylethanolamine)amide (Gd.DOTA.DSPE) and gadolinium (III) diethylenetriamine-1,1,4,7,10-penta(acetic acid)-10-acetic acid mono(Nl-cholesteryloxy-3-carbonyl-1,2-diaminoethane)amide (Gd.DTPA.Chol).

These labelling moieties may be introduced into the microparticles via microparticle components (i), (ii), or (iii), a molecular binding element, or via an additional component (e.g., another lipid component). Where a labelling moiety is added via an existing microparticle component(s), the molar ratio of the unlabelled component(s) (e.g., component (i), (ii), (iii), or the conjugation ratio of the molecular binding elements) may remain unchanged or reduced proportionately so that the sum molar ratio of a particular component(s) with label plus without label remains unchanged. For example, if a Gd label is introduced via component (i) as Gd.DOTA.DSPC at molar ratio of 20 to a component composition comprising DSPC/DSPE-$PEG_{2000}$-Mal/$PEG_{40}$ stearate at molar ratio of 75/9/14, the final component composition can either comprise Gd.DOTA.DSPC/DSPC/DSPE-$PEG_{2000}$-Mal/$PEG_{40}$ stearate at molar ratio 20/75/9/14 or 20/55/9/14. In another embodiment, where a label is added via an additional component (e.g., via DPPE as another lipid), the molar ratio of the other microparticle components may remain unchanged. For example, if a Gd label is introduced via an additional lipid DSPE as Gd.DOTA.DSPE at molar ratio of 20 to a component composition comprising DSPC/DSPE-$PEG_{2000}$-Mal/$PEG_{40}$ stearate at molar ratio of 75/9/14, the final component composition may then comprise Gd.DOTA.DSPE/DSPC/DSPE-$PEG_{2000}$-Mal/$PEG_{40}$ stearate at molar ratio 20/75/9/14/2. In most cases, the label forms a minor molar content of the overall microparticle formulation, but in sufficient quantities commensurate with the detection sensitivity of the intended imaging method(s). For example, more gadolinium label would be needed for MRI detection than fluorine-18 label for PET detection or Dil label for fluorescence microscopy detection. More than one type of label may be used for a given microparticle, using the same principles above.

The microparticle formulation may also comprise a further lipid component such as distearoylphosphatidylethanolamine (DSPE). When such a lipid is DSPE, the molar ratio of DSPE in the composition may be 0.5 to 5, preferably 1 to 4, even more preferably 1.5 to 3. In particular, it is preferable that DSPE is present in the microparticle composition when a label (e.g., a fluorescent dye such as Dil) is absent.

In a further embodiment of the present invention, the microparticle formulation may further comprise a tumour targeting agent. Microparticles of the present invention comprising a tumour targeting agent typically comprise a ligand for a receptor that is over-expressed in tumour cells relative to the expression of said receptors in the cells of non-tumourous tissue of mammals. In some embodiments, the molecular binding element is a tumour targeting agent.

One example of such a tumour targeting agent is one which comprises a folate moiety. In preferred aspects of the present invention, the tumour targeting agent is a phospholipid-polyethylene glycol-folate compound. More preferably the phospholipid-polyethylene glycol-folate compound is DSPE-$PEG_{2000}$-Folate [distearoylphosphatidylethanolamine-polyethylene glycol (2000)-folate].

Where appropriate, the central area of the core microparticle structure may comprise a gaseous label(s), examples include, but are not limited to, xenon-133, krypton-81m, nitrogen-13, technetium-99m DTPA).

In addition to ultrasound imaging, microparticles not containing labels may also be suitable for MRI, CT, X-ray imaging or microscopy.[51, 52] In addition, further lipids for improving magnetic resonance imaging and nuclear magnetic resonance imaging may be included.

Where appropriate, for therapy or investigational purposes, the microparticle formulation may also comprise active, therapeutic or tag materials (examples include, but are not limited to, antibodies, peptides, drugs, radionuclide containing compounds, radiopharmaceuticals, radioactive gas, or genetic materials either (i) in the central area of the core microparticle structure, (ii) in/on the core microparticle shell, or (iii) both (i) and (ii).[53]

In a particularly preferred embodiment, the microparticle composition comprises microparticles, wherein the microparticles comprise: a core microparticle structure having a central area and a shell, and at least one molecular binding element covalently attached to the shell of the core microparticle structure, wherein the core microparticle structure comprises: (i) a saturated $C_{10-20}$ phosphatidylcholine lipid; (ii) a saturated $C_{10-20}$ phosphatidylethanolamine lipid comprising a polyethylene glycol chain with a molecular weight of at least 1000, and at least one maleimide moiety; and (iii) a saturated $C_{10-20}$ polyethylene glycol fatty acid ester, wherein the microparticles possess a composition in which the molar ratio of (i) to (iii) satisfies the following ranges: (i) 70 to 80; (ii) 5 to 15; and (iii) 10 to 20, and the central

area of the core microparticle structure comprises a physiologically acceptable gas selected from air, nitrogen, sulfur hexafluoride, chlorotrifluoromethane, dichlorodifluoro-methane, bromotrifluoromethane, bromochlorodifluoromethane, tetrafluoromethane, dibromo-difluoromethane dichlorotetrafluoroethane, chloropentafluoroethane, hexafluoroethane, hexafluoropropylene, decafluorobutane, dodecafluoropentane, tetradecafluorohexane and octafluoropropane. In this embodiment, it is even more advantageous if the maleimide moiety is linked to the saturated $C_{10-20}$ phosphatidylethanolamine lipid by way of the polyethylene glycol chain, and the molecular binding element is an antibody molecule.

Further, in the above embodiments, the core microparticle structure may comprise (i) DSPC as the saturated $C_{10-20}$ phosphatidylcholine lipid; (ii) DSPE-$PEG_{2000}$-Mal as the saturated $C_{10-20}$ phosphatidylethanolamine lipid comprising a polyethylene glycol chain with a molecular weight of at least 1000, and at least one maleimide moiety; and (iii) $PEG_{40}$ stearate as the saturated $C_{10-20}$ polyethylene glycol fatty acid ester.

In the above embodiments, the molar ratio of components (i) to (iii) may satisfy the following ranges: (i) 72 to 78; (ii) 7 to 12; and (iii) 12 to 18.

In another highly preferred embodiment of the invention, the microparticle composition comprises microparticles, wherein the microparticles comprise: a core microparticle structure having a central area and a shell, and at least one molecular binding element covalently attached to the shell of the core microparticle structure, wherein the core microparticle structure comprises: (i) DSPC; (ii) DSPE-$PEG_{2000}$-Mal; and (iii) $PEG_{40}$ stearate, wherein the microparticles possess a composition in which the molar ratio of (i) to (iii) satisfies the following ranges: (i) 72 to 78; (ii) 7 to 12; and (iii) 12 to 18, the central area of the core microparticle structure comprises a physiologically acceptable gas selected from air, nitrogen, sulfur hexafluoride, chlorotrifluoromethane, dichlorodifluoro-methane, bromotrifluoromethane, bromochlorodifluoromethane, tetrafluoromethane, dibromo-difluoromethane dichlorotetrafluoroethane, chloropentafluoroethane, hexafluoroethane, hexafluoropropylene, decafluorobutane, dodecafluoropentane, tetradecafluorohexane and octafluoropropane (preferably octafluoropropane) and the molecular binding element is an antibody molecule. This embodiment preferably also comprises a further phosphatidylethanolamine lipid in a molar ratio of 0.5 to 5, preferably 1 to 4, even more preferably 1.5 to 3. This further phosphatidylethanolamine lipid also prefereably comprises fatty acid chains of $C_{7-24}$, preferably $C_{8-22}$, more preferably $C_{10-20}$, even more preferably saturated $C_{10-20}$, most preferably it is a distearoylphosphatidylethanolamine (DSPE).

The microparticles containing the desired physiologically acceptable gas may be stored immediately after formation by freezing at, for example, -80 °C in an aqueous solution. To achieve this, the particles may be snap frozen in liquid nitrogen (with or without prior wash purification to remove, for example, unincorporated microparticle components or fragments). Then, to use the particles when needed, they may be warmed up by gently raising the temperature (e.g., by hand). This applies to microparticles with or without a molecular binding element(s) attached to the surface of the shell.

The formed microparticles (with/without prior purification to remove, for example, unincorporated microparticle components or fragments) may also be stored as a lyophilisate (dry powder). For example, the lyophilisate (powder) can be stored in an enclosed vial containing a desired physiologically acceptable gas in the headspace of the vial. Then, to use the particles when needed, they may be reconstituted by instilling a suitable aqueous-based solution (e.g., normal saline, 5% dextrose, 5% glucose, phosphate buffered saline (PBS) followed by shear-mixing (e.g., shaking by hand or a machine).[4, 54]

A suitable aqueous-based solution (examples include but are not limited to, normal saline, 5% dextrose, 5% glucose, PBS) may comprise suitable additive(s), examples of the latter include but are not limited to, matrix agents (carbohydrates such as saccharide, maltose); cryoprotectants (such as propylene glycol, glycerol), buffers (sodium phosphate monobasic monohydrate, sodium phosphate dibasic heptahydrate), pharmaceutically acceptable carrier/excipient, or a combination thereof.

The present invention also concerns an intermediate microparticle composition suitable for producing a molecular imaging microparticle composition, wherein the intermediate composition comprises: (i) a phosphatidylcholine lipid; (ii) a phosphatidylethanolamine lipid comprising at least one maleimide moiety; and (iii) an alkoxylated fatty acid. Such a composition represents a stable intermediate formulation which may be used as the basis for forming or reforming microparticles, and can be easily stored, transported and then reformulated (with the required molecular binding element, where appropriate). Examples include (but are not limited to):

(a) a lyophilisate (dry powder) of the microparticle components previously mixed and dissolved in a suitable organic solvent;
(b) a lyophilisate (dry powder) of (a) previously suspended in a suitable aqueous-based solution;
(c) a lyophilisate (dry powder) of formed microparticles previously in a suitable aqueous-based solution; and
(d) an aqueous suspension (solution) of lyophilisate (a), (b), or (c) above, in a suitable aqueous-based solution.

The intermediate microparticle composition has a molar ratio of (i) to (iii) which satisfies the following ranges: (i) 70 to 80; (ii) 5 to 15; and (iii) 10 to 20.

The intermediate microparticle composition may further comprise at least one molecular binding element. In

this case, the at least one molecular binding element may be covalently attached to the at least one maleimide moiety.

In a preferred embodiment, the intermediate microparticle composition is a lyophilisate. For example, an intermediate composition may be prepared by providing a mixture comprising components (i)-(iii) in the molar ratio specified above. The components may be mixed by dissolving in a suitable solvent (e.g., chloroform) and then prepared for storage by lyophilisation. The intermediate composition may share any of the features mentioned above in relation to the microparticle composition suitable for molecular imaging. Thus, the intermediate composition may comprise the same lipids and additional components.

The intermediate composition may be prepared by providing a mixture comprising components (i) to (iii) in the molar ratio specified above. The components may be mixed by dissolving in a suitable solvent (e.g., cyclohexane:chloroform in ratio 1:2) and then prepared for storage by lyophilisation.

Lyophilisation may allow exchange of the solvent to a suitable aqueous-based solution. Microparticles may then be formed by shear-mixing (e.g., using sonication, machine or hand agitation) of the intermediate composition in the presence of a desired physiologically acceptable gas. The microparticles formed may then be prepared for storage as a dry powder by lyophilisation, with/without prior wash purification to remove, for example, unincorporated microparticle components or fragments.

The intermediate microparticle composition (with/without prior purification to remove, for example, unincorporated microparticle components or fragments) may be stored as a lyophilisate (dry powder). For example, the lyophilisate (powder) can be stored in an enclosed vial containing a desired physiologically acceptable gas in the headspace of the vial. Then, to use the particles when needed, they may be reconstituted by instilling a suitable aqueous-based solution followed by shear-mixing (e.g., shaking by hand or a machine).

In addition, there is provided a kit comprising an intermediate microparticle composition according to the invention, and a source of a fluid medium which comprises a physiologically acceptable gas. In particular, the physiologically acceptable gas may be selected from air, nitrogen, carbon dioxide, xenon, krypton, sulfur hexafluoride, chlorotrifluoromethane, dichlorodifluoro-methane, bromotrifluoromethane, bromochlorodifluoromethane, tetrafluoromethane, dibromo-difluoromethane, dichlorotetrafluoroethane, chloropentafluoroethane, hexafluoroethane, hexafluoropropylene, octafluoropropane, hexafluoro-butadiene, octafluoro-2-butene, octafluorocyclobutane, decafluorobutane, perfluorocyclopentane, dodecafluoropentane, and tetradecafluorohexane. For example, the kit may comprise an enclosed vial containing an aqueous (preferably micellar) lipid dispersion (e.g., a suspension of the lyophilisate in a suitable aqueous-based solution) with the physiologically acceptable gas in the headspace of the vial.

Alternatively, the kit may comprise an enclosed vial containing the intermediate microparticle composition as a lyophilisate (dry powder) with a physiologically acceptable gas in the headspace of the vial. When required, the microparticles may be reconstituted by instilling a suitable aqueous-based solution followed by shear-mixing (e.g., shaking by hand or a machine).

In a further aspect, there is provided a method of preparing a microparticle composition according to the invention, the method comprising: forming a core microparticle structure having a central area and a shell from components (i) to (iii). The method may further comprise covalently attaching at least one molecular binding element to the shell of the core microparticle structure.

The core microparticle structure having a central area and a shell may be formed in any suitable way. Commonly used methods for manufacturing microparticles involve, for example, high-shear mixing, such as sonication of an aqueous medium containing the shell-forming components. The solution (aqueous micellar dispersion, in case of lipids) may be sonicated with a probe-type ultrasound generator while gas is sparged through the solution. Gas is dispersed in the aqueous phase by ultrasound-induced shear. Microparticle gas particles are stabilized with the shell components immediately deposited at the gas-liquid interface. Instead of sonication, hand agitation or high-speed mixing can be used, as, for example, in dental amalgam processing. An enclosed vial with an aqueous micellar lipid dispersion and a gas headspace may be placed in an amalgamator. After a short (e.g. less than 1 minute) mixing (e.g. at approximately 2000-4000 rpm), microparticles are ready for use. Alternatively, microparticle shell components or microparticles may be prepared as dry precursors (lyophilisate). Microparticle shell components or microparticles may be codissolved or suspended, respectively, in a suitable solution with/without a suitable matrix agent (e.g. carbohydrate), placed in vials, and freeze-dried (lyophilised). Vials may then be filled with desired gas and sealed. Vials with the lyophilisate (dry powder) are very stable in storage. Immediately before use, they may be reconstituted with a suitable solution (e.g., 5% dextrose, or 5% glucose, or normal saline) and shear-mixed (e.g., shaking by hand or machine) to form the microparticles.[4, 54, 55]

Also provided is a method of preparing a microparticle composition according to the invention, the method comprising: (i) shear-mixing (e.g., by sonication, or mechanical/hand agitation as described above) a suspension of components (i) to (iii) so as to form a core microparticle structure having a central area and a shell; and (ii) covalently attaching at least one molecular binding element to the shell of the core microparticle structure.

For embodiments in which a physiologically acceptable gas is encapsulated in the central area of the microparticles, the physiologically acceptable gas may be incorporated in step (i) of the above method, preferably by conducting the step in the presence of the gas.

More specifically, the microparticles may be prepared from the intermediate microparticle composition mentioned above. As such, the lyophilisate (powder) may be resuspended with a suitable aqueous-based solution. The particles can then be formed using shear-mixing approaches in the presence of the desired gas (e.g., $C_3F_8$), by sonication, hand agitation, or machine agitation (e.g., dental amalgamator at e.g., 2000-4000 rpm for e.g. 45-60s using e.g., Wig-L-Bug Crescent Dental (IL, USA) or Espe Capmix (Espe, Germany)). The formed particles may then be washed prior to conjugation with molecular binding element. After conjugation, the particles may be washed (purified) to remove unconjugated molecular binding elements/bubble fragments etc. by spin-flotation with exchange of the subnatent with a physiologically suitable aqueous-based solution.

Microparticles may also be regenerated from the lyophilisate (powder) of previously formed microparticles with or without molecular binding elements attached. As such, the lyophilisate (powder) of the previously formed microparticles may be resuspended in a suitable aqueous-based solution. The particles may then be regenerated using shear-mixing approaches in the presence of the desired gas (e.g., $C_3F_8$), by sonication, hand agitation, or machine agitation (e.g., dental amalgamator at e.g., 2000-4000 rpm for e.g. 45-60s using e.g., Wig-L-Bug Crescent Dental (IL, USA) or Espe Capmix (Espe, Germany)).

In the case of microparticles without molecular binding elements attached, the regenerated microparticles are preferably purified to remove unincorporated microparticle components or fragments etc., prior to use or storage. The microparticles may be purified before conjugation with molecular binding elements. After the conjugation reaction, the microparticles are preferably purified to remove unconjugated molecular binding elements/bubble fragments etc., prior to storage. For use (e.g., administration into a subject), the microparticles are preferably purified first.

In the case of microparticles with molecular binding elements attached, microparticles re-formed from the lyophilisate may or may not be purified prior to use. Any suitable method can be used for the purification of microparticles. For example, washing by spin-flotation with exchange of the subnatent with a suitable solution or physiologically suitable aqueous-based solution.

The microparticles may be stored according to any of the following methods:

Storage method 1: freezing of formed (washed or unwashed) microparticles (with or without molecular binding element(s) attached). This involves snap freezing and storing the microparticles at -80 °C with or without the desired gas in the headspace of the container.

Storage method 2: lyophilisation of formed (washed or unwashed) microparticles (with or without molecular binding element(s) attached). A matrix agent (e.g., carbohydrate) may or may not be added to the formed microparticles before lyophilisation. To reform the microparticles, a suitable aqueous-based solution may be added and then shear-mixed in the presence of desired gas (sonication or preferably hand/machine shaking).

Storage method 3: lyophilisation of the lipid blend in organic solvent (thus making the initial lyophilisate) for microparticles with or without molecular binding element(s) attached. The components are dissolved and mixed in organic solvent (e.g., cyclohexane:chloroform). Then a matrix agent (e.g., carbohydrate) may or may not be added prior to lyophilisation. To form the microparticles, the lyophilisate is dissolved in a suitable aqueous-based solution, then the solution may or may not be brought up to above the chain melting temperature (Tc) of the lipids in the mixture (e.g., 60-65 °C) prior to shear-mixing in the presence of the desired gas.

It will be appreciated that the microparticles may be produced by carrying out the above steps (i) and (ii) in any order, i.e. the preparation method is not limited to carrying out step (ii) after step (i). Step (i) may be carried out first, followed by step (ii). Alternatively, step (ii) may be carried out first, followed by step (i). Thus, the molecular binding element(s) may be conjugated to the shell of the core microparticle structure via component (ii) prior to particle formation. For this, the molecular binding element(s) may be mixed with component (ii) or the lipid-blend of components comprising component (ii). In either case, non-conjugated molecular binding elements may preferably be removed. For example, the conjugated component (ii) containing the molecular binding element(s) may be purified prior to use. Also, residual maleimide moieties (i.e. those containing active maleimide function) on component (ii) may be deactivated by, for example, hydrolysis or by reaction with excess thiols. In particular, it may be preferable to use a suitable molecule with a single thiol group per molecule, e.g., L-Cysteine (purification to remove unreacted thiols may subsequently be required). Any suitable method for purification can be used, for example high-performance liquid chromatography (HPLC). Verification of the conjugated component can be by any suitable method, for example mass spectrometry, nuclear magnetic resonance, and amino acid analysis (depending on the nature of the molecular binding element(s) and/or reactants).

The conjugation reaction used to covalently attach the molecular binding element to the shell of the core microparticle structure was found to be particularly effective when the conjugation reaction ratio used in the conjugation reaction is at least 2 x $10^6$ molecular binding elements (e.g. antibody molecules) per microparticle. In some embodiments, the conjugation reaction ratio may be at least 3 x $10^6$ molecular binding elements per microparticle. Further, the conjugation reaction ratio may be at least 4 x $10^6$ molecular binding elements per microparticle. When using reaction ratios of this order, microparticles are produced which are able to sufficiently bind under flow conditions as well as static

conditions. Lower levels (e.g. approximately 1x$10^6$ molecular binding elements per particle) produce microparticles which are only able to target under static conditions.

Expressed in another way, and assuming that all of component (ii) is incorporated into the shell of the microparticles, the conjugation reaction ratio used in the reaction to conjugate the molecular binding element to the microparticle is such that the conjugation reaction molar ratio of the at least one molecular binding element to component (ii) is ≥1:1. Otherwise, the conjugation reaction molar ratio of the at least one molecular binding element to component (ii) may be ≥5:1. Further, the conjugation reaction molar ratio may be ≥8:1, ≥9:1, or ≥10:1. In some embodiments, the conjugation reaction molar ratio may be about 10:1.

It was also found that by keeping the conjugation sites on each ligand to a minimum, such as by reducing only 1 interchain disulfide bond to produce 2 -SH groups per antibody, significant particle aggregation due to cross-linking could be avoided. This can be further enhanced by deactivating any unreacted -SH groups after particle conjugation (e.g. by alkylating unreacted - SH groups).

Other features of the composition as described above are also applicable to the above method so a skilled person will appreciate that these features can also be features of the method.

In addition, there is provided a pharmaceutical composition comprising a microparticle composition according to the invention, and a pharmaceutically acceptable carrier or excipient. Such a composition may comprise, for example, (i) microparticles having a single type of molecular binding element attached to the surface of the microparticles, (ii) microparticles having more than one type of molecular binding element attached to the surface of the microparticles, or (iii) a mixture of microparticles, each having a different molecular binding element attached to the surface of the microparticles.

Pharmaceutically acceptable carriers (adjuvants or vehicles) that may be used in the pharmaceutical compositions include, but are not limited to, ion exchangers, alumina, aluminium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulphate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene- polyoxypropylene-block polymers, polyethylene glycol and wool fat.

As described herein, the microparticle composition of the invention, or a pharmaceutical composition thereof, is suitable for use as a contrast agent in molecular imaging, for example ultrasound imaging, MRI, scintigraphy, SPECT, PET, CT, X-ray imaging/fluoroscopy, fluorescence imaging, bioluminescence imaging, microscopy, optical methods, or multi-modal variants thereof. In this case, the specific microparticle composition facilitates (i) minimal non-specific retention in remote non-RES tissues not expressing the molecular moiety/moieties of interest (target molecule) following systemic or iv administration, (ii) effective real-time ultrasound molecular imaging, and (iii) acoustic quantification of the molecular moiety/moieties of interest to a high quantitative degree. Furthermore, the invention enables use of existing clinical ultrasound systems, whereby a low ultrasound-power non-linear (microparticle-specific) imaging mode (available on established clinical ultrasound systems) may be used. The low-power and real-time nature of the imaging technique is especially advantageous in terms of biosafety and the superior imaging information obtained. Continuous, multi-plane, or three-dimensional imaging with single microparticle-bolus administration is also feasible with this technology.

The microparticles, in any form, or the intermediate composition, may be sterilised by a suitable method used in the art, such as by ultraviolet or gamma irradiation.

Also provided is an imaging method comprising: administering the microparticle composition of the invention, or a pharmaceutical composition thereof, to a subject; and imaging the subject using an imaging technique. As mentioned above, the imaging technique may be one known extensively in the art, such as ultrasound imaging or MRI. Other known imaging techniques are scintigraphy, SPECT, PET, CT, X-ray imaging/fluoroscopy, fluorescence imaging, bioluminescence imaging, microscopy, optical methods, or multi-modal variants thereof.

This method allows the imaging of the microparticles which are attached to a molecular moiety of interest as a result of the molecular binding element being bound to the molecular moiety. For example, when the molecular binding element of the microparticles is an Esel antibody, after administration, the microparticles will bind to Esel (an endothelial adhesion molecule expressed on activated endothelium during inflammation) in the subject so that the imaging method allows the imaging detection for the presence/absence and location of Esel expression, as well as imaging quantification of the degree of Esel expression (and endothelial activation or inflammation) in the subject.

The method also allows the imaging of microparticles which act as negative controls or calibration for molecular imaging. For example, when the molecular binding element is absent or is a negative control or irrelevant antibody, after administration, the microparticles will not be bound to the molecular moiety of interest in the subject, so that the imaging method allows the imaging of the presence/absence, location and degree of non-specific microparticle retention in the subject, or acts as negative control for imaging interpretation, or acts as a calibration agent for the imaging signals (e.g., signal intensity vs. known amount or concentration of microparticles administered).

The subject may be any suitable subject. Preferably, the subject is a mammalian subject such as a human.

Detailed Description of the Invention

The invention will now be described in more detail by way of example only and with reference to the following Figures.

**Figure 1.** Frozen section immunohistochemistry of the mouse heart. Representative example from mice 6h post lipopolysaccharide (LPS) pre-treatment. Magnification 200x. Low power magnification (low mag) 40x.

**Figure 2.** E-selectin (Esel) targeting microparticles & *in vitro* validation. (a) A schematic representation of Esel targeting particles. (b) Morphology of Esel targeting particles. (c) Size distribution of Esel targeting particles. Each bar represents mean ±SEM, n = 5 batches of particles prepared on separate occasions. (d) *In vitro* validation of Esel targeting particles. Each bar represents mean ±SEM of attached particle density relative to that of Esel targeting particles on dish E.

**Figure 3.** *In vivo* validation of the Esel targeting microparticles. Intravital microscopy (IVM) of Esel targeting particles in the mouse cremaster. (a) Elimination of circulating-particles against time in the wild-type (WT) and Esel knock-out (KO) group of mice. Data point represents mean ±SD. (b) Accumulation of attached particles against time in 5 WT and 5 Esel KO mice. (c) Silicon intensifier target (SIT) camera images (fluorescence microscopy, magnification 200x) of attached particles in a WT (i) and Esel KO (ii) mice. (d) Quantification of attached particles on the cremaster venular wall. Each point represents one animal; bars represent group mean ±SEM. (e) Confocal microscopy of Esel targeting particles in the mouse cremaster venule. Esel in green (i, ii), particles in red (iii, iv), endothelium (PECAM-1) in grey (v, vi). Combined all 3 components (vii, viii).

**Figure 4.** Real-time ultrasound (US) molecular imaging of Esel expression in the mouse heart. (a) Sequential 14MHz Contrast Pulse Sequencing (CPS) images of the heart in end-diastole parasternal short-axis (PSA) view, in a (1) WT and (2) Esel KO mouse pre-treated with LPS, respectively. TICs of the LV cavity (from region of interest (ROI) C) and myocardium (from ROI M) for the respective animal are shown beneath; each data point represents background subtracted mean signal intensity (*I*) ±SD; suggested bolus (B), distribution (D) and elimination-phase (E) of the time signal intensity curve (TIC) are indicated. (b) PSA, parasternal long-axis (PLA) and apical 4-chamber (A4C) views of the heart at 14 and 7MHz CPS, >20min post particle administration (when freely circulating-particles have cleared from the blood pool (left ventricular (LV) cavity)). Animal, gain and MI were the same between both frequencies. Arrow indicates mid anteroseptal wall. Baseline images before particle administration are shown in Figure 6.

**Figure 5a.** Real-time US molecular imaging of Esel expression in the mouse abdomen. Combined 14MHz 'CPS-contrast only' and 'B-mode images' before (i, ii, v, vi) and ≈30min post (iii, iv, vii, viii) particle administration. Kidney (K), liver (L), spleen (S).

**Figure 5b.** Real-time US molecular imaging of Esel expression in the mouse lower abdomen. Separate 14MHz 'CPS-contrast only' and 'B-mode images' before (ix, x, xiii, xiv) and ≈30min post (xi, xii, xv, vxi) particle administration. Kidney (K), spleen (S).

**Figure 6.** Baseline images (before particle administration) for Figure 4b.

**Figure 7.** Temporal expression of Esel in the mouse heart. (a) Esel mRNA. Each data point represents one animal. Exponential line of best fit ±95% confidence interval (CI) is shown. (b) Esel cell-surface protein. Each data point represents mean ±95% CI for 5, 5, 4, 5 mice at $LPS_{Time}$ 3, 5, 8, 24h, respectively. Exponential line of best fit ±95% CI is shown. (c) Esel mRNA vs. Esel cell-surface protein.

**Figure 8.** Acoustic quantification of Esel expression in the mouse heart. Pearsons r: (a) WT = - 0.87, KO = -0.08; (b) WT = 0.84, KO = not applicable; (c) WT = -0.78, KO = -0.05; (d) WT = 0.81, KO = not applicable. Solid circle = WT, open square = Esel KO. Error bar represents SD.

## Examples

### *Experimental Details*

### Antibodies

MES-1 monoclonal antibody (mAb), a rat IgG2a,*k* against mouse Esel,[10] and its $F(ab')_2$ fragment (MES-1 $F(ab')_2$) were provided by Dr D Brown (UCB Celltech, UK). AF488-MES-1 (MES-1 labelled with 7 Alexa Fluor® 488 fluorescence dye) and reduced MES-1 $F(ab')_2$ (2 thiol (SH) groups per $F(ab')_2$ from tris(2-carboxyethyl)phosphine hydrochloride (TCEP) reduction) were prepared as described below. MEC13.3 mAb, rat IgG2a,*k* against mouse PECAM-1 (BD Biosciences). Allophycocyanin-labelled mAb against mouse PECAM-1 (BD Pharmingen). Rat IgG2a,*k* isotype negative control mAb (BD Biosciences). Biotinylated rabbit mAb against rat IgG2a (secondary antibody) (Vector Laboratories).

**(a) Reduction of MES-1 $F(ab')_2$ for microparticle conjugation.** MES-1 $F(ab')_2$ was reduced using 4 molar excess TCEP (Sigma-Aldrich): MES-1 $F(ab')_2$ (83.3μM, 8.3mg/ml) and TCEP (333.3μM, 0.096 mg/ml) in Exchange Buffer (50mM 2-(N-Morpholino)ethanesulfonic acid (Sigma-Aldrich), 2mM ethylenediaminetetraacetic acid (Sigma-Aldrich), pH 6) were incubated for 1h at 37°C under constant agitation. The reaction volume ranged 1-1.6ml. The reaction was stopped by placing on ice and immediate purification of the reduced $F(ab')_2$ using spin column gel filtration chromatography with a 5ml-Zeba Desalt Spin Column (size exclusion limit 1,000Da) according to the manufacturer's instructions (Perbio Science), at 4°C. The spin column was previously equilibrated in cold Exchange Buffer. The degree of reduction of the $F(ab')_2$ (number of SH per $F(ab')_2$) was determined spectrophotometrically using Ellman's test with Ellman's Reagent (Perbio Science) according to the manufacturer's instructions with the following modifications: the Ellman's reaction consisted of 2.5μl Ellman's Reagent (10mM, 4mg/ml), 7.5μl Exchange Buffer and 90μl of the purified reduced $F(ab')_2$ in Exchange Buffer, incubated at room temperature (rt) covered with aluminium foil to minimise light exposure; absorbance at 412nm ($A_{412}$) was measured at 24min from the start of the Ellman's reaction, to determine the concentration of SH in the reduced $F(ab')_2$ sample by reference to a standard curve of Ellman's reaction with known concentrations of SH-containing compound, L-Cysteine hydrochloride (Perbio Science) in Exchange Buffer (pH6); duplicate Ellman's 'blank' reaction where the Exchange Buffer was added in place of the reduced $F(ab')_2$ was used for baseline subtraction of $A_{412}$ from the test samples. The concentration of reduced $F(ab')_2$ was determined from $A_{280}$ in the absence of Ellman's Reagent (with the spectrophotometer zeroed using Exchange Buffer), as the latter would interfere with $A_{280}$. The degree of $F(ab')_2$ reduction was calculated as:

SH per $$\mathrm{F(ab')_2} = \frac{[\mathrm{SH\ in\ }\mu\mathrm{M}]}{[\mathrm{F(ab')_2\ in\ }\mu\mathrm{M}]}.$$ The reduced $F(ab')_2$ contained 2 SH groups per molecule of $F(ab')_2$. The purified reduced $F(ab')_2$ was kept at concentration of ≈8mg/ml (80μM) in Exchange Buffer for subsequent conjugation to microparticles.

**(b) Labelling of MES-1 mAb with Alexa Fluor® 488 (AF488) fluorescence dye for confocal microscopy.** MES-1 was labelled with Alexa Fluor® 488 carboxylic acid, 2,3,5,6-tetrafluorophenyl ester, 5-isomer (Molecular Probes) as follows. A 100μl-labelling reaction mixture consisting of 50μM MES-1 mAb (25.7μl at 194μM or 29.1mg/ml in phosphate buffered saline (PBS) pH 7.5), 750μM AF488 (6.6μl at 11.3mM or 10mg/ml in water), 2.6μl (1/10$^{th}$ volume of MES-1 mAb added) 1M NaHCO3 pH 8.5 and 65.1μl distilled $H_2O$, was incubated at rt for 1h, with gentle manual agitation at 30min. Controls included no dye or no MES-1 mAb. AF488-labelled MES-1 was then purified from the reaction mixture and suspended in PBS (pH 7.5), using gel-filtration chromatography spin column with 6kDa size exclusion limit (Bio-Spin P-6 Column with SSC packing buffer, BioRad) according to the manufacturer's instructions with the following specific conditions: the Bio-Spin column was buffer exchanged first with PBS using 3 wash cycles; all centrifugations were carried out at 20°C. The concentration and degree of labelling of AF488-labelled MES-1 was determined using a spectrophotometer. Samples were diluted in PBS to 100μl volume in duplicates, and absorbance measured at 280nm ($A_{280}$) and 495nm ($A_{495}$). The concentration of the labelled mAb was calculated as: [mAb] in $$\mathrm{mg/ml} = \frac{A_{280} - (A_{495} \times 0.11)}{1.4} \times \mathrm{dilution\ factor},$$ where 0.11 is the correction factor for AF488's contribution to $A_{280}$. The concentration of the mAb in mg/ml was converted to μM using: [mAb] in

$$\mu M = \frac{[mAb] \text{ in mg/ml}}{150000} \times 10^6,$$

where 150,000 is the molecular weight of mAb in Da, $10^6$ is the multiplication factor for converting M to μM. The concentration of AF488 was calculated as: [AF488] in

$$\mu M = \frac{A_{495}}{71000} \times \text{dilution factor} \times 10^6,$$

where 71,000 is the approximate molar extinction coefficient of AF488 dye (in $cm^{-1}M^{-1}$) at 494nm, $10^6$ is the multiplication factor for converting M to μM. Finally, the degree of labelling was calculated as:

$$\text{Degree of labelling} = \frac{[AF488] \text{ in } \mu M}{[mAb] \text{ in } \mu M}.$$

Aluminium foil was used at all stages to minimise light exposure. Under this protocol, unreacted AF488 was retained in the column, confirmed by the 'no MES-1 mAb' control reaction. The labelling reaction molar ratio of 15:1 (AF488:MES-1) yielded MES-1 labelled with 7 AF488 molecules each, labelling efficiency = 46%, yield ≈89%. The purified AF488-labelled-MES-1 (6.748mg/ml, 44.984μM) was aliquoted, wrapped in aluminium foil and store at -20°C until use. The preservation of sensitivity and specificity to Esel of the AF488-labelled MES-1 was confirmed using immunohistochemistry on frozen heart sections of wild-type (WT) and Esel knock-out (KO) mice pre-treated with lipopolysaccharide (LPS).

**Microparticle Preparation**

Native (unconjugated) maleimide-functionalised lipid-shelled octafluoropropane ($C_3F_8$) microparticles were prepared by sonication of an aqueous suspension containing 1,2-distearoyl-*sn*-glycero-3-phosphocholine (DSPC; Avanti Polar Lipids, AL), 1,2-distearoyl-*sn*-glycero-3-phosphoethanolamine-N-(maleimide(polyethylene glycol)-2000) (DSPE-$PEG_{2000}$-Mal; Avanti Polar Lipids), mono-stearate poly(ethylene)glycol ($PEG_{40}$ stearate; Sigma-Aldrich), and fluorescent dye 1,1'-dioctadecyl-3,3,3',3'-tetramethylindocarbocyanine perchlorate (Dil; Molecular Probes) at 75:9:14:2 molar ratio, in the presence of $C_3F_8$. To make Esel targeting particles, TCEP-reduced MES-1 $F(ab')_2$ containing 2 SH groups per $F(ab')_2$ were grafted to the shell outer-surface of these native particles by maleimide-thiol conjugation. The conjugation reaction ratio was $4.338 \times 10^6$ $F(ab')_2$ molecules per particle (7.2nmol $F(ab')_2$ per $10^9$ particles). (NB. The total number of DSPE-$PEG_{2000}$-Mal molecules in the aqueous suspension / total number of unwashed particles produced from the aqueous suspension = $4.338 \times 10^6$. If ≤10% of the components in the aqueous suspension were incorporated onto the particle shell, and the molar ratio of the components on the shell remained close to that of the initial aqueous suspension,[56] then a particle of population mean size would contain ≤$4.338 \times 10^5$ maleimide molecules, and the estimated $F(ab')_2$:maleimide conjugation reaction molar ratio would be ≥10:1). The conjugation reaction was carried out for 30 minutes (min) at 4°C, near neutral pH, under $C_3F_8$ atmosphere with constant gentle agitation; the reaction was terminated by adding excess N-Ethylmaleimide (NEM) to quench any unreacted SH. Particles were washed with cold degassed normal saline using multiple cycles of centrifugation flotation under $C_3F_8$ atmosphere at 4°C before and after particle conjugation, to remove unincorporated components and particle fragments. Freshly prepared particles were immediately divided into 20-50μl aliquots, capped and sealed with parafilm (American National Can), then snap frozen in liquid nitrogen and stored at -80°C until use. The concentration of subsequently thawed Esel targeting particles ranged 1-3x$10^9$ particles/ml amongst 5 batches prepared at different times.

Detailed experimental conditions are as follows. Native (unconjugated) microparticles were prepared by dispersing DSPC, DSPE-$PEG_{2000}$-Mal, $PEG_{40}$ stearate and Dil at molar ratio of 75:9:14:2 in a small amount of cyclohexane:chloroform solvent (1:2), in a 50ml round-bottomed flask. Excess solvent was extracted using a stream of gaseous nitrogen. The lipid was then transferred to a freeze dryer and lyophilised to full dryness under a reduced atmosphere (e.g., $1.3 \times 10^4$ Pa) at -78.5°C (using a jacket of dry ice). The dry powder (lyophilisate) was then dispersed in a suitable aqueous-based solution (e.g., normal saline or normal saline containing 0.01% propylene glycol (PGNS: propylene glycol 103.5mg/ml, glycerol 126.2mg/ml, NaCl 6.8mg/ml, pH ≈7.4)), to a concentration of 4mg/ml, homogenised by sonication in an ultrasonic bath at 60-65°C until transparent. Once fully dissolved, the solution was gently sparged with $C_3F_8$ gas (F2 Chemicals). Microparticles were then formed using a shear- mixing approach, by sonic dispersion of $C_3F_8$ using a sonicator (Misonix 3000, QSonica, CT). The probe tip was positioned about 2mm into the solution and sonication was performed with a high-intensity ultrasound horn (20-21 kHz) for 30-60s at an acoustic power of approximately 120W with an initial temperature of approximately 60°C. More $C_3F_8$ gas was sparged into the microbubble dispersion, and the vessel capped and immediately plunged into ice cold water (3min) to dissipate the heat generated during the sonication process. Microparticles produced were washed (purified) by centrifugation flotation at 1,000g 4°C for 15-25min, using a Beckman Coulter Allegra X-15R Centrifuge (Beckman Coulter): particles float to the top of the sample vial after centrifugation, the subnatent was removed and replaced with equal volume of cold degassed normal saline (pH 7.4).

The wash step was repeated 7 times to remove unincorporated shell components and particle fragments. To produce Esel targeting microparticles, these washed native microparticles were added to reduced MES-1 F(ab')$_2$ whilst mixing (each reduced F(ab')$_2$ molecule contained 2 SH groups, prepared as described above). The conjugation reaction ratio used to produce the successful Esel targeting particles was $4.338 \times 10^6$ F(ab')$_2$ molecules per particle. The concentration of particles and F(ab')$_2$ in the conjugation reaction mixture ranged 5-8x$10^9$/ml and 35-60uM (3.5-6mg/ml), respectively. The reaction mixture contained approximately 2/3 volume of Exchange Buffer (pH 6) from the reduced F(ab')$_2$ and 1/3 volume of normal saline (pH 7.4) from the washed particles. The conjugation reaction was incubated at 4°C for 30min, continuously mixed gently on a vertically tilted rotating wheel. Particle conjugation was terminated by adding 80mM NEM (Sigma-Aldrich) dissolved in dry dimethyl sulfoxide (DMSO, Sigma-Aldrich) at 20 molar excess to F(ab')$_2$ - the reaction mixture was incubated at 4°C for 30-60min on the rotator. Typically, the concentration of NEM and DMSO in the reaction mixture was $\approx$1mM and $\leq$1.7% v/v, respectively. The particles were then washed 4 times with cold normal saline by centrifugation flotation as described above, at 160g 4°C for 5min. This removed unincorporated F(ab')$_2$, unreacted NEM, DMSO and particle-fragments. To minimise particle loss, all washes and incubations were performed with the particle concentrations kept high ($\geq 1 \times 10^9$ particles/ml), under $C_3F_8$ atmosphere (to reduce the concentration gradient for diffusion of $C_3F_8$-gas out of the particles) and at 4°C (to reduce the rate of gas diffusion). To preserve the particles Dil fluorescent dye, the Dil compound, lyophilisate or particles were protected from light.

**Microparticle Morphology, Concentration, Size Distribution and Charge Analysis** Particle integrity (spherical morphology, absence of aggregation) was examined under microscopy with the particles placed in a haemocytometer (Reichert Bright-Line Metallized Hemocytometer, Hausserscientific, PA) at e.g., 1:200 dilution in cold normal saline. Particle concentration and size distribution were determined by electrozone sensing in a Coulter Multisizer IIe equipped with a 30$\mu$m-diameter orifice counting tube (Coulter Electronics), according to the manufacturer's instructions. The set-up allowed size detection range 0.72-18$\mu$m, resolution 0.09$\mu$m. For particle charge analysis, the particles were dispersed in 1ml of 1mM KCl (pH 7.4) at $\approx 10^7$ particles/ml. Its net charge was determined as the zeta potential by light scattering in a Zetasizer Nano ZS (Malvern Instruments), according to the manufacturer's instructions.

## Targeting Microparticle Binding Assay *In Vitro*

100$\mu$l of Esel targeting or non-targeting (native) particles at $2.5 \times 10^7$ particles/ml were placed on inverted polystyrene petri-dishes coated with 200$\mu$l of recombinant homodimeric mouse Esel protein (R&D Systems) at 7nM (dish E), or on Esel coated dishes previously blocked with 500$\mu$l of excess MES-1 F(ab')$_2$ at 67nM (dish B), or on non-coated dishes where phosphate buffered saline pH 7.5 (PBS) was used instead of Esel for dish coating (dish P). Unattached particles were gently washed off after 1min. The dishes were then re-filled with cold degassed PBS for immediate examination under an upright light microscope equipped with immersion objective lens. The number of particles attached on each dish was counted and averaged from 10 random optical fields (OFs) to determine the attached particle density.

The detailed methodology is as follows. Polystyrene petri-dish (Corning® 35mm Not TC-Treated Culture Dish, Corning Life Sciences) was coated with 200$\mu$l recombinant homodimeric mouse Esel protein (R&D Systems) at 1.25$\mu$g/ml (7nM, diluted in phosphate buffered saline pH 7.5 (PBS)) for 1h at rt (dish E). PBS was used instead of Esel as 'blank' negative control (dish P). Non-specific binding sites were then blocked with 4ml bovine serum albumin (BSA, Sigma-Aldrich) at 2.5% w/v in PBS for 2h at rt. BSA was then discarded and the dish washed with PBS. To prepare Esel coated dish blocked with excess MES-1 F(ab')$_2$ (dish B), 500$\mu$l MES-1 F(ab')$_2$ at 6.67$\mu$g/ml (67nM) was placed in dish E for 30min at rt, then washed with PBS. Due to the buoyancy of the particles, the dishes were inverted for incubation with particles for Imin at rt: 100$\mu$l Esel targeting or non-targeting (native) particles at $2.5 \times 10^7$ particles/ml (diluted in cold degassed PBS) were used. Unattached particles were then gently washed off and the dishes re-filled with cold degassed PBS for immediate examination using an upright light microscope equipped with immersion objective lens, connected to a camera and monitor. The number of particles attached to each dish was counted and averaged from 10 random optical fields (OFs) on the monitor display, the surface area of the latter determined using a stage micrometer. The attached particle density was thus determined.

## Animals

Wild-type (WT) mice: adult male C57B16/Jax (Charles River, UK). Esel knock-out (KO) mice: adult male Esel homozygote KO on C57B16 background,[57] bred locally from mice donated by Dr K Norman and Prof P Hellewell (University of Sheffield, UK). All the animal work was carried out under Project Licences and Personal Licences granted by the Home Office under the Animals (Scientific Procedures) Act 1986; ethical approval was additionally obtained from the local Ethical Review Panel.

**Lipopolysaccharide (LPS) mouse model (experimental endotoxaemia)**

WT and Esel KO mice were pre-treated with 50μg LPS from *E Coli* 0111:B4 (Sigma-Aldrich), made up to 200μl volume in normal saline, by *intraperitoneal* (*ip*) injection to induce systemic inflammation. Systemic administration of LPS by *ip* injection produces systemic inflammation, which includes induction of Esel expression in multiple organs including the heart and kidneys.[58, 59] This animal model differs from others used in particle-targeting (such as ischaemia-reperfusion injury, heart transplant rejection, thrombosis, chronic-ischaemia/tumour angiogenesis animal models) in that: (i) it does not require surgical procedures which may introduce confounding variables such as surgical trauma or blood clots; (ii) the particles target molecules present in multiple organs (not just one), uniquely allowing assessment of targeting particle specificity in multiple organs simultaneously, and assessing the imaging technique's ability to detect target molecule in a tissue of interest in the presence of 'particle steal' by other tissues; and (iii) the target molecule expression in the myocardium was essentially global and uniform, uniquely allowing the study of targeted particle signal attenuation. Additionally, the combination of Esel being solely expressed on activated endothelial cells and its essentially global uniform expression in the myocardium of LPS pre-treated mice, makes the LPS mouse model-Esel combination an ideal *in vivo* model for testing acoustic quantification of molecular expression.

**Immunohistochemistry**

Immunohistochemistry was performed on acetone-fixed cryosections of freshly harvested hearts of WT (with/without LPS pre-treatment) and Esel KO (pre-treated with LPS) mice. After blocking non-specific binding sites with 100μl of 1:1000 rabbit serum (Sigma-Aldrich) for 1 hour (h) at room temperature (rt), sections were incubated for 1h at rt with 100μl of 0.01mg/ml primary antibody: MES-1 (for Esel), MEC13.3 (for PECAM-1, endothelial marker) or isotype negative control. Each section was then incubated with 100μl of 0.005mg/ml biotinylated secondary antibody for 60min at rt. After blocking of endogenous peroxidase with 0.3% $H_2O_2$ methanol for 20-30min at rt, the horseradish peroxidase-based detection system, Vectastain ABC kit (Vector Laboratories), was used with 3,3'-Diaminobenzidine solution (SIGMA*FAST*™ DAB tablet, Sigma-Aldrich) as the chromagen substrate. Sections were counterstained using Harris Modified Hematoxylin Solution (Sigma-Aldrich) and 1% $NaHCO_3$, then dehydrated through 70-100% ethanol, dried and mounted with Histomount (VWR), and examined under light microscopy. The duration between the time of LPS pre-treatment and sacrifice of the animal for immediate tissue harvesting was noted as the $LPS_{Time}$.

**Reverse Transcriptase - Real Time Quantitative Polymerase Chain Reaction (RT-qPCR).**

WT mice were pre-treated with LPS as described above. The duration between the time of LPS pre-treatment and sacrifice of the animal for immediate tissue harvesting was noted as the $LPS_{Time}$. Freshly harvested tissues were kept in RNAlater® solution (Ambion) to preserve ribonucleic acid (RNA) in-situ; total RNA was subsequently extracted using TRIzol reagent (Invitrogen) according to the manufacturer's instructions. First-strand complementary deoxyribonucleic acid (cDNA) synthesis was then performed using the Qiagen Omniscript® Reverse Transcription kit (Qiagen) according to the manufacturer's instructions. This was followed by real-time qPCR with the SYBR®Green detection method for Esel and hypoxanthine phosphoribosyltransferase-I (HPRT-I), carried out on a 96-well plate in the iCycler™ (iCycler iQ Real-Time PCR Detection System, Bio-Rad) according to the manufacturer's instructions. All PCR reactions were carried out in triplicate wells on the same plate. The primer sequences were: Esel forward primer 5'-CTCATTGCTCTACTTGTTGATG-3', Esel reverse primer 5'-GCATTTGTGTTCCTGATTG-3', HPRT-I forward primer 5'-ATTAGCGATGATGAACCAG-3', HPRT-I reverse primer 5'-AGTCTTTCAGTCCTGTCCAT-3'. For data analysis, the threshold cycle (*Ct*) was determined from the amplification plot using the iCycler™ iQ Optical System Software Version 3.0a (Bio-Rad). As PCR efficiency of the Esel and HPRT-I primer pairs differed by ≤5% (93 ±4% and 92 ±3% (mean ±SD), respectively; n = 4 each), comparative *Ct* method was used to estimate the amount of Esel messenger (mRNA) relative to that of HPRT-I, using the formula: Esel mRNA (%HPRT - 1) = $2^{-\Delta Ct}$, where $\Delta Ct = Ct_{Esel} - Ct_{HPRT-I}$, subscripts refer to the gene of interest. Mean of the replicates was used and plotted against $LPS_{Time}$ for each animal.

Further details of the methodology are as follows. The yield of total RNA from the mouse heart was typically ≈1μg pure RNA per 1mg tissue, kept at concentrations over ≈1mg/ml in molecular grade (RNase-free) $H_2O$ (Sigma-Aldrich). The RT reaction mixture for first-strand cDNA synthesis consisted of 1μg total RNA, 2μl 10x buffer RT, 2μl deoxyribonucleotide triphosphate (dNTP) mix (5mM each 2'-deoxyadenosine 5'-triphosphate (dATP), 2'-deoxycytidine 5'-triphosphate (dCTP), 2'-deoxyguanosine 5'-triphosphate (dGTP), 2'-deoxythymidine 5'-triphosphate (dTTP), 1μ (4 units) Omniscript reverse transcriptase, 2μl (1μg) oligo$(dT)_{12-18}$ primer (Invitrogen) and molecular grade $H_2O$ made up to a total reaction volume of 20μl, incubated for 1h at 37°C. qPCR was carried out in a 25μl-reaction volume in each well of a 96-well 0.2ml thin-wall PCR plate (Bio-Rad) covered with an Optical Quality Sealing Tape (Bio-Rad). The qPCR reaction mixture consisted of 5μl cDNA template (1:50 water dilution of the finished RT reaction), 0.5μl (10μM) each of the forward and reverse primer for the respective gene (see text for primer sequence; the primers were custom ordered

from Invitrogen), 6.5μl molecular grade $H_2O$ and 12.5μl iQ™ SYBR® Green Supermix (Bio-Rad). The qPCR cycling condition was: initial 3min denaturing step at 95°C (Well Factor analysis in first 90s); then 40 cycles of 15s at 95°C, 1min at 56°C; melt-curve analysis in 0.5°C steps (lmin denaturation at 95°C, 1min reset at 56°C, then 80 cycles of 10s at 60°C with 0.5°C increment for each cycle); final cooling step at 4°C. Esel and HPRT-I were amplified on the same plate for each animal; notemplate negative control using molecular grade $H_2O$ in place of cDNA template for both primer pairs were included in all plates. For data analysis, wells with abnormal amplification plot or melt-curve were excluded.

**Intravital Microscopy (IVM) Set-up**

Inflammation of the cremaster muscle was produced by intrascrotal injection of 50ng recombinant IL-1$\beta$ (R&D Systems) 2h before surgery in WT and Esel KO mice. The tail vein was cannulated with a 24G 0.7x19mm intravenous (*iv*) catheter (dead space ≈50μl) (BD Medical). Long duration anaesthesia was achieved using intraperitoneal (ip) injection of 200-300μl mixture containing 1mg/ml xylazine (Rompum, Bayer) and 10mg/ml ketamine hydrochloride (Ketalar, Parke-Davis) in normal saline. The animal was placed on a custom-built thermo-controlled (37°C) IVM stage. Under a dissection microscope, the right or left testis was gently exteriorised through a scrotal incision. A longitudinal incision was made along the cremaster muscle, which was then spread out and pinned down across a translucent microscopy stage. The exteriorised muscle was maintained by continuous super-fusion of thermo-controlled Tyrode's Salt buffer solution (9.6g Tyrode's Salts (Sigma-Aldrich) + 1g sodium hydrogen carbonate, made up to 1L volume with sterile distilled water). Observations and recordings were made using an upright microscope (Axioskop, Carl Zeiss) equipped for bright field and fluorescence microscopy, with 20x and 40x immersion objective lens (Water Achroplan, Carl Zeiss), a charge coupled device (CCD) camera (Color Chilled 3CCD Camera with controller, Hamamatsu Phototonics), a silicon intensifier target (SIT) camera (C-2400-08, Hamamatsu Photonics), a monitor (Triton, Sony), a S-VHS recorder (Model AG 6730 SVHS 625, Panasonic) and a Personal Computer. To compare shear rates against particle attachment between WT and Esel KO animals, an Optical Doppler Velocimeter (Microcirculation Research Institute, Texas A&M University, Texas), was used to measure microvascular centreline red blood cell velocities ($V_{rbc}$) in 20-40μm diameter venules of the WT and KO mice, before particle injection. 5 random vessel-segments were measured per animal.

**IVM of Esel Targeting Microparticles in the Mouse Cremaster**

$1.5 \times 10^7$ Esel targeting particles in 100μl normal saline were administered as a rapid iv bolus through a tail vein catheter, immediately followed by a 100μl normal saline flush, for intravital microscopy assessment in the cremaster of anaesthetised (xylazine/ketamine mixture *ip*) WT and Esel KO mice. All animals were pre-treated with 50ng recombinant IL-1$\beta$ (R&D Systems) intrascrotally, 2 hours (h) before exteriorization of the muscle, to induce cremaster inflammation. Observations were made using an upright microscope equipped for bright field and fluorescence microscopy, with 20x and 40x immersion objective lens, CCD and SIT cameras. To determine the shear rates against particle attachment, $V_{rbc}$ were measured in 20-40μm diameter venules from 5 random vessel-segments per animal, using an Optical Doppler Velocimeter before particle injection. All recordings were made using a S-VHS recorder and Personal Computer. All animals were sacrificed at the end of the experiment. See above for details of the set-up. Blood flow and particles were assessed over several OFs encompassing a number of different vessels under bright field and fluorescence microscopy. The number of freely circulating-particles in an OF on the monitor were counted over 10s under fluorescence microscopy at 5, 7, 10 ± 15min after particle injection. The accumulation of attached particles (defined as not moving for >3s) in an OF field were assessed for up to 15min post particle injection. The number of particles attached to 20-40μm diameter venules were counted at ≈5min after particle injection (when freely circulating-particles were absent/minimal) from 1-5 400μm length segments per venule, 2-6 venules per animal. In some animals, the attached particles in the same OF were followed-up for up to 90min using intermittent combined bright field and fluorescence microscopy, looking for the presence/absence of transmigration into the tissue interstitium or cellular internalisation. For analysis, the density of attached particles was expressed as the number of particles per vessel surface area (VSA), where $VSA\,(\mathrm{mm}^2) = \pi D(mm) \times L(mm)$, $D$ and $L$ were the vessel segment diameter and length, respectively. The attached particle density for each venule was taken as the mean of its segments, and that for each animal was taken as the mean of its venules. Shear rate was estimated from $V_{rbc}$ as: Shear rate $(\mathrm{s}^{-1}) = 8\frac{V_b\,(\mathrm{cm/s})}{D\,(\mathrm{cm})}$, where $V_b\,(\mathrm{cm/s}) = \frac{V_{rbc}\,(\mathrm{cm/s})}{\alpha}$, $V_b$ is the mean bulk velocity, $\alpha$ the factor converting $V_{rbc}$ to $V_b$, taken as 1.6 for Poiseuille flow in veins.[60] The shear rate for each animal was taken as the mean of the 5 random venule segments sampled.

**Confocal Microscopy of Esel Targeting Microparticles in the Mouse Cremaster**

1.5x$10^7$ Esel targeting particles were administered to WT and Esel KO mice pre-treated 2.5h beforehand with 50ng IL-1β intrascrotally, using methods described for IVM above. 15min later, a rapid *iv* bolus of 150μl cocktail containing 50μg AF488-MES-1 (for Esel) and 25μg allophycocyanin-labelled mAb against mouse PECAM-1 (endothelial marker) in normal saline was administered *iv,* followed by a 100μl normal saline flush. After a further 15-20min, animals were given terminal anaesthesia using *iv* bolus of a xylazine/ketamine mixture, followed by perfusion with PBS to remove unattached particles and mAb in the circulation. Immediately thereafter, the cremasters were harvested and fixed in 4% paraformaldehyde PBS for confocal microscopy (z-stacked). 3 different fluorescence, Dil (particles), Alexa Fluor® 488 (Esel) and allophycocyanin (PECAM-1) were scanned in series at each depth before moving on to the next depth in the z-axis, using an upright confocal laser-scanning microscope (LSM 5 PASCAL, Carl Zeiss) with a 40x immersion objective lens. Images were processed using Zeiss LSM 5 Image Browser software.

Further details of the methodology are as follows. Following the *iv* administration of Esel targeting particles and antibody cocktail containing AF488-MES-1 + allophycocyanin-labelled mAb against mouse PECAM-1, unattached particles and mAb in the circulation were removed by perfusion with PBS. This was achieved by exposing the heart and upper abdomen by dissection. A snip incision was then made in the right atrium followed by injection of PBS into the LV cavity using a needle connected to a 20ml-syringe. This allowed the PBS to perfuse the body, it and the blood left the circulation via the incision in the right atrium. Adequate PBS perfusion was assumed when the liver turned pale due to the replacement of blood by PBS. The cremaster muscle was then harvested immediately, spread out and fixed in 4% paraformaldehyde PBS solution for 30min at rt, then placed in cold PBS at 4°C for 5min. Aluminium foil was used to minimise light exposure to the tissue. Fresh tissues were examined immediately under confocal microscopy (z-stacked), using an upright confocal laser-scanning microscope (LSM 5 PASCAL, Carl Zeiss) with a 40x immersion objective lens (Water Achroplan, Carl Zeiss). 3 different fluorescence, Dil for particles (excite at 543nm, detect at 560-615nm), Alexa Fluor® 488 for Esel (excite at 488nm, detect at 505-530nm) and allophycocyanin for PECAM-1 (excite at 633nm, detect at 650nm) were scanned in series at each depth before moving on to the next depth in the Z-axis. Images were processed using accompanying software, Zeiss LSM 5 Image Browser.

**Ultrasound (US) Imaging**

WT and Esel KO mice were all pre-treated with LPS, tail vein cannulated and anaesthetised with xylazine/ketamine mixture as described above. The chest, abdomen and pelvis were then shaved and the animal placed supine. ECG electrode pads (Ambu® Blue Sensor P, Ambu) were applied to the paws and connected to the US machine (Acuson Sequoia® 512 US system, Siemens, CA) equipped with 'Small Animal ECG Filter'. A layer of warm gel (Gel for ultrasonic & electrical transmission, Henleys Medical) was coupled between the skin and US transducer (15L8-s linear array transducer, foot print 26mm, Siemens). US settings used were: 14MHz (P14MHz, spatial resolution ≈0.2mm) Contrast Pulse Sequencing (CPS) mode, transmission power 9dB giving low mechanical index (MI) 0.22-0.26 estimated by the scanner, dynamic range 55*dB,* time gain 0%, CPS gain 8, fundamental 2D gain 15dB, colour map M:3 (particle signal presented in heated object scale ('CPS-contrast only' images), tissue signal in grey scale ('B-mode' images)), TEQ was not used. Before particle injection, baseline parasternal short axis (PSA) view at the papillary muscle level, parasternal long axis (PLA) and apical 4-chamber (A4C) views of the heart with and without 'regional expansion selection' (RES; giving magnified images with enhanced resolution) were recorded as 3s-digital clips. Thereafter, imaging was maintained in the PSA view with the transducer fixed in position using a free standing clamp. A stopwatch was then started and 1x$10^8$ Esel targeting particles (in 100μl volume made up with normal saline) injected at 10s via the tail vein catheter as a rapid iv bolus over 1-2s, followed by a 100μl-normal saline flush over 1-2s at 20s. Continuous US insonation was applied without pausing from time 0-1min 23s on the stop-watch, then paused, then resumed only for 3s each time for digital image acquisition. 3s-digital clips (RES activated) of the heart containing several consecutive cardiac cycles were recorded at 10s and 13s, then at 10s intervals from 20s-1min 20s, then at 1min intervals from 2min20s-10min20s, then at 2min intervals from 12min20s-30min20s, then at 5min intervals until 60min20s on the stopwatch (image acquisition was stopped earlier if particle contrast enhancement in the left ventricular (LV) cavity (central blood pool) was no longer visible). Unmagnified (non-RES) images of the thorax containing the heart in the PSA view and surrounding tissues were recorded at ≈5min intervals. Other views of the heart (PLA and A4C views) were acquired at the end. In some animals, 7MHz (P7MHz, spatial resolution ≈0.4mm) CPS imaging at MI 0.22 (gain and other settings kept the same as 14MHz imaging) was also acquired at baseline and the end of the 14MHz imaging study. When switching from 14MHz to 7MHz CPS imaging, the transmit power was first reduced from -9dB to -19dB before reducing the US frequency, to avoid an increase in MI (up to ≈0.7) causing inadvertent particle destruction. In some animals, imaging of other tissues in the thorax, abdomen and pelvis were performed in the antero-posterior projection at baseline and at the end of the cardiac imaging study, to look for particle retention in extra-cardiac tissues. To do this, the probe was positioned transversely and moved slowly caudally from just below the neck to the pelvis during 3s-image acquisitions; non-RES images

in 14 and 7MHz CPS were acquired. All animals received only one dose of particles to avoid carry-over effect from previous particle dosing (e.g., blocking of Esel binding sites). At the end of imaging, animals were sacrificed and tissues immediately harvested for frozen section immunohistochemistry and qRT-PCR as described above.

**Time-signal Intensity Curve (TIC) Generation**

Videodensitometric method was used to quantify particle signal intensity off-line, using the YABCO© software (LLC Charlottesville, Virginia). End-diastolic image frames of the heart in the PSA view ('CPS-contrast only' images) were selected and aligned, those that could not be aligned (e.g., due to large movement artefact) were excluded. Regions of interest (ROIs) were placed on the mid-anterior wall of the myocardium (M) and adjacent region in the LV cavity (C), as shown in Figure 4a. These regions were chosen because they were consistently least or minimally affected by US attenuation in all animals. The video signal intensities (VI) were 'linearised' by log-decompression using the formula: Linearised $VI = 255\times10^{(\frac{VI-255}{255}\times\frac{dB}{20})}$, where *dB* is the dynamic range (55*dB* in this study). Linearised VI (*I*) was expressed in arbitrary acoustic units (AU). *I* of several end-diastolic image frames within the 3s-recording period at each time point were averaged, then corrected for background noise by subtracting away average *I* of the baseline images (images before particle administration) in the respective animals. TICs of the myocardium (tissue) and LV cavity (central blood pool) were constructed by plotting background-subtracted *I* of the myocardium and LV cavity, respectively, against time post particle administration.

**Acoustic Quantification of Esel Expression and Particle Half-Life *In Vivo***

Baseline-subtracted bubble signal intensity in the myocardium at 20min 10s post microparticle administration (R20), obtained as per TIC above, was used for analysis. An alternative novel method based on quantitative analysis of the TIC (TIC-based method) was also used to quantify the level of Esel expression. The TIC-based method also allowed other variables to be determined simultaneously, including the retained- and circulating-microparticle half-life *in vivo.* The $LPS_{Time}$ for US molecular imaging was taken as the duration between the time of LPS pre-treatment and administration of the targeting microparticles. The mean heart rate (HR) for each animal was calculated from all HRs recorded at different time points during cardiac imaging.

**Statistics**

Pearson correlation, linear or non-linear regression analysis was performed as indicated. Student's t-test or ANOVA with Turkey's post-hoc analysis was used for significance testing where indicated, with $p < 0.05$ taken as significant.

***Results***

**Esel Expression**

Frozen section immunohistochemistry showed that Esel was expressed in the heart of all WT mice pre-treated with LPS (n = 35, $LPS_{Time}$ = 4-9h). The spatial distribution was essentially uniform throughout the myocardium but limited to the post-capillary venules and capillaries. Esel was not detectable in the negative controls: WT mice not treated with LPS (n = 5), and Esel KO mice pre-treated with LPS (n = 10, $LPS_{Time}$ = 4-7h), Figure 1.

**Quantification of Esel expression by qRT-PCR.** RT-qPCR showed that the concentration of Esel mRNA in the heart decreased exponentially with time after ≈3h post LPS pre-treatment, reaching very low levels by ≈9h (n = 42, $LPS_{Time}$ = 3.2-15.7h), Figure 7a. This trend was similar to that of the cell-surface Esel protein concentration (expressed as % injected dose of radioactivity/g tissue (%ID/g tissue)) determined using *iv* radio-labelled mAb by Eppihimer *et al.*[58] using the same strain, sex, and age of mice, as well as the same dose and route of LPS administration (n=19), Figure 7b.

From the best-fit curves of Esel mRNA concentration *vs.* $LPS_{Time}$ (mRNA (in %HPRT - I) = $3600e^{-0.7LPS_{Time}(\text{in hours})}$, $R^2$ = 0.75) and that of Esel cell-surface protein concentration *vs.* $LPS_{Time}$ (protein (in % ID/g tissue) = $1.21e^{-013LPS_{Time}(\text{in hours})}$ +0.07 , $R^2$ = 0.88), using $LPS_{Time}$ as the common denominator, an empirical formula describing the relationship between the concentration of Esel mRNA and cell-surface protein could be derived as:

$$\text{Protein (in \% ID/g tissue)} = 1.21\left(\frac{\text{mRNA (in \%HPRT - I)}}{3600}\right)^{\frac{0.13}{0.7}} + 0.07$$

, Figure 7c. Thus the Esel cell-surface protein concentration was predictable from its mRNA concentration in the heart, in this LPS mouse model. Within the mRNA or protein concentration range (55-238% HPRT-I or 0.63-0.80% ID/g tissue, respectively) used for US quantification of Esel expression in this study (see later), the relationship between the concentration of mRNA and cell-surface protein was approximately linear, allowing direct use of the mRNA concentration as a surrogate quantifier for the cell-surface protein concentration (the latter being the actual target of the targeting microparticles).

**Esel Targeting Microparticles**

A schema of the Esel targeting particles engineered is shown in Figure 2a. These particles showed spherical morphology; particle-particle aggregation or cross-linkage was minimal, Figure 2b. The particle size distribution was reproducible amongst 5 batches prepared on separate occasions; particle diameter = 2.2(mean) $\pm$0.2(SEM) $\mu$m, 98.6% or 100% of the particles were under 6 or 10$\mu$m in diameter, respectively, Figure 2c. The particles (washed) had a near neutral charge, zeta potential approximately 5mV at pH 7.4.

The particles were sufficiently echogenic, stable, lacked non-specific binding, and produced no immediate adverse effects *in vivo*. Particles made using other compositions did not exhibit all of these desired properties. The suitable native particles also contained enough maleimide groups for conjugating sufficient targeting elements onto the particle surface for efficient target binding under flow conditions. The conjugation reaction ratio used to produce the successful Esel targeting particles was $4.338 \times 10^6$ F(ab')$_2$ molecules per particle. Lower F(ab')$_2$:particle reaction ratio $1 \times 10^6$:1 produced particles that could only attach to target under static conditions. It was also found that by keeping the conjugation sites on each binding element to a minimum (e.g., reducing only 1 interchain disulfide bond to produce 2 SH per F(ab')$_2$), and inactivating any unreacted ones after particle conjugation (e.g., alkylation of unreacted SH), significant particle aggregation due to cross-linking could be avoided. Particles targeting other molecules can be readily made in the same way by substitution with suitable targeting ligands. The use of F(ab')$_2$ instead of whole mAb in this case eliminated any Fc-medicated non-specific interaction or immunogenic side effects.

The site-directed maleimide-thiol conjugation of targeting ligands to particles described herein is a departure from the immunogenic (strept)avidin-biotin conjugation chemistry traditionally used in preparing targeting particles. Although other conjugation chemistries can be used, the site-directed maleimide-thiol conjugation method was advantageous due to its low immunogenicity, strong and rapid thioether bond formation at near neutral pH (bond strength in the order of nanonewtons; second-order rate constant 0.8-$1 \times 10^4$ $M^{-1}s^{-1}$). The near neutral pH was advantageous in avoiding negative impact on the binding elements and particles during preparation, and prevents dissociation of the binding elements from the particle shell *in vivo.* Targeting particles based on similar conjugation chemistry, including non-covalent conjugation of targeting ligands to a phospholipid species before particle assembly, exist but only a few are progressing to ultrasound molecular imaging *in vivo,* [44-47, 61-64] none of which showed all of the following desirable attributes which differentiate our microparticles from them: (i) high target binding specificity with proven minimal non-specific retention in remote non-RES tissues not expressing the target molecule, following *iv* administration; (ii) effective for real-time US molecular imaging; and (iii) effective for acoustic quantification of molecular targets to a high quantitative degree.

***In Vitro* Validation of Esel Targeting Microparticles**

Esel targeting particles attached to Esel on coated dish (dish E): 2060(mean) $\pm$1070(SEM) particles/mm$^2$, n = 5, Figure 2d. Specificity of the targeting particles against Esel was demonstrated by their minimal attachment to Esel previously blocked with excess anti-Esel antibody (dish B): 8(mean) $\pm$4(SEM) % of targeting particles on dish E, n = 5. Additional negative controls using targeting or non-targeting (native) particles on dish not coated with Esel (dish P), or non-targeting particles on dish E and B showed similar low levels of particle attachment: targeting particles on dish P (9 $\pm$3%, n = 5), non-targeting particles on dish E (10 $\pm$9%, n = 2), B (7 $\pm$7%, n = 2) and P (7 $\pm$7%, n = 2). ANOVA with Turkey's post-hoc analysis showed significant differences in the relative density of attached particles between targeting particles on dish E and the negative controls ($p < 0.0001$); no significant difference was observed amongst the negative controls.

***In Vivo* Validation of Esel Targeting Microparticles**

Esel targeting particles were administered to 5 WT (body weight: 25(mean) $\pm$2(SD) g, range 23-27g)) and 5 Esel KO (24 $\pm$2g, range 22-27g) mice at 3:06-4:03h and 3:17-4:30h post IL-1$\beta$ pre-treatment, respectively. The particles

were seen to circulate and reach the cremaster muscle ≈7-17s post *iv* bolus administration through the tail vein. The number of freely circulating-particles decreased with time, their clearance from the blood pool occurred sooner in the WT than KOs, Figure 3a. Beyond 10min after particle administration, the number of freely circulating-particles in the blood pool was minimal or undetectable in all animals. The particles attached and accumulated on the cremaster venular wall of the WT animals (370(mean) ±46(SEM) particles/$mm^2$ VSA, n = 5 animals); this was minimal in the KOs (11 ±3, n = 5), p <0.0001 (student's t-test), Figure 3c-d. Particle accumulation on the cremaster vessel wall plateaued soon after particle-bolus administration, Figure 3b. The accumulated particles persisted beyond the clearance of freely circulating ones from the blood pool. Rolling was observed in a small minority of particles; complete detachment of the attached particles was infrequently seen. Intravascular obstruction by the particles was not observed. Transmigration into the tissue interstitium or cellular internalisation of the attached particles were not detected, when observed intermittently for up to 90min. Confocal microscopy showed that the attached particles co-localised with Esel expressed on the endothelial cell surface of the WT cremaster venule (n = 3 animals, body weight 21.8-24g); Esel expression was not detectable in the KOs (n = 2, 25.7-28.4g), Figure 3e.

Shear rates in the 20-40μm diameter venules, determined from 3 WT (body weight: 26(mean) ±2(SD) g, range 25-28g) and 3 KO (27 ±1g, range 26-29g) mice, were higher in the WT (329(mean) ±46(SEM) $s^{-1}$, n = 3) than KO group (211 ±37$s^{-1}$, n = 3), probably due to smaller vessel diameters sampled in the former (WT 30(mean) ±3(SEM) μm, n = 3; KO 34 ±1μm, n = 3). However, these differences were not statistically significant, p = 0.30 and 0.12 (student's t-test) for shear rate and vessel diameter group comparison, respectively.

## Ultrasound Molecular Imaging

**(1) Animals.** 15 WT (age 5.7(mean) ±0.2(SD) weeks, range 5.1-6.1 weeks; body weight 19.5(mean) ±1.5(SD) g, range 17-22g) and 8 Esel KO (age 7.9 ±3.2 weeks, range 5-13.6 weeks; body weight 22.3 ±3.7g, range 17-28g) mice were imaged. The duration between LPS injection and administration of targeting particles ($LPS_{Time}$) ranged 3:26-5:59h for the WT and 4:27-5:39h for the KO group.

**(2) Cardiac imaging.** Non-linear CPS artefacts (orange colour) present both before and after particle administration could be seen in WT and Esel KO animals; these artefacts were small and outside the myocardium, Figure 4a (frame 0:10), Figure 4a2 (frame 20:20). Following *iv* particle-bolus administration, particle signal was first detected in the right heart chambers within 4 heart beats (≈1s), the signal intensity rose rapidly. This was followed by the appearance of particle signal in the left heart chambers as the particles returned from the pulmonary circulation. Particle signal intensity in the LV cavity and myocardium peaked within 6-7 heart beats (≈1.5-2s) and 9-12 heart beats (≈2-3s) after particle administration, respectively. Particle signal intensity then decreased in the heart chambers and myocardium over time, Figure 4a. Significant US attenuation due to high particle concentration occurred early following particle-bolus administration, with major loss of signal in regions of the heart located distally in the US path (e.g., 5-10 o'clock positions in the myocardium and adjacent LV cavity in the PSA view, Figure 4a). As the circulating-particle concentration in the blood pool (LV cavity) decreased over time, the attenuation diminished (compare frame 0:30 *vs.* 6:20, Figure 4a) but did not disappear (frame 20:20, Figure 4a1) - most likely due to attenuation caused by overlying bone, lung air ± retained-particles. Importantly, Esel expression in the myocardium was visualised in real-time, indicated by the persistence of particle signal in the WT myocardium as the freely circulating-particles in the blood pool (LV cavity) decreased and disappeared over time. In the KO myocardium, the persistence of particle signal was low/minimal, consistent with a low/minimal degree of non-specific particle retention in the myocardium, Figure 4a-b. US attenuation caused by overlying bone, lung air ± retained-particles located proximally in the US path, affected certain parts of the myocardium depending on the imaging scan plane - this caused pseudo-loss of targeted particle signal for Esel in the WT animals (e.g., there was artefactual loss of retained-particle signal in the mid-posterior, -inferior, -posteroseptal and -anteroseptal walls of the LV (anticlockwise from 5-10 o'clock positions, respectively) in the PSA view with 14MHz CPS imaging). However, by changing the scan plane (e.g., from PSA to PLA or A4C view) to alter the relative position of overlying entities, or by lowering the US frequency to increase its penetrative depth (e.g., from 14 to 7MHz), these attenuation effects could be overcome with good recovery of the retained-particle signals, Figure 4b. The global expression of Esel in the WT myocardium was thus demonstrated on US imaging, consistent with the immunohistochemistry data.

In the above situation, US detection of molecular target was limited by late distal attenuation from overlying bone/air and retained-particles located proximally in the US path. However, it was found that such attenuation could be overcome by using lower frequency US (greater penetrative depth) or a different imaging angle. From the human imaging perspective, where the use of lower frequency US (e.g., 3-7 MHz) and multi-plane imaging are the norm, and the footprint of the transducer is much smaller relative to the body size (making it easier to achieve optimal probe position/angle and avoid overlying bone/air), these attenuation issues are likely less important. Nevertheless,

refinements in the machine's attenuation correction algorithm may further minimise the attenuation artefacts.

**(3) Imaging of other tissues.** Comparison of the thoracic, abdominal and pelvic scans between the WT and Esel KO animals with reference to the baseline images (before particle administration), showed that non-specific retention of the targeting particles in non-RES tissues was low/minimal. Esel expression was detected in the renal cortex of WT but not KO animals (Figures 5a & 5b). Immunohistochemistry and confocal microscopy confirmed that Esel was expressed predominantly on endothelial cells of renal glomeruli, the latter concentrated in the renal. As expected, the targeting particles were taken up by the spleen and liver in both WT and KO animals (Figures 5a & 5b), the major RES tissues involved in particle elimination.

**(4) Adverse effects.** No death or significant adverse events attributable to the Esel targeting particles were observed. No significant particle-medicated intravascular obstruction in the myocardium, causing loss of regional myocardial perfusion manifest as regional wall motion abnormality, was detected.

**Ultrasound TIC of the Myocardium (tissue) and LV Cavity (central blood pool)**

Three phases with distinct characteristics were discernible from the TICs following iv bolus administration of the targeting particles, Figure 4a:

(1) Bolus-phase (B): lasting a few seconds, characterised by an initial rapid rise in particle signal intensity reaching peak or saturating/attenuation levels (as particle concentration increased) within seconds of bolus injection.

(2) Distribution-phase (D): lasting up to 1-2min (takes a few circulatory cycles to complete), characterised by a short rapid decrease in particle signal intensity, as particles dilute by mixing and distribution to tissues. High particle concentration resulted in signal saturation/attenuation, obscuring re-circulation peaks. As the particle concentration decreased further over time, the particle signal intensity was frequently observed to paradoxically increase as attenuation decreased, giving rise to a second lower peak within 0.5-1min of particle administration (arrow in insets of Figure 4a).

(3) Elimination-phase (E): lasting several minutes, characterised by a long slow decrease in particle signal intensity. Particle signal intensity in the LV cavity (representing freely circulating-particle concentration in the central blood pool) became undetectable sooner in the WT than Esel KO animals; in both groups they were essentially undetectable by 20min post particle administration. In the myocardium, particle signal intensity of WT animals decreased slower than that of KOs, and persisted beyond the disappearance of particle signal in the LV cavity. In KO animals, particle signal in the myocardium became undetectable before the disappearance of particle signal in the LV cavity (as expected for relative myocardial blood volume (rMBV) $\leq$24%), except when a detectable degree of non-specific particle retention was present.

**Acoustic Quantification of Esel Expression**

**(1) Animals.** 12 WT (age 5.7(mean) $\pm$0.3(SD) weeks, range 5.1-6.1 weeks; body weight 19.7(mean) $\pm$1.4(SD) g, range 18-22g) and 8 Esel KO (age 7.9 $\pm$3.2 weeks, range 5-13.6 weeks; body weight 22.3 $\pm$3.7g, range 17-28g) mice were used. $LPS_{Time}$ ranged 3:53-5:59h for the WT and 4:27-5:39h for the KO group. Excluded from quantitative analysis here were 3 WT animals because of: (i) microparticle dosing error (1 animal); (ii) uncertainties regarding Esel expression level (2 animals); and (iii) 1 of these 3 animals has a TIC that could not be adequately quantified, possibly due to severe attenuation artefact.

**(2) Single late time point based method.** In both WT and Esel KO groups, freely circulating-particle signal in the blood pool (LV cavity) was absent/minimal by $\approx$20min following *iv* bolus administration of $10^8$ particles. Therefore, background-subtracted *I* in the myocardium at 20min 10s post bubble administration (R20) was used to represent that of the retained-particles only, as signal contribution from any residual freely circulating-particles by this time was negligible due to their low concentrations in the central blood pool (signal intensity in the LV cavity). E.g., 0.1AU in the LV cavity would contribute only 0.005-0.024AU in the myocardium assuming a relative myocardial blood volume (% of myocardium that is blood) of $\approx$5-24%.[65-67] R20 was significantly higher in the WT (0.46 $\pm$0.16 (SEM), range 0.01-1.61, n = 12) than KO (0.06 $\pm$0.03, -0.01-0.24, n = 8) animals, p<0.05. R20 correlated strongly with $LPS_{Time}$ in the WT (r = -0.78, p <0.05, n = 12) but not KO mice (r = -0.05, p = 0.9, n = 8), Figure 8c. It also correlated strongly with the concentration of Esel mRNA (r = 0.81, p <0.005, n = 12), Figure 8d.

**(3) TIC-based method.** The maximum retained-particle signal intensity in the myocardium, $A_r$, was significantly higher in the WT (2.3$\pm$0.4 (SEM), range 0.8-4.2, n = 12) than Esel KO (0.4$\pm$0.1, 0-1, n = 8) animals, p<0.005. The low $A_r$ values in the KOs suggested minor degrees of non-specific particle retention. $A_r$ correlated strongly with $LPS_{Time}$ in the WT (r = - 0.87, p <0.0005, n = 12) but not KO mice (r = -0.08, p = 0.86, n = 8), Figure 8a. By converting

$LPS_{Time}$ to the concentration of Esel mRNA using the curve in Figure 7a, Figure 8b showed that $A_r$ correlated strongly with the concentration of Esel mRNA (r = 0.84, p <0.001, n = 12), the latter in the range that was approximately linearly related to the concentration of the cell surface Esel protein, Figure 7c.

### Acoustic Quantification of Particle Half-life *In Vivo* (using TIC-based method)

The half-life of the freely circulating-particles *in vivo* was ≈2(mean) ±1(SD) min, range 1-5min for the WT (n = 12) and KO (n = 8) animals. This was comparable with most commercially available non-targeting microbubbles (range ≈1-3min), demonstrating that the microparticles produced were of commercial quality or better. The elimination of the retained-particles in the myocardium decreased with increased maximum concentration of retained-particles in the myocardium. The relationship was non-linear and could be empirically fitted to an exponential or sigmoidal function. This resulted in the half-life of the retained-particles being shorter the lower the maximum retained-particle concentration. The *in vivo* half-life of the acoustically effective retained-particles in the myocardium in these groups of animals was ≈7(mean) ±5(SD) min, range 3-18min in the WT (n = 12) compared with 4 ±4min, range 1-14min in the KO animals (n = 8).

### Targeting Microparticle Pharmacokinetics and Accumulation Kinetics on the Target Surface

The accumulation of attached microparticles on the target surface was essentially complete soon after particle-bolus administration. Using the novel TIC-based method, it was discovered that the elimination of the retained and freely-circulating targeting microparticles followed first-order kinetics *in vivo.*

These results demonstrate that the targeting microparticles are specific and effective *in vivo* for highly quantitative real-time ultrasound molecular imaging of one or more organs. The microparticles have favourable characteristics *in vivo* which include, but are not limited to, being non-toxic, sufficiently stable for continuous and multi-plane imaging with a single-bolus microparticle administration, having favourable kinetics and acoustic response for highly quantitative analysis of the molecular moiety of interest. They have sufficiently high targeting specificity and efficiency to the molecular moiety of interest *in vivo,* and lack non-specific binding/persistence in tissues not expressing the molecular moiety of interest (except in the liver and spleen which are the usual routes of microparticle elimination in the body). In conclusion, these targeting microparticles are different and superior to the prior art.

### References

1 J. R. Lindner, et al., Circulation 2001, 104, 2107-2112.
2 J. R. Lindner, et al., Circulation 2000, 102, 2745-2750.
3 J. B. Fowlkes, J Ultrasound Med 2008, 27, 503-15.
4 R. Lukac, et al., Langmuir 2011, 27, 4829-37.
5 P. J. Bjorkman, P. Parham, Annu Rev Biochem 1990, 59, 253-88.
6 K. Nord, et al., Nat Biotechnol 1997, 15, 772-7.
7 K. Nord, et al., J Biotechnol 2000, 80, 45-54.
8 E. Harlow, D. Lane, Antibodies: A Laboratory Manual. Editor, Cold Spring Harbor Laboratory Press, Cold Spring Harbor, New York, 1988, pp. 726.
9 G. Kansas, in Physiology of inflammation, ed. by K. Ley, Oxford University Press, Oxford, 2001, pp. 222-241.
10 P. R. Reynolds, et al., Radiology 2006, 241, 469-76.
11 F. Jamar, et al., Rheumatology 2002, 41, 53-61.
12 B. A. Kaufmann, et al., Eur Heart J 2007, 28, 2011-7.
13 S. Andonian, et al., J Endourol 2009, 23, 373-8.
14 B. A. Kaufmann, et al., J Am Soc Echocardiogr 2010, 23, 79-85.
15 P. Hauff, et al., Radiology 2004, 231, 667-73.
16 F. S. Villanueva, et al., Circulation 2007, 115, 345-52.
17 G. E. Weller, et al., Circulation 2003, 108, 218-224.
18 R. A. Linker, et al., J Autoimmun 2005, 25, 199-205.
19 M. Reinhardt, et al., Neuroimage 2005, 27, 267-78.
20 B. A. Kaufmann, et al., Circulation 2007, 116, 276-84.
21 C. Z. Behm, et al., Circulation 2008, 117, 2902-11.
22 C. Bachmann, et al., Gastroenterology 2006, 130, 8-16.
23 J. W. Xuan, et al., Mol Imaging 2009, 8, 209-20.
24 A. Lyshchik, et al., J Ultrasound Med 2007, 26, 1575-86.

25 D. J. Lee, et al., J Ultrasound Med 2008, 27, 855-66.
26 J. K. Willmann, et al., Radiology 2008, 249, 212-9.
27 J. J. Rychak, et al., Mol Imaging 2007, 6, 289-96.
28 M. A. Pysz, et al., Radiology 2010, 256, 519-527.
29 S. Pochon, et al., Invest Radiol 2010, 45, 89-95.
30 R. Pillai, et al., Bioconjug Chem 2010, 21, 556-562.
31 G. Korpanty, et al., Clin Cancer Res 2007, 13, 323-30.
32 J. K. Willmann, et al., Radiology 2008, 248, 936-44.
33 M. Palmowski, et al., Mol Cancer Ther 2008, 7, 101-9.
34 M. Palmowski, et al., Neoplasia 2009, 11, 856-63.
35 H. Y. Jun, et al., Acad Radiol 2010, 17, 54-60.
36 J. K. Willmann, et al., J Nucl Med 2010, 51, 433-40.
37 H. Leong-Poi, et al., Circulation 2003, 107, 455-460.
38 H. Leong-Poi, et al., Circulation 2005, 111, 3248-54.
39 D. B. Ellegala, et al., Circulation 2003, 108, 336-341.
40 S. M. Stieger, et al., Contrast Media Mol Imaging 2008, 3, 9-18.
41 G. E. Weller, et al., Cancer Res 2005, 65, 533-9.
42 M. A. Kuliszewski, et al., Cardiovasc Res 2009, 83, 653-62.
43 T. Sakuma, et al., Cardiovasc Res 2005, 66, 552-61.
44 A. Alonso, et al., Stroke 2007, 38, 1508-14.
45 I. Tardy, et al., Acad Radiol 2002, 9 Suppl 2, S294-6.
46 M. Takeuchi, et al., J.Am.Soc.Echocardiogr. 1999, 12, 1015-1021.
47 B. Wang, et al., Acad Radiol 2006, 13, 428-33.
48 X. X. Jing, et al., Clin Imaging 2008, 32, 178-82.
49 J. P. Christiansen, et al., Circulation 2002, 105, 1764-1767.
50 I. Kondo, et al., Circulation 2004, 109, 1056-1061.
51 J. S. Cheung, et al., Neuroimage 2009, 46, 658-64.
52 R. Tang, et al., Phys Med Biol 2011, 56, 3503-12.
53 F. Yan, et al., Ultrasound Med Biol 2011, 37, 768-79.
54 A. Della Martina, et al., Eur J Pharm Biopharm 2008, 68, 555-64.
55 S. Unnikrishnan, A. L. Klibanov, AJR Am J Roentgenol 2012, 199, 292-9.
56 A. L. Klibanov, et al., Proceed Int'l Symp Control Rel Bioact Mater 1999, 26, 230.
57 M. A. Labow, et al., Immunity. 1994, 1, 709-720.
58 M. J. Eppihimer, et al., Circ. Res. 1996, 79, 560-569.
59 J. W. Fries, et al., Am J Pathol 1993, 143, 725-37.
60 M. J. Hickey, et al., J Immunol 1999, 162, 1137-43.
61 C. R. Anderson, et al., Invest Radiol 2010, 45, 579-85.
62 C. R. Anderson, et al., Invest Radiol 2011, 46, 215-24.
63 G. Hu, et al., Thromb Haemost 2012, 107, 172-83.
64 J. Bzyl, et al., Eur Radiol 2011, 21, 1988-95.
65 J. U. Streif, et al., Magn Reson Med 2005, 53, 584-92.
66 C. Waller, et al., Radiology 2000, 215, 189-97.
67 R. Coulden, in Functional Computed Tomography, ed. by K. Miles, et al., ISIS Medical Media, Oxford, 1997, pp. 133-156.

## Claims

**1.** A microparticle composition suitable for molecular imaging, the composition comprising microparticles, wherein the microparticles comprise: a core microparticle structure having a central area and a shell, and wherein the core microparticle structure comprises:

(i) a phosphatidylcholine lipid;
(ii) a phosphatidylethanolamine lipid comprising at least one maleimide moiety; and
(iii) an alkoxylated fatty acid, and

wherein the molar ratio of (i) to (iii) satisfies the following ranges:

(i) 70 to 80;
(ii) 5 to 15; and
(iii) 10 to 20.

**2.** A microparticle composition according to claim 1, further comprising at least one molecular binding element, wherein the at least one molecular binding element is covalently attached to the shell of the core microparticle structure,

wherein the at least one molecular binding element is optionally covalently attached to the core microparticle structure via the at least one maleimide moiety, and/or
wherein the at least one molecular binding element comprises a protein, peptide, or small organic moiety, such as wherein the at least one molecular binding element is an antibody, and/or
wherein the conjugation reaction molar ratio of the at least one molecular binding element to component (ii) is ≥1:1, optionally ≥5:1, and/or
wherein the microparticles have at least about 1 x $10^5$ molecular binding elements per microparticle.

**3.** A microparticle composition according to claim 1 or claim 2, wherein the core microparticle structure is micellar, and/or

wherein the core microparticle structure further comprises a labelling moiety, wherein the molar ratio of the labelling moiety in the composition is optionally 0.2 to 50, optionally 0.5 to 5, and wherein the labelling moiety is optionally a fluorescent dye, such as 1,1'-dioctadecyl-3,3,3',3'-tetramethylindocarbocyanine perchlorate (Dil), and/or
wherein the core microparticle structure further comprises a phosphatidylethanolamine lipid, wherein the phosphatidylethanolamine lipid is optionally a $C_{10-20}$ saturated phosphatidylethanolamine lipid, such as a distearoylphosphatidylethanolamine (DSPE), wherein the molar ratio of the phosphatidylethanolamine lipid in the composition is optionally 0.5 to 5, and/or
wherein the central area of the core microparticle structure contains a fluid medium, wherein the fluid medium optionally comprises a physiologically acceptable gas, such as a physiologically acceptable gas selected from air, nitrogen, carbon dioxide, xenon, krypton, sulfur hexafluoride, chlorotrifluoromethane, dichlorodifluoro-methane, bromotrifluoromethane, bromochlorodifluoromethane, tetrafluoromethane, dibromo-difluoromethane, dichlorotetrafluoroethane, chloropentafluoroethane, hexafluoroethane, hexafluoropropylene, octafluoropropane, hexafluoro-butadiene, octafluoro-2-butene, octafluorocyclobutane, decafluorobutane, perfluorocyclopentane, dodecafluoropentane, and tetradecafluorohexane, optionally wherein the physiologically acceptable gas comprises octafluoropropane, and/or
wherein the phosphatidylcholine lipid is a $C_{10-20}$ saturated phosphatidylcholine lipid, such as a 1,2-distearoyl-*sn*-glycero-3-phosphocholine, and/or
wherein the phosphatidylethanolamine lipid comprising at least one maleimide moiety is a $C_{10-20}$ saturated phosphatidylethanolamine lipid, and/or
wherein the phosphatidylethanolamine lipid comprising at least one maleimide moiety comprises a polyethylene glycol chain with a molecular weight of at least 500, wherein the at least one maleimide moiety is optionally attached to the polyethylene glycol chain, such as wherein the phosphatidylethanolamine lipid comprising at least one maleimide moiety is a 1,2-distearoyl-*sn*-glycero-3-phosphoethanolamine-*N*-[maleimide(polyethylene glycol)-2000], and/or
wherein the alkoxylated fatty acid is a polyethylene glycol fatty acid ester, wherein the polyethylene glycol fatty acid ester is optionally a $C_{10-20}$ saturated polyethylene glycol fatty acid ester, such as a $PEG_{40}$ stearate, and/or
wherein the average diameter of the microparticles is in the range of 0.5 to 5 μm.

**4.** An intermediate microparticle composition suitable for producing a molecular imaging microparticle composition, wherein the intermediate composition comprises:

(i) a phosphatidylcholine lipid;
(ii) a phosphatidylethanolamine lipid comprising at least one maleimide moiety; and
(iii) an alkoxylated fatty acid, and

wherein the molar ratio of (i) to (iii) satisfies the following ranges:

(i) 70 to 80;
(ii) 5 to 15; and
(iii) 10 to 20.

**5.** An intermediate microparticle composition according to claim 4, further comprising at least one molecular binding element, wherein the at least one molecular binding element is optionally covalently attached to the at least one maleimide moiety, wherein the at least one molecular binding element optionally comprises a protein, peptide, or small organic moiety, such as wherein the at least one molecular binding element is an antibody, wherein the conjugation reaction molar ratio of the at least one molecular binding element to component (ii) is optionally ≥1:1 or ≥5:1, and/or

wherein the intermediate microparticle composition further comprises a labelling moiety, wherein the molar ratio of the labelling moiety in the composition is optionally 0.2 to 50, optionally 0.5 to 5, and wherein the labelling moiety is optionally a fluorescent dye, such as a 1,1'-dioctadecyl-3,3,3',3'-tetramethylindocarbocyanine perchlorate (DiI), and/or
wherein the intermediate microparticle composition further comprises a phosphatidylethanolamine lipid, wherein the phosphatidylethanolamine lipid is optionally a $C_{10-20}$ saturated phosphatidylethanolamine lipid, such as a distearoylphosphatidylethanolamine (DSPE), wherein the molar ratio of the phosphatidylethanolamine lipid in the composition is optionally 0.5 to 5, and/or
wherein the phosphatidylcholine lipid is a $C_{10-20}$ saturated phosphatidylcholine lipid, such as a 1,2-distearoyl-*sn*-glycero-3-phosphocholine, and/or
wherein the phosphatidylethanolamine lipid comprising at least one maleimide moiety is a $C_{10-20}$ saturated phosphatidylethanolamine lipid, and/or
wherein the phosphatidylethanolamine lipid comprising at least one maleimide moiety comprises a polyethylene glycol chain with a molecular weight of at least 500, wherein the at least one maleimide moiety is optionally attached to the polyethylene glycol chain, such as wherein the phosphatidylethanolamine lipid comprising at least one maleimide moiety is a 1,2-distearoyl-*sn*-glycero-3-phosphoethanolamine-*N*-[maleimide(polyethylene glycol)-2000], and/or
wherein the alkoxylated fatty acid is a polyethylene glycol fatty acid ester, wherein the polyethylene glycol fatty acid ester is optionally a $C_{10-20}$ saturated polyethylene glycol fatty acid ester, such as a $PEG_{40}$ stearate.

**6.** An intermediate microparticle composition according to claim 4 or claim 5, wherein the composition is a lyophilisate or an aqueous dispersion.

**7.** A kit comprising an intermediate composition according to any of claims 4 to 6, and a source of a fluid medium which comprises a physiologically acceptable gas.

**8.** A kit according to claim 7, wherein the physiologically acceptable gas is selected from air, nitrogen, carbon dioxide, xenon, krypton, sulfur hexafluoride, chlorotrifluoromethane, dichlorodifluoro-methane, bromotrifluoromethane, bromochlorodifluoromethane, tetrafluoromethane, dibromo-difluoromethane, dichlorotetrafluoroethane, chloropentafluoroethane, hexafluoroethane, hexafluoropropylene, octafluoropropane, hexafluoro-butadiene, octafluoro-2-butene, octafluorocyclobutane, decafluorobutane, perfluorocyclopentane, dodecafluoropentane, and tetradecafluorohexane, optionally wherein the physiologically acceptable gas comprises octafluoropropane.

**9.** A method of preparing a microparticle composition according to any one of claims 1 to 3 or an intermediate microparticle composition according to any one of claims 4 to 6, the method comprising:

(i) forming a core microparticle structure having a central area and a shell comprising components (i) to (iii), and optionally further comprising:
(ii) covalently attaching at least one molecular binding element to the shell of the core microparticle structure.

**10.** A method of preparing a microparticle composition according to claim 9, wherein step (i) is conducted in the presence of a physiologically acceptable gas, and/or
wherein the conjugation reaction molar ratio of the at least one molecular binding element to component (ii) in step (i) is ≥1:1, optionally ≥5:1.

**11.** A pharmaceutical composition comprising a microparticle composition according to any of claims 1 to 3, and a pharmaceutically acceptable carrier or excipient.

**12.** A pharmaceutical composition according to claim 11 for use as a contrast agent in molecular imaging.

**13.** A microparticle composition according to any of claims 1 to 3 for use as a contrast agent in molecular imaging.

**14.** An imaging method comprising:

administering the microparticle composition of any of claim 1 to 3, or a pharmaceutical composition of claim 11 to a subject; and
imaging the subject using at least one imaging technique,
wherein the imaging technique optionally comprises ultrasound imaging (US) or magnetic resonance imaging (MRI).

**Patentansprüche**

**1.** Mikropartikel-Zusammensetzung, die für die molekulare Bildgebung geeignet ist, wobei die Zusammensetzung Mikropartikel umfasst, wobei die Mikropartikel eine Mikropartikel-Kernstruktur mit einem zentralen Bereich und einer Hülle umfassen, und wobei die Mikropartikel-Kernstruktur umfasst:

(i) ein Phosphatidylcholin-Lipid,
(ii) ein Phosphatidylethanolamin-Lipid, das mindestens einen Maleimidrest umfasst, und
(iii) eine alkoxylierte Fettsäure und

wobei der molare Anteil von (i) bis (iii) die folgenden Bereiche abdeckt:

(i) 70 bis 80,
(ii) 5 bis 15, und
(iii) 10 bis 20.

**2.** Mikropartikel-Zusammensetzung nach Anspruch 1, die ferner mindestens ein molekulares Bindungselement umfasst, wobei das mindestens eine molekulare Bindungselement kovalent an die Hülle der Mikropartikel-Kernstruktur gebunden ist,
wobei das mindestens eine molekulare Bindungselement gegebenenfalls über den mindestens einen Maleimidrest kovalent an die Mikropartikel-Kernstruktur gebunden ist, und/oder
wobei das mindestens eine molekulare Bindungselement ein Protein, Peptid oder einen kleinen organischen Rest umfasst, etwa wobei das mindestens eine molekulare Bindungselement ein Antikörper ist, und/oder
wobei das Molverhältnis der Konjugationsreaktion des mindestens einen molekularen Bindungselements zu Komponente (ii) >1:1, gegebenenfalls >5:1, ist und/oder
wobei die Mikropartikel mindestens etwa 1 x $10^5$ molekulare Bindungselemente pro Mikropartikel aufweisen

**3.** Mikropartikel-Zusammensetzung nach Anspruch 1 oder Anspruch 2, wobei die Mikropartikel-Kernstruktur mizellar ist und/oder
wobei die Mikropartikel-Kernstruktur weiterhin einen Markierungsrest umfasst, wobei der molare Anteil des Markierungsrests in der Zusammensetzung wahlweise 0,2 bis 50, gegebenenfalls 0,5 bis 5 beträgt, und wobei der Markierungsrest gegebenenfalls ein Fluoreszenzfarbstoff, wie 1,1'-Dioktadecyl-3,3,3',3'-tetramethylindocarbocyaninperchlorat (DiI), ist, und/oder
wobei die Mikropartikel-Kernstruktur ferner ein Phosphatidylethanolamin-Lipid umfasst, wobei das Phosphatidylethanolamin-Lipid gegebenenfalls ein gesättigtes $C_{10-20}$-Phosphatidylethanolamin-Lipid, wie ein Distearoylphosphatidylethanolamin (DSPE), ist, wobei der molare Anteil des Phosphatidylethanolamin-Lipids in der Zusammensetzung gegebenenfalls 0,5 bis 5 ist, und/oder
wobei der zentrale Bereich der Mikropartikel-Kernstruktur ein fluides Medium enthält, wobei das fluide Medium gegebenenfalls ein physiologisch zulässiges Gas, etwa ein physiologisch zulässiges Gas, ausgewählt aus Luft, Stickstoff, Kohlendioxid, Xenon, Krypton, Schwefelhexafluorid, Chlortrifluormethan, Dichlordifluormethan, Bromtrifluormethan, Bromchlordifluormethan, Tetrafluormethan, Dibromdifluormethan, Dichlortetrafluorethan, Chlorpentafluorethan, Hexafluorethan, Hexafluorpropylen, Oktafluorpropan, Hexafluorbutadien, Oktafluor-2-buten, Oktafluorcyclobutan, Dekafluorbutan, Perfluorcyclopentan, Dodekafluorpentan und Tetradekafluorhexan umfasst, wobei das physiologisch zulässige Gas gegebenenfalls Oktafluorpropan umfasst, und/oder
wobei das Phosphatidylcholin-Lipid ein gesättigtes $C_{10-20}$-Phosphatidylcholin-Lipid, etwa 1,2-Distearoyl-sn-glycero-3-phosphocholin, ist, und/oder
wobei das Phosphatidylethanolamin-Lipid, das mindestens einen Maleimidrest umfasst, ein gesättigtes $C_{10-20}$-Phosphatidylethanolamin-Lipid ist, und/oder
wobei das Phosphatidylethanolamin-Lipid, das mindestens einen Maleimidrest umfasst, eine Polyethylenglykol-Kette mit einem Molekulargewicht von mindestens 500 umfasst, wobei der mindestens eine Maleimidrest gegebenenfalls an die Polyethylenglykol-Kette gebunden ist, etwa wobei das Phosphatidylethanolamin-Lipid, das mindestens einen Maleimidrest umfasst, ein 1,2-Distearoyl-*sn*-glycero-3-phosphoethanolamin-N-[maleimid(polyethylenglykol)-2000] ist, und/oder
wobei die alkoxylierte Fettsäure ein Polyethylenglykol-Fettsäureester ist, wobei der Polyethylenglykol-Fettsäureester gegebenenfalls ein gesättigter $C_{10-20}$-Fettsäure-Polyethylenglykolester, etwa ein $PEG_{40}$-Stearat, ist, und/oder
wobei der mittlere Durchmesser der Mikropartikel im Bereich von 0,5 bis 5 μm liegt.

**4.** Eine Mikropartikel-Zwischenproduktzusammensetzung, die zur Herstellung einer Mikropartikel-Zusammensetzung für die molekulare Bildgebung geeignet ist, wobei die Zwischenproduktzusammensetzung umfasst:

(i) ein Phosphatidylcholin-Lipid,
(ii) ein Phosphatidylethanolamin-Lipid, das mindestens einen Maleimidrest umfasst, und
(iii) eine alkoxylierte Fettsäure und

wobei der molare Anteil von (i) bis (iii) die folgenden Bereiche abdeckt:

(i) 70 bis 80,
(ii) 5 bis 15, und
(iii)10 bis 20.

**5.** Mikropartikel-Zwischenproduktzusammensetzung nach Anspruch 4, die ferner mindestens ein molekulares Bindungselement umfasst, wobei das mindestens eine molekulare Bindungselement gegebenenfalls kovalent an die mindestens einen Maleimidrest gebunden ist, wobei das mindestens eine molekulare Bindungselement gegebenenfalls ein Protein, Peptid oder einen kleinen organischen Rest umfasst, etwa wobei das mindestens eine molekulare Bindungselement ein Antikörper ist, wobei der molare Anteil der Konjugationsreaktion des mindestens einen molekularen Bindungselements zu Komponente (ii) gegebenenfalls >1:1 oder >5:1 ist, und/oder
wobei die Mikropartikel-Zwischenproduktzusammensetzung ferner einen Markierungsrest umfasst, wobei der molare Anteil des Markierungsrests in der Zusammensetzung wahlweise 0,2 bis 50, gegebenenfalls 0,5 bis 5, beträgt und wobei der Markierungsrest gegebenenfalls ein Fluoreszenzfarbstoff, wie ein 1,1'-Dioktadecyl-3,3,3',3'-tetramethylindocarbocyaninperchlorat (Dil), ist, und/oder
wobei die Mikroteilchen-Zwischenproduktzusammensetzung ferner ein Phosphatidylethanolamin-Lipid umfasst, wobei das Phosphatidylethanolamin-Lipid gegebenenfalls ein gesättigtes $C_{10-20}$-Phosphatidylethanolamin-Lipid, etwa Distearoylphosphatidylethanolamin (DSPE), ist, wobei der molare Anteil des Phosphatidylethanolamin-Lipids in der Zusammensetzung gegebenenfalls 0,5 bis 5 ist, und/oder
wobei das Phosphatidylcholin-Lipid ein gesättigtes $C_{10-20}$-Phosphatidylcholin-Lipid, etwa 1,2-Distearoyl-*sn*-glycero-3-phosphocholin, ist, und/oder
wobei das Phosphatidylethanolamin-Lipid, das mindestens einen Maleimidrest umfasst, ein gesättigtes $C_{10-20}$-Phosphatidylethanolamin-Lipid ist, und/oder
wobei das Phosphatidylethanolamin-Lipid, das mindestens einen Maleimidrest enthält, eine Polyethylenglykolkette mit einem Molekulargewicht von mindestens 500 umfasst, wobei der mindestens eine Maleimidrest gegebenenfalls an die Polyethylenglykolkette gebunden ist, etwa wobei das Phosphatidylethanolamin-Lipid, das mindestens einen Maleimidrest umfasst, ein 1,2-Distearoyl-*sn*-glycero-3-phosphoethanolamin-N-[maleimid(polyethylenglykol)-2000] ist, und/oder
wobei die alkoxylierte Fettsäure ein Polyethylenglykol-Fettsäureester ist, wobei der Polyethylenglykol-Fettsäureester gegebenenfalls ein gesättigter $C_{10-20}$-Fettsäure-Polyethylenglykolester, etwa ein $PEG_{40}$-Stearat, ist.

**6.** Mikropartikel-Zwischenproduktzusammensetzung nach Anspruch 4 oder Anspruch 5, wobei die Zusammensetzung ein Lyophilisat oder eine wässrige Dispersion ist.

**7.** Satz, umfassend eine Zwischenproduktzusammensetzung nach einem der Ansprüche 4 bis 6 und eine Quelle für ein fluides Medium, das ein physiologisch zulässiges Gas umfasst.

**8.** Satz nach Anspruch 7, wobei das physiologisch zulässige Gas ausgewählt ist aus Luft, Stickstoff, Kohlendioxid, Xenon, Krypton, Schwefelhexafluorid, Chlortrifluormethan, Dichlordifluormethan, Bromtrifluormethan, Bromchlordifluormethan, Tetrafluormethan, Dibromdifluormethan, Dichlortetrafluorethan, Chlorpentafluorethan, Hexafluorethan, Hexafluorpropylen, Oktafluorpropan, Hexafluorbutadien, Oktafluor-2-buten, Oktafluorcyclobutan, Dekafluorbutan, Perfluorcyclopentan, Dodekafluorpentan und Tetradekafluorhexan, wobei das physiologisch zulässige Gas

gegebenenfalls Oktafluorpropan umfasst.

**9.** Verfahren zur Herstellung einer Mikropartikel-Zusammensetzung nach einem der Ansprüche 1 bis 3 oder einer Mikropartikel-Zwischenproduktzusammensetzung nach einem der Ansprüche 4 bis 6, wobei das Verfahren umfasst:

(i) Bilden einer Mikropartikel-Kernstruktur mit einem zentralen Bereich und einer Hülle, umfassend die Komponenten (i) bis (iii) und gegebenenfalls ferner umfassend:
(ii) kovalentes Anbringen mindestens eines molekularen Bindungselements an die Hülle der Mikropartikel-Kernstruktur.

**10.** Verfahren zur Herstellung einer Mikropartikel-Zusammensetzung nach Anspruch 9, wobei Schritt (i) in Gegenwart eines physiologisch zulässigen Gases durchgeführt wird und/oder wobei der molare Anteil der Konjugationsreaktion des mindestens einen molekularen Bindungselements zu der Komponente (ii) in Schritt (i) >1:1, gegebenenfalls >5:1, ist.

**11.** Pharmazeutische Zusammensetzung, umfassend eine Mikropartikel-Zusammensetzung nach einem der Ansprüche 1 bis 3 und einen pharmazeutisch zulässigen Träger oder Exzipienten.

**12.** Pharmazeutische Zusammensetzung nach Anspruch 11 zur Verwendung als Kontrastmittel bei der molekularen Bildgebung.

**13.** Mikropartikel-Zusammensetzung nach einem der Ansprüche 1 bis 3 zur Verwendung als Kontrastmittel bei der molekularen Bildgebung.

**14.** Abbildungsverfahren, umfassend:

Verabreichen der Mikropartikel-Zusammensetzung nach einem der Ansprüche 1 bis 3 oder einer pharmazeutischen Zusammensetzung nach Anspruch 11 an eine Person, und
Abbilden der Person mit mindestens einem bildgebenden Verfahren,
wobei das Bildgebungsverfahren wahlweise Ultraschall-Bildgebung (US) oder Magnetresonanz-Bildgebung (MRI) umfasst.

## Revendications

**1.** Composition de microparticules appropriée pour l'imagerie moléculaire, la composition comprenant des microparticules ayant une structure de microparticules de noyau avec une zone centrale et une enveloppe, et dans laquelle la structure de microparticules de noyau comprend :

(i) un lipide de phosphatidylcholine ;
(ii) un lipide de phosphatidyléthanolamine ayant au moins un groupement maléimide ; et
(iii) un acide gras alcoxylé, et

le rapport molaire de (i) à (iii) correspond aux plages suivantes :

(i) 70 à 80 ;
(ii) 5 à 15 ; et
(iii) 10 à 20.

**2.** Composition de microparticules selon la revendication 1, comprenant en outre au moins un élément de liaison moléculaire qui est fixé de manière covalente à l'enveloppe de la structure de microparticules du noyau,

- dans laquelle au moins cet élément de liaison moléculaire est éventuellement fixé de manière covalente à la structure de microparticules du noyau par au moins un fragment maléimide, et/ou
- dans laquelle au moins cet élément de liaison moléculaire comprend une protéine, un peptide ou un petit fragment organique, et au moins cet élément de liaison moléculaire étant un anticorps, et/ou
- dans laquelle le rapport molaire de la réaction de conjugaison de cet élément de liaison moléculaire au composant (ii) est > 1:1, éventuellement >5:1, et/ou

- dans laquelle les microparticules ont au moins environ 105 éléments de liaison moléculaire par microparticule.

**3.** Composition de microparticules selon la revendication 1 ou la revendication 2 dans laquelle le noyau de la structure des microparticules est micellaire, et/ou

- dans laquelle la structure de microparticules du noyau comprend en outre un fragment de marquage, le rapport molaire du fragment de marquage dans la composition étant en option, de l'ordre de 0,2 à 50, de l'ordre de 0,5 à 5, et le fragment de marquage étant en option, un colorant fluorescent, tel que le perchlorate de l,r-dioctadécyl-3,3,3',3'-tétraméthylindocarbocyanine (Dil), et/ou
- dans laquelle la structure des microparticules du noyau comprend en outre un lipide de phosphatidyléthanolamine, qui est éventuellement un lipide de phosphatidyléthanolamine saturé 10-20, tel qu'une distéaroylphosphatidyléthanolamine (DSPE), dont le rapport molaire du lipide de phosphatidyléthanolamine dans la composition est éventuellement de 0,5 à 5, et/ou
- dans laquelle la zone centrale de la structure des microparticules du noyau contient un milieu fluide, comprenant en option un gaz physiologiquement compatible choisi parmi : air, azote, dioxyde de carbone, xénon, krypton, hexafluorure de soufre, chlorotrifluorométhane, dichlorodifluoro-méthane, bromotrifluorométhane, bromochlorodifluorométhane, tétrafluorométhane, dibromo- difluorométhane, dichlorotétrafluoroéthane, chloropentafluoroéthane, hexafluoroéthane, hexafluoropropylène, octafluoropropane, hexafluoro-butadiène, octafluoro-2-butène, octafluorocyclobutane, décafluorobutane, perfluorocyclopentane, dodécafluoropentane et tétradécafluorohexane, le gaz physiologiquement compatible comprenant éventuellement de l'octafluoropropane, et/ou
- dans laquelle le lipide de phosphatidylcholine est un lipide de phosphatidylcholine saturé C10-20, tel qu'un 1,2-distéaroyl-sn-glycéro-3-phosphocholine, et/ou
- dans laquelle le lipide de phosphatidyléthanolamine comprenant au moins un groupement maléimide est un lipide de phosphatidyléthanolamine saturé C10-20, et/ou
- dans lequel le lipide de phosphatidyléthanolamine comprenant au moins un fragment maléimide comprend une chaîne de polyéthylèneglycol de poids moléculaire d'au moins 500, au moins ce fragment maléimide étant éventuellement fixé à la chaîne de polyéthylèneglycol, et le lipide de phosphatidyléthanolamine comprenant au moins un fragment maléimide étant un 1,2-distéaroyl-sn-glycéro-3-phosphoéthanolamine-7V- [maléimide(polyéthylèneglyco l)-2000], et/ou
- dans laquelle l'acide gras alcoxylé est un ester d'acide gras de polyéthylène glycol, qui est en option un ester d'acide gras de polyéthylène glycol saturé C10-20, tel qu'un stéarate de PEG40, et/ou
- dans laquelle le diamètre moyen des microparticules est compris entre 0,5 et 5 pm.

**4.** Composition intermédiaire de microparticules pour la production d'une composition de microparticules pour l'imagerie moléculaire, cette composition intermédiaire comprenant :

(i) un lipide de phosphatidylcholine ;
(ii) un lipide phosphatidyléthanolamine ayant au moins un groupement maléimide ; et
(iii) un acide gras alcoxylé, et

- dans laquelle le rapport molaire de (i) à (iii) correspond aux plages suivantes :

(i) 70 à 80 ;
(ii) 5 à 15 ; et
(iii) 10 à 20.

**5.** Composition de microparticules intermédiaires selon la revendication 4, comprenant en outre au moins un élément de liaison moléculaire, qui en option est fixé de manière covalente à au moins ce groupement maléimide, au moins cet élément de liaison moléculaire comprenant en option une protéine, une peptide ou un petit groupement organique, cet élément de liaison moléculaire étant un anticorps,

- dans laquelle le rapport molaire de la réaction de conjugaison de l'élément de liaison moléculaire au composant (ii) est en option, >1:1 ou >5:1, et/ou
- dans laquelle la composition de microparticules intermédiaire comprend en outre un fragment de marquage, le rapport molaire du fragment de marquage dans la composition étant en option, de 0,2 à 50, en option de 0,5 à 5, et le fragment de marquage étant en optiuon un colorant fluorescent, tel qu'un perchlorate de l,r-dioctadécyl-3,3,3',3'-tétraméthylindocarbocyanine (Dil), et/ou
- dans laquelle la composition de microparticules intermédiaires comprend en outre un lipide de phosphatidyléthanolamine, qui en option un lipide de phosphatidyléthanolamine saturé C10-20, tel qu'une distéaroylphosphatidyléthanolamine (DSPE), le rapport molaire du lipide de phosphatidyléthanolamine dans la composition étant en option 0. 5 à 5, et/ou

- dans laquelle le lipide de phosphatidylcholine est un lipide de phosphatidylcholine saturé C10-20, tel que 1,2-distéaroyl-sn-glycéro-3-phosphocholine, et/ou
- dans laquelle le lipide de phosphatidyléthanolamine comprenant au moins un groupement maléimide est un lipide de phosphatidyléthanolamine saturé en C10-20, et/ou
- dans laquelle le lipide de phosphatidyléthanolamine comprenant au moins un groupement maléimide a une chaîne de polyéthylèneglycol avec un poids moléculaire d'au moins 500, ce un groupement maléimide étant en option fixé à la chaîne de polyéthylèneglycol, et le lipide de phosphatidyléthanolamine comprenant au moins un groupement maléimide étant un 1,2-distcaroyl-.XXXs77-glycero-3-phosphocthanolamine N [maléimide(polyéthylène glyco l)-2000], et/ou
- dans laquelle l'acide gras alcoxylé est un ester d'acide gras de polyéthylèneglycol, qui est en option un ester d'acide gras de polyéthylène glycol saturé C10-20, tel qu'un stéarate de PEG40.

**6.** Composition intermédiaire de microparticules selon la revendication 4 ou la revendication 5, est un lyophilisat ou une dispersion aqueuse.

**7.** Kit comprenant une composition intermédiaire selon l'une quelconque des revendications 4 à 6, et une source d'un milieu fluide comprenant un gaz physiologiquement compatible.

**8.** Kit selon la revendication 7, dans lequel le gaz physiologiquement compatible est choisi parmi air, azote, dioxyde de carbone, xénon, krypton, hexafluorure de soufre, chlorotrifluorométhane, dichlorodifluorométhane, bromotrifluorométhane,bromochlorodifluorométhane, tétrafluorométhane, dibromo-difluorométhane,dichlorotétrafluoroéthane, chloropentafluoroéthane, hexafluoroéthane, hexafluoropropylène, octafluoropropane, hexafluoro-butadiène, octafluoro-2-butène, octafluorocyclobutane, décafluorobutane, perfluorocyclopentane, dodécafluoropentane, et tétradécafluorohexane, et en option le gaz physiologiquement comprenant de l'octafluoropropane.

**9.** Procédé de préparation d'une composition de microparticules selon l'une quelconque des revendications 1 à 3 ou d'une composition intermédiaire de microparticules selon l'une quelconque des revendications 4 à 6, le procédé consistant à :

(i) former une structure de microparticules de noyau ayant une zone centrale et une enveloppe comprenant les composants (i) à (iii), consistant en option en outre à:
(ii) fixer par covalence au moins un élément de liaison moléculaire à l'enveloppe de la structure de microparticules de noyau.

**10.** Procédé de préparation d'une composition de microparticules selon la revendication 9, dans lequel l'étape (i) est faite en présence d'un gaz physiologiquement compatible, et/ou
dans laquelle le rapport molaire de la réaction de conjugaison de l'élément de liaison moléculaire au composant (ii) dans l'étape (i) est >1:1, en option >5 :1

**11.** Composition pharmaceutique comprenant une composition de microparticules selon l'une des revendications 1 à 3, et un support ou excipient pharmaceutiquement compatible.

**12.** Composition pharmaceutique selon la revendication 11, utilisable comme agent de contraste pour l'imagerie moléculaire.

**13.** Composition de microparticules selon l'une des revendications 1 à 3 utilisable comme agent de contraste en imagerie moléculaire.

**14.** Procédé d'imagerie consistant à :
administrer à un sujet la composition de microparticules selon l'une quelconque des revendications 1 à 3, ou une composition pharmaceutique selon la revendication 11 à un sujet ; et

- faire l'imagerie du sujet en utilisant au moins une technique d'imagerie, comprend en option, l'échographie ou l'imagerie par résonance magnétique (IRM)

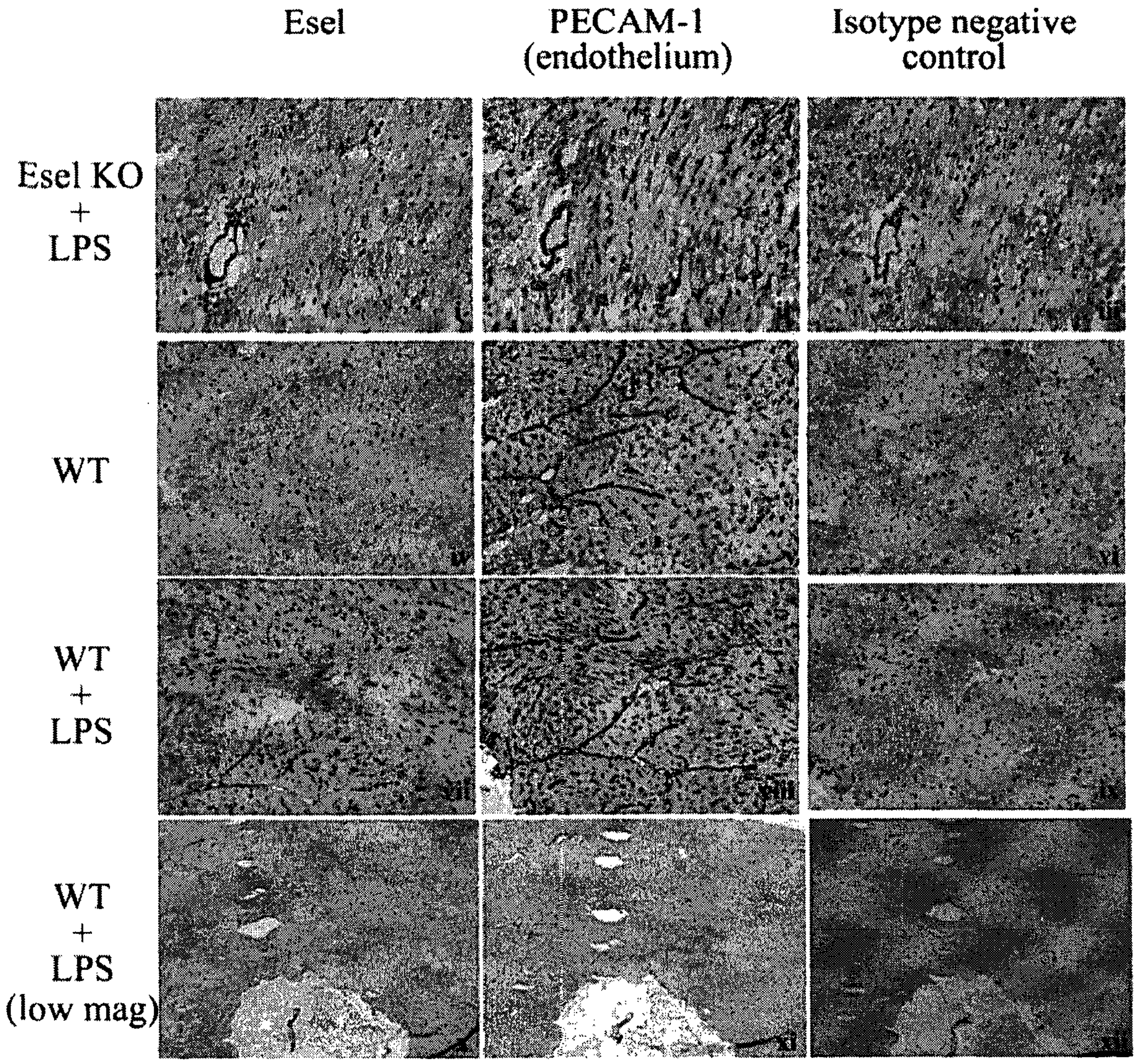
Esel
PECAM-1 (endothelium)
Isotype negative control
Esel KO + LPS
WT
WT + LPS
WT + LPS (low mag)

Figure 1

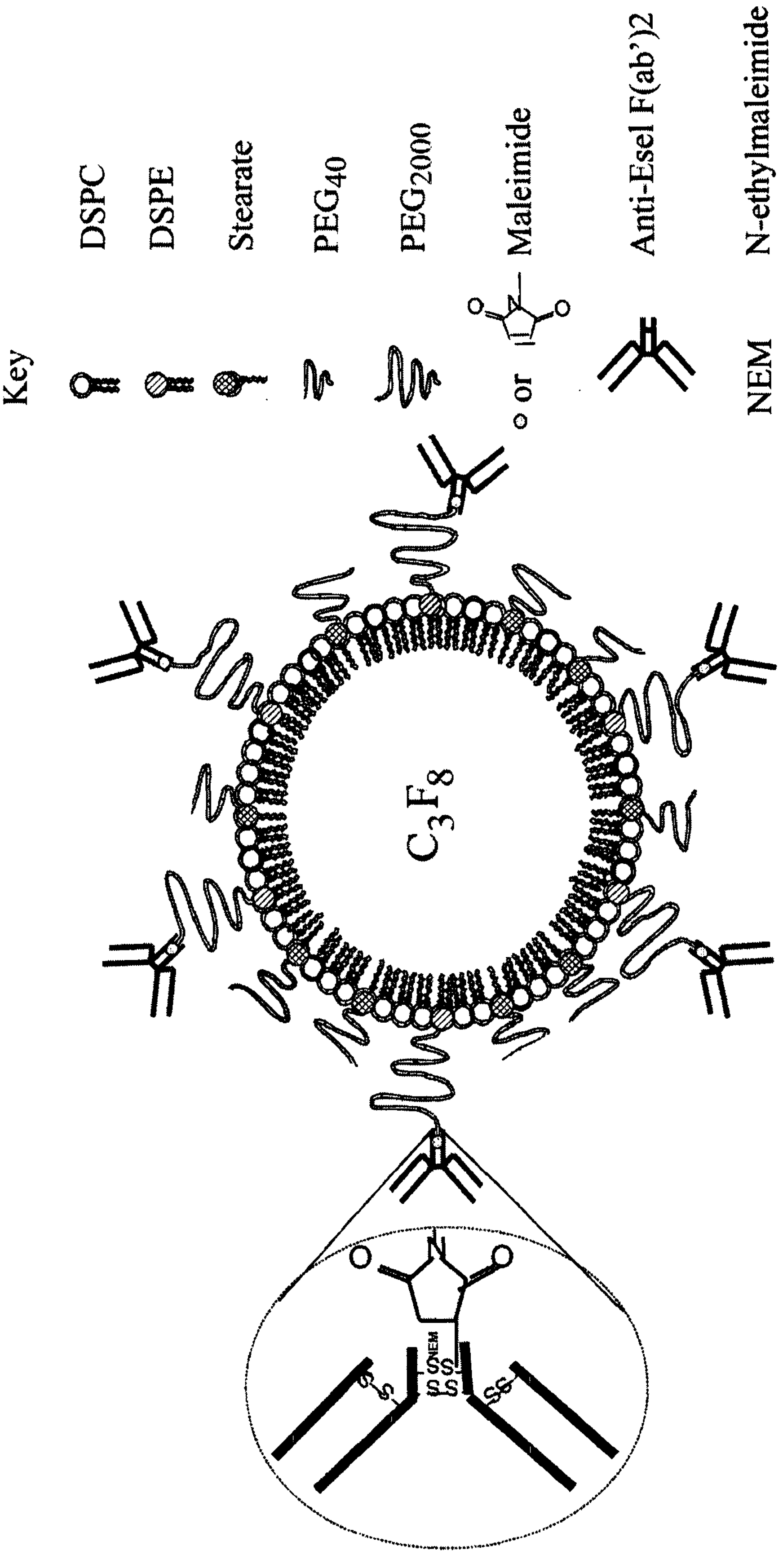
Key
DSPC
DSPE
Stearate
$PEG_{40}$
$PEG_{2000}$
or
Maleimide
Anti-Esel F(ab')2
NEM
N-ethylmaleimide
$C_3F_8$
NEM

Figure 2(a)

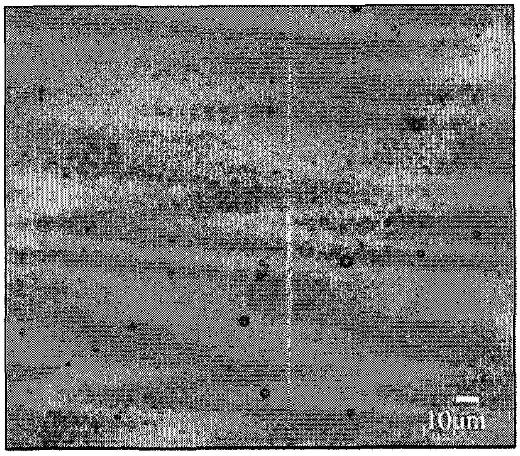


Figure 2(b)

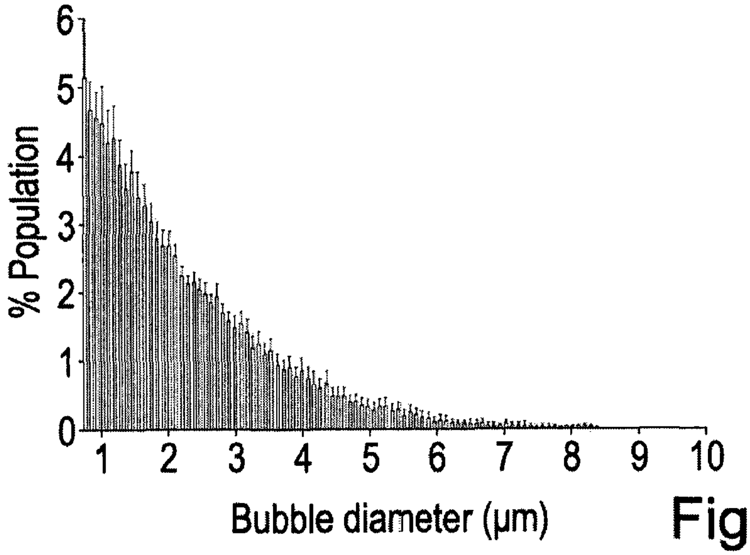


Figure 2(c)

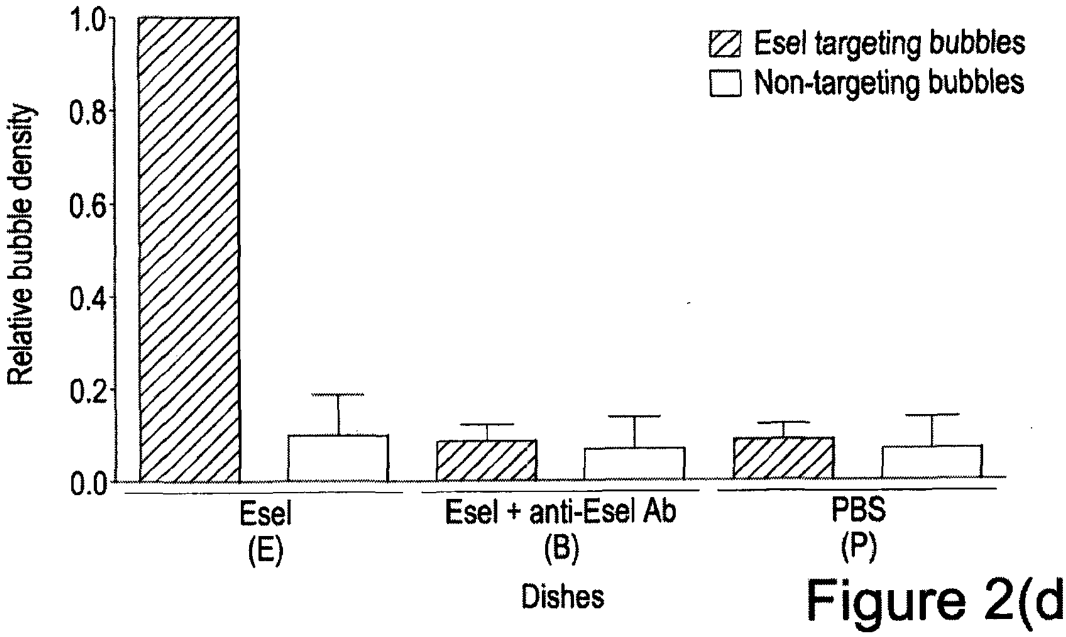


Figure 2(d)

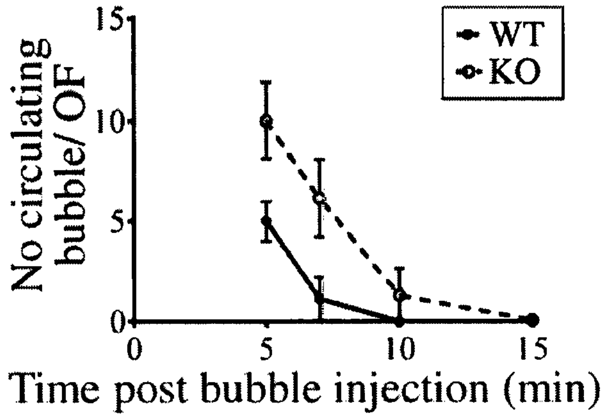


Figure 3(a)

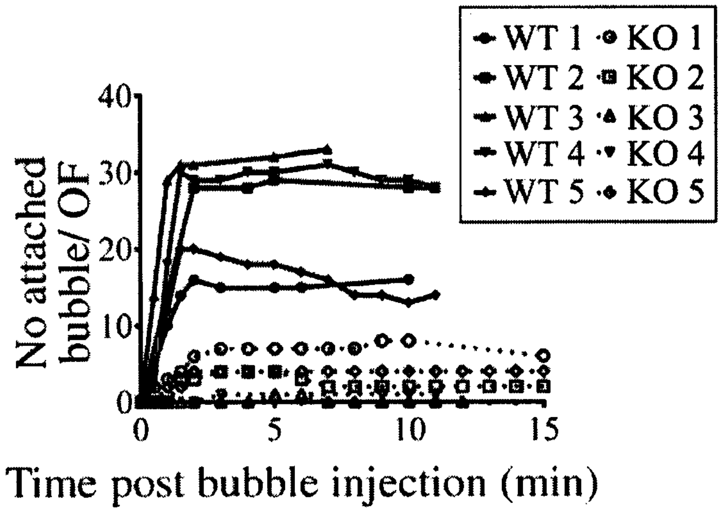


Figure 3(b)

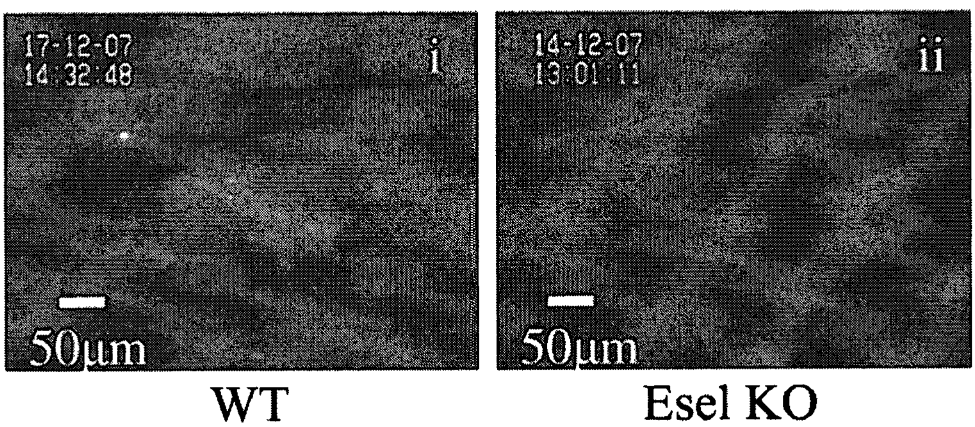


Figure 3(c)

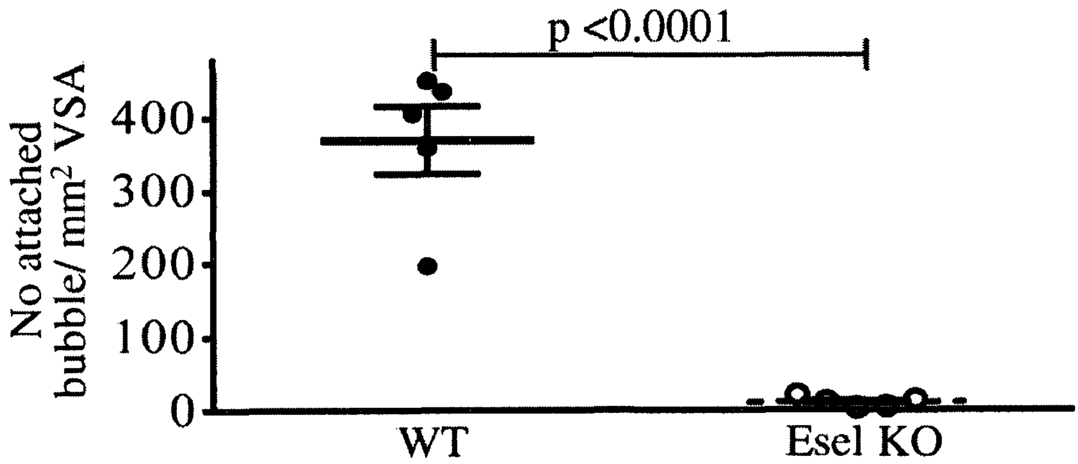


Figure 3(d)

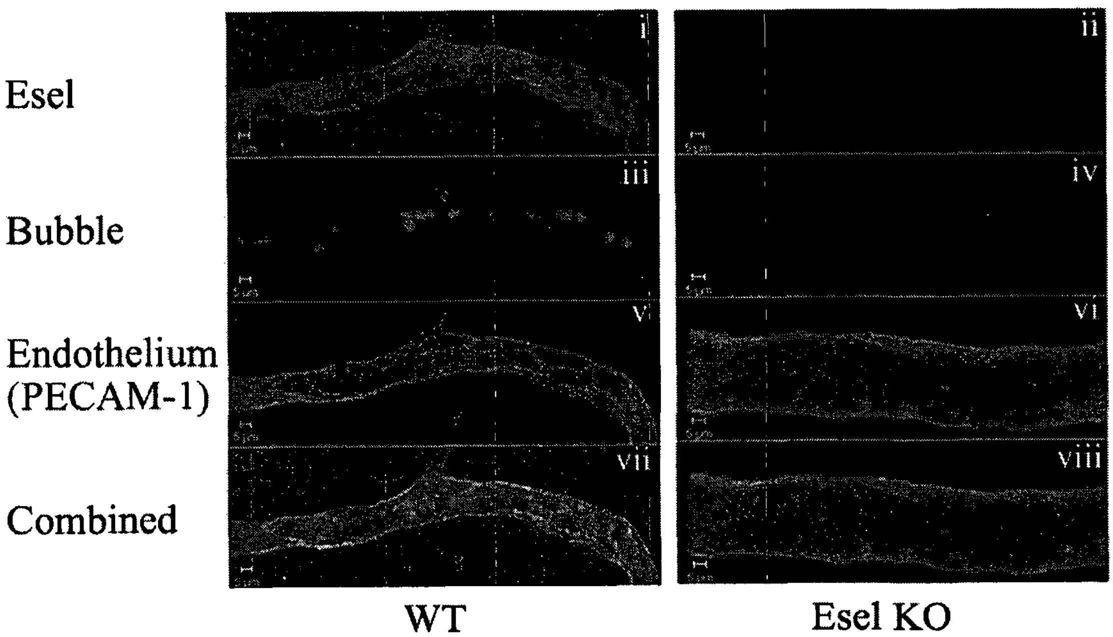


Figure 3(e)

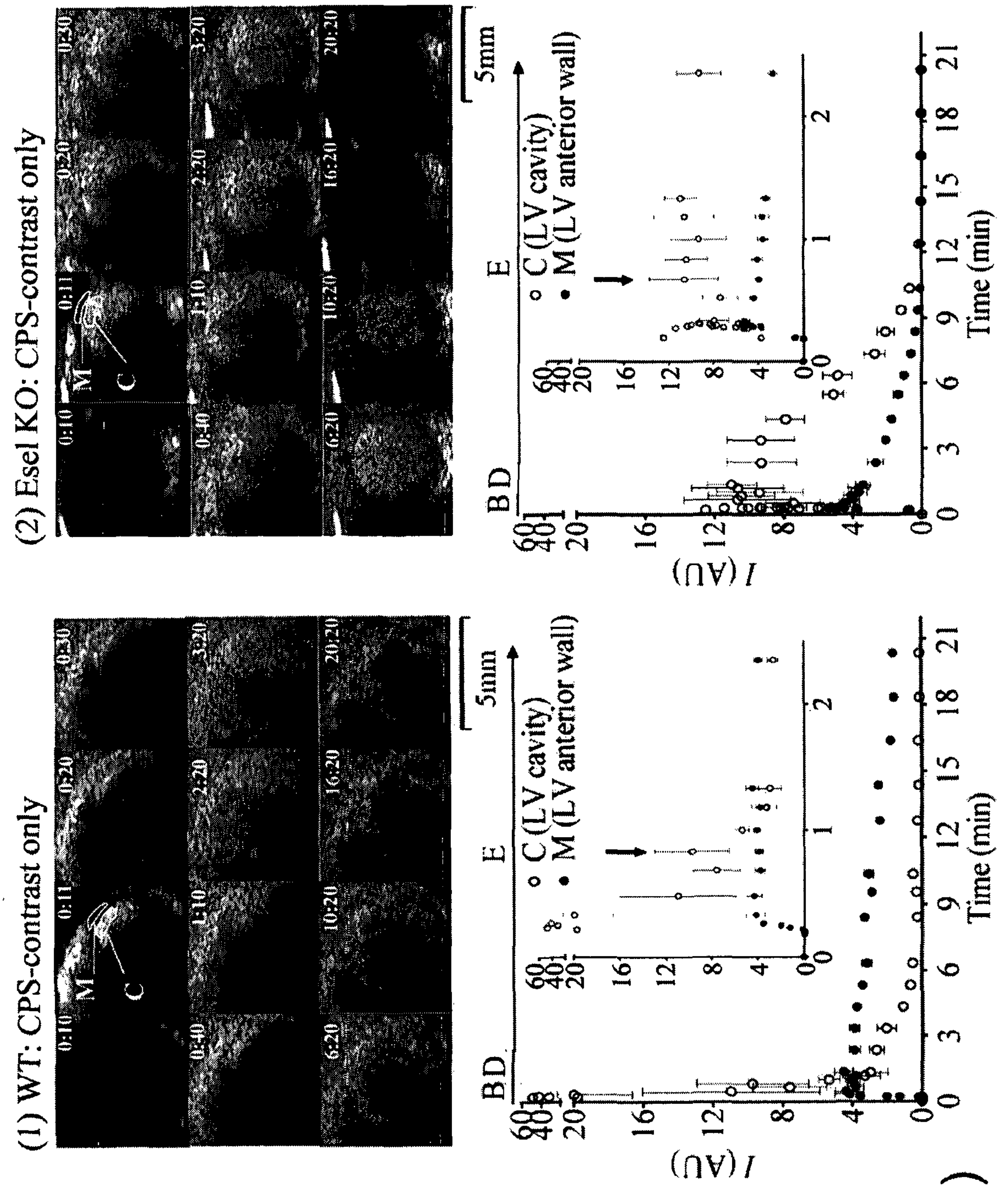
(1) WT: CPS-contrast only
(2) Esel KO: CPS-contrast only
M
C
5mm
BD
E
C (LV cavity)
M (LV anterior wall)
I (AU)
Time (min)

Figure 4(a)

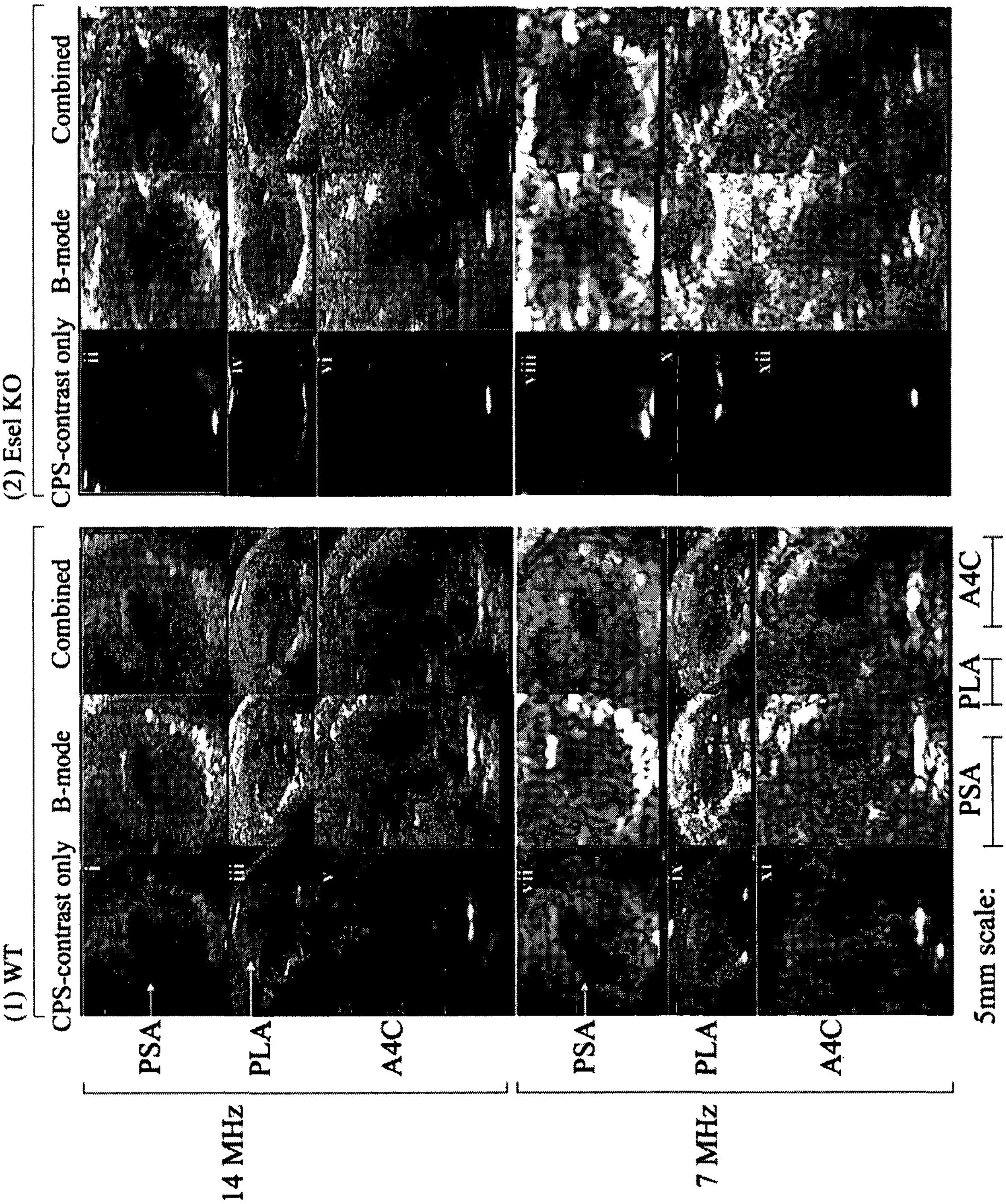
(1) WT
(2) Esel KO
CPS-contrast only
B-mode
Combined
CPS-contrast only
B-mode
Combined
14 MHz
7 MHz
PSA
PLA
A4C
PSA
PLA
A4C
5mm scale:
PSA
PLA
A4C

Figure 4(b)

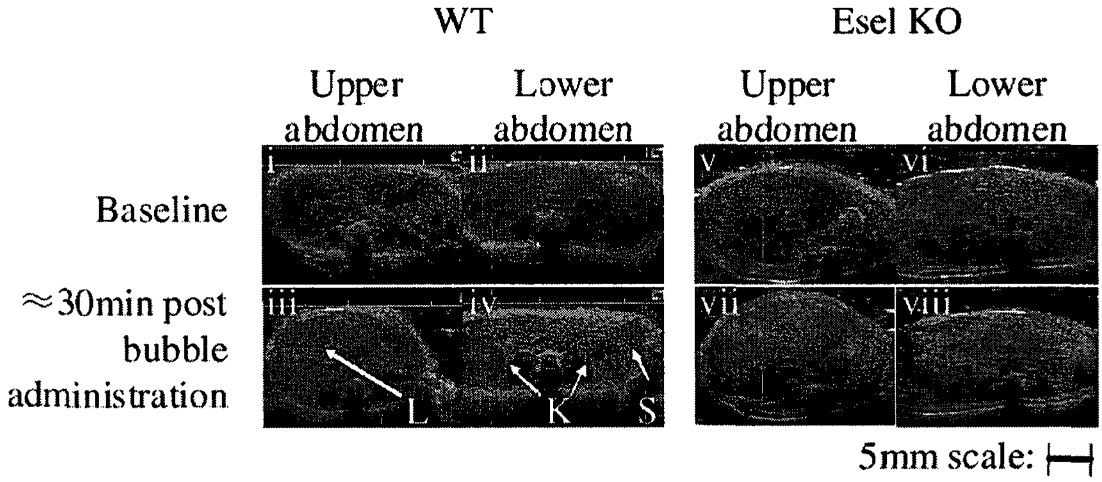


Figure 5a

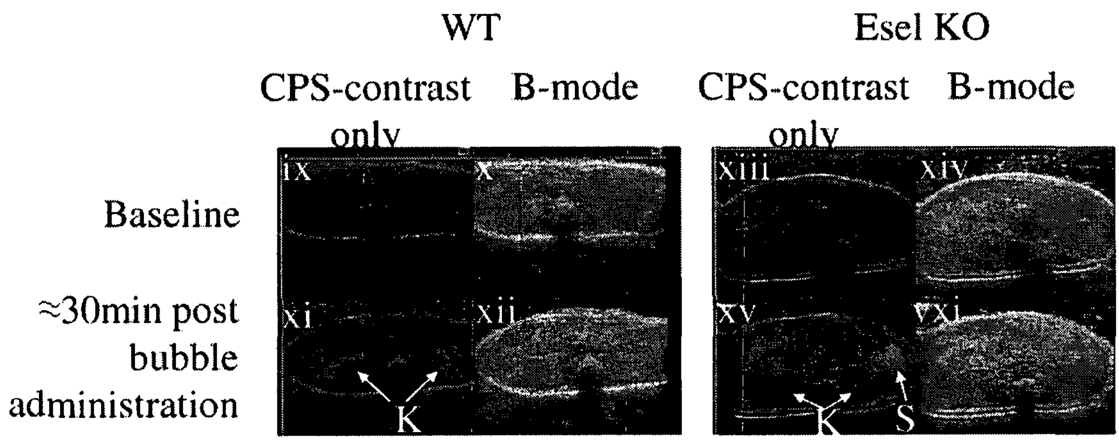


Figure 5b

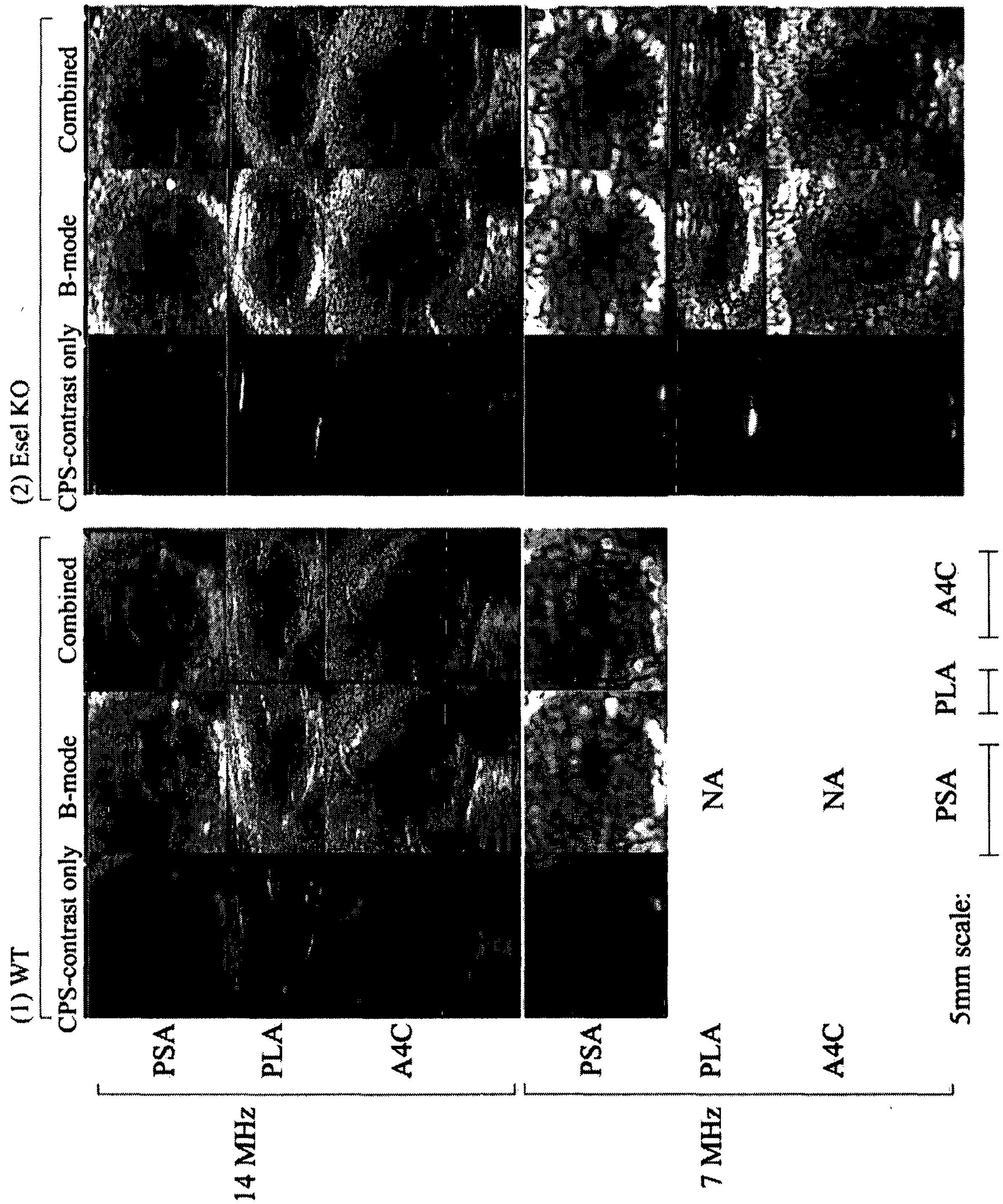
(1) WT
CPS-contrast only
B-mode
Combined
(2) Esel KO
CPS-contrast only
B-mode
Combined
PSA
PLA
A4C
14 MHz
PSA
PLA
A4C
7 MHz
NA
NA
5mm scale:
PSA
PLA
A4C

Figure 6

(a)
(b)
(c)

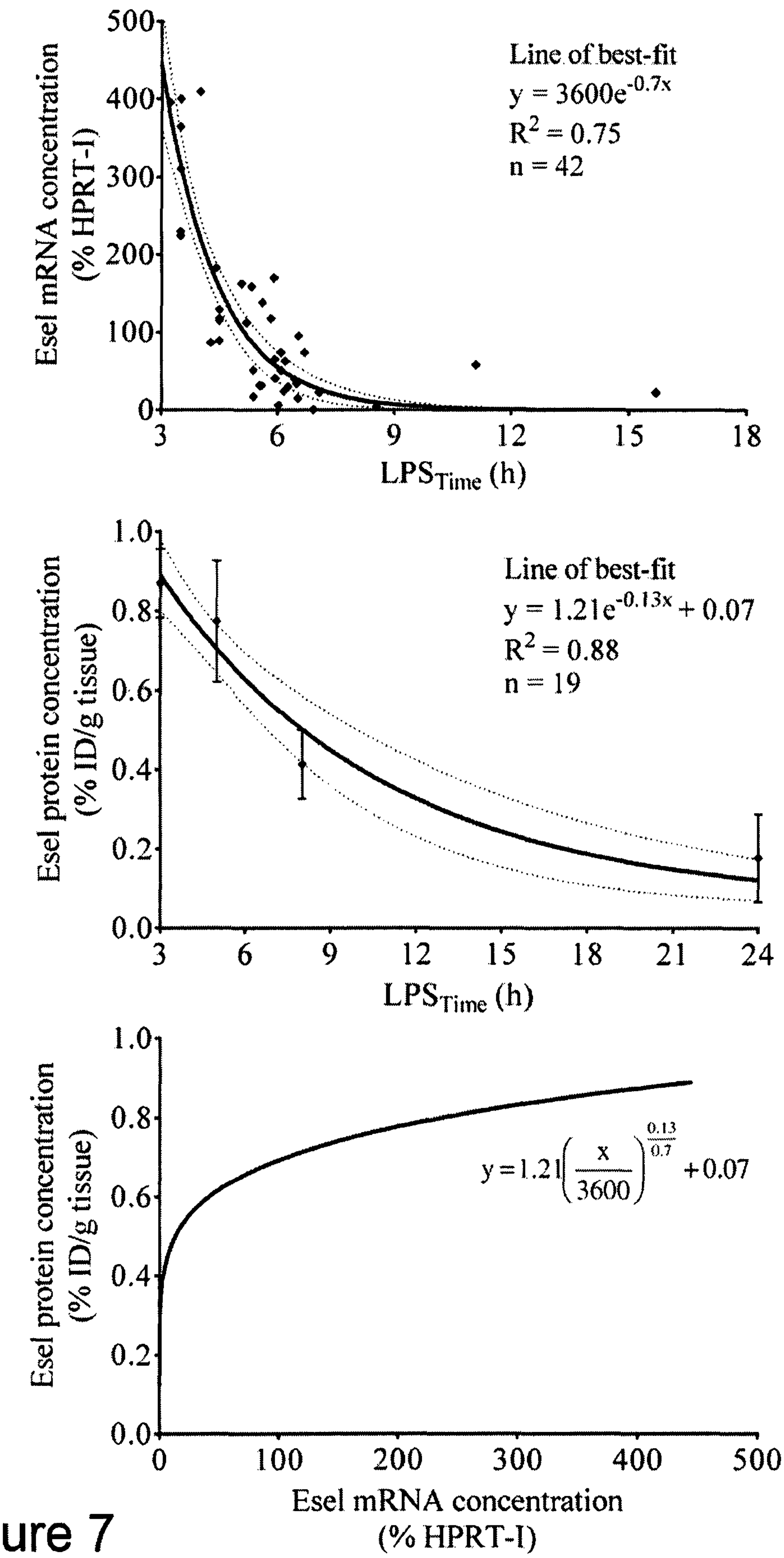
Line of best-fit
y = 3600e^-0.7x
R^2 = 0.75
n = 42
Esel mRNA concentration (% HPRT-I)
LPS_Time (h)
Line of best-fit
y = 1.21e^-0.13x + 0.07
R^2 = 0.88
n = 19
Esel protein concentration (% ID/g tissue)
LPS_Time (h)
y = 1.21(x/3600)^(0.13/0.7) + 0.07
Esel protein concentration (% ID/g tissue)
Esel mRNA concentration (% HPRT-I)

Figure 7

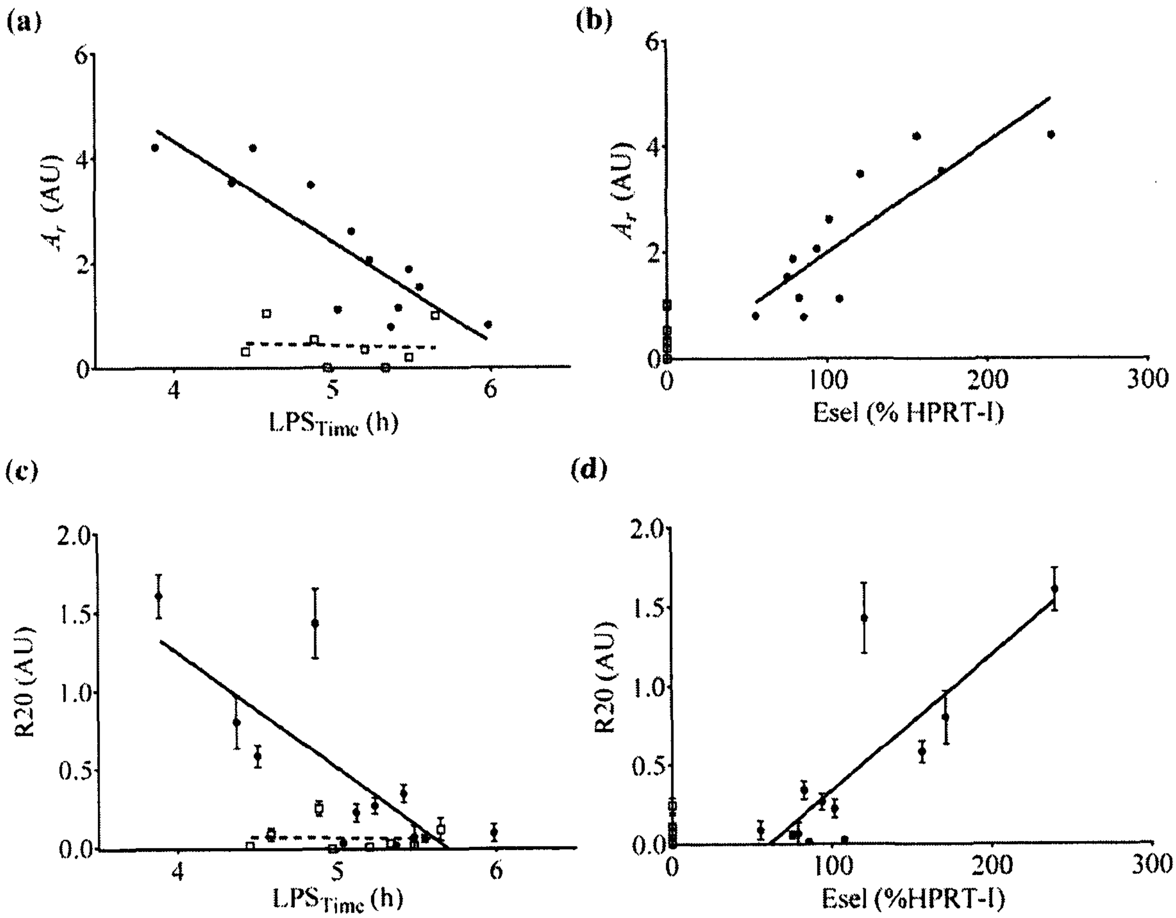
(a)
6
4
2
0
$A_r$ (AU)
4
5
6
$LPS_{Time}$ (h)
(b)
6
4
2
0
$A_r$ (AU)
0
100
200
300
Esel (% HPRT-I)
(c)
2.0
1.5
1.0
0.5
0.0
R20 (AU)
4
5
6
$LPS_{Time}$ (h)
(d)
2.0
1.5
1.0
0.5
0.0
R20 (AU)
0
100
200
300
Esel (%HPRT-I)

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9519274 A1


### Non-patent literature cited in the description


- **Y. NEGISHI et al.** *Biomaterials,* 22 October 2012, vol. 34, 501-507
- **J. R. LINDNER et al.** *Circulation,* 2001, vol. 104, 2107-2112
- **J. R. LINDNER et al.** *Circulation,* 2000, vol. 102, 2745-2750
- **J. B. FOWLKES.** *J Ultrasound Med,* 2008, vol. 27, 503-15
- **R. LUKAC et al.** *Langmuir,* 2011, vol. 27, 4829-37
- **P. J. BJORKMAN ; P. PARHAM.** *Annu Rev Biochem,* 1990, vol. 59, 253-88
- **K. NORD et al.** *Nat Biotechnol,* 1997, vol. 15, 772-7
- **K. NORD et al.** *J Biotechnol,* 2000, vol. 80, 45-54
- **E. HARLOW ; D. LANE.** Antibodies: A Laboratory Manual. Cold Spring Harbor Laboratory Press, 1988, 726
- **G. KANSAS.** Physiology of inflammation. Oxford University Press, 2001, 222-241
- **P. R. REYNOLDS et al.** *Radiology,* 2006, vol. 241, 469-76
- **F. JAMAR et al.** *Rheumatology,* 2002, vol. 41, 53-61
- **B. A. KAUFMANN et al.** *Eur Heart J,* 2007, vol. 28, 2011-7
- **S. ANDONIAN et al.** *J Endourol,* 2009, vol. 23, 373-8
- **B. A. KAUFMAN et al.** *J Am Soc Echocardiogr,* 2010, vol. 23, 79-85
- **P. HAUFF et al.** *Radiology,* 2004, vol. 231, 667-73
- **F. S. VILLANUEVA et al.** *Circulation,* 2007, vol. 115, 345-52
- **G. E. WELLER et al.** *Circulation,* 2003, vol. 108, 218-224
- **R. A. LINKER et al.** *J Autoimmun,* 2005, vol. 25, 199-205
- **M. REINHARDT et al.** *Neuroimage,* 2005, vol. 27, 267-78
- **B. A. KAUFMANN et al.** *Circulation,* 2007, vol. 116, 276-84
- **C. Z. BEHM et al.** *Circulation,* 2008, vol. 117, 2902-11
- **C. BACHMANN et al.** *Gastroenterology,* 2006, vol. 130, 8-16
- **J. W. XUAN et al.** *Mol Imaging,* 2009, vol. 8, 209-20
- **A. LYSHCHIK et al.** *J Ultrasound Med,* 2007, vol. 26, 1575-86
- **D. J. LEE et al.** *J Ultrasound Med,* 2008, vol. 27, 855-66
- **J. K. WILLMANN et al.** *Radiology,* 2008, vol. 249, 212-9
- **J. J. RYCHAK et al.** *Mol Imaging,* 2007, vol. 6, 289-96
- **M. A. PYSZ et al.** *Radiology,* 2010, vol. 256, 519-527
- **S. POCHON et al.** *Invest Radiol,* 2010, vol. 45, 89-95
- **R. PILLAI et al.** *Bioconjug Chem,* 2010, vol. 21, 556-562
- **G. KORPANTY et al.** *Clin Cancer Res,* 2007, vol. 13, 323-30
- **J. K. WILLMANN et al.** *Radiology,* 2008, vol. 248, 936-44
- **M. PALMOWSKI et al.** *Mol Cancer Ther,* 2008, vol. 7, 101-9
- **M. PALMOWSKI et al.** *Neoplasia,* 2009, vol. 11, 856-63
- **H. Y. JUN et al.** *Acad Radiol,* 2010, vol. 17, 54-60
- **J. K. WILLMANN et al.** *J Nucl Med,* 2010, vol. 51, 433-40
- **H. LEONG-POI et al.** *Circulation,* 2003, vol. 107, 455-460
- **H. LEONG-POI et al.** *Circulation,* 2005, vol. 111, 3248-54
- **D. B. ELLEGALA et al.** *Circulation,* 2003, vol. 108, 336-341
- **S. M. STIEGER et al.** *Contrast Media Mol Imaging,* 2008, vol. 3, 9-18
- **G. E. WELLER et al.** *Cancer Res,* 2005, vol. 65, 533-9
- **M. A. KULISZEWSKI et al.** *Cardiovasc Res,* 2009, vol. 83, 653-62

- **T. SAKUMA et al.** *Cardiovasc Res,* 2005, vol. 66, 552-61
- **A. ALONSO et al.** *Stroke,* 2007, vol. 38, 1508-14
- **I. TARDY et al.** *Acad Radiol,* 2002, vol. 9 (2), 294-6
- **M. TAKEUCHI et al.** *J.Am.Soc.Echocardiogr.,* 1999, vol. 12, 1015-1021
- **B. WANG et al.** *Acad Radiol,* 2006, vol. 13, 428-33
- **X. X. JING et al.** *Clin Imaging,* 2008, vol. 32, 178-82
- **J. P. CHRISTIANSEN et al.** *Circulation,* 2002, vol. 105, 1764-1767
- **I. KONDO et al.** *Circulation,* 2004, vol. 109, 1056-1061
- **J. S. CHEUNG et al.** *Neuroimage,* 2009, vol. 46, 658-64
- **R. TANG et al.** *Phys Med Biol,* 2011, vol. 56, 3503-12
- **F. YAN et al.** *Ultrasound Med Biol,* 2011, vol. 37, 768-79
- **A. DELLA MARTINA et al.** *Eur J Pharm Biopharm,* 2008, vol. 68, 555-64
- **S. UNNIKRISHNAN ; A. L. KLIBANOV.** *AJR Am J Roentgenol,* 2012, vol. 199, 292-9
- **A. L. KLIBANOV et al.** *Proceed Int'l Symp Control Rel Bioact Mater,* 1999, vol. 26, 230
- **M. A. LABOW et al.** *Immunity,* 1994, vol. 1, 709-720
- **M. J. EPPIHIME et al.** *Circ. Res.,* 1996, vol. 79, 560-569
- **J. W. FRI et al.** *Am J Pathol,* 1993, vol. 143, 725-37
- **M. J. HICKEY et al.** *J Immunol,* 1999, vol. 162, 1137-43
- **C. R. ANDERSON et al.** *Invest Radiol,* 2010, vol. 45, 579-85
- **C. R. ANDERSON et al.** *Invest Radiol,* 2011, vol. 46, 215-24
- **G. HU et al.** *Thromb Haemost,* 2012, vol. 107, 172-83
- **J. BZYL et al.** *Eur Radiol,* 2011, vol. 21, 1988-95
- **J. U. STREIF et al.** *Magn Reson Med,* 2005, vol. 53, 584-92
- **C. WALLER et al.** *Radiology,* 2000, vol. 215, 189-97
- **R. COULDEN et al.** Functional Computed Tomography. ISIS Medical Media, 1997, 133-156